# EUROPEAN PATENT APPLICATION

(11) **EP 1 295 610 A2**
(43) Date of publication of application: **26.03.2003**
(21) Application number: 02077880.9
(22) Date of filing: 17.07.2002
(51) Int. Cl.: A61K 47/48

(54) **Crystals of free and antibiotic complexed large ribosomal subunits**

(30) Priority: 24.09.2001 US 324060 P; 25.02.2002 US 358728 P; 29.03.2002 US 109572
(71) Applicant: YEDA RESEARCH & DEVELOPMENT COMPANY, LTD., 76100 Rehovot (IL); Max Planck Society, Max Planck-Gesellschaft zur Förderung der Wissenschaften e.V., 80539 München (DE)
(72) Inventor: Bashan, Anat, Nes Ziona (IL); Zarivach, Raz, 81513 Yavne (IL); Albrecht, Renate, 12107 Berlin (DE); Yonath, Ada, 76 345 Rehovot (IL); Schluenzen, Frank, 22880 Wedel (DE); Harms, Joerg, 22607 Hamburg (DE)
(74) Representative: Schmitz, Jean-Marie

(57) **Abstract**

Methods of growing crystals of free and antibiotic complexed large ribosomal subunits, coordinates defining the 3D atomic structure thereof and methods of utilizing such coordinates for rational design or identification of antibiotics or large ribosomal subunits having desired characteristics are disclosed.

## Description

### FIELD AND BACKGROUND OF THE INVENTION

The present invention relates to methods of growing large ribosomal subunit (LRS) crystals and antibiotic-LRS complex crystals and to methods of identifying putative antibiotics. In particular, embodiments of the present invention relate to methods of growing crystals of the *D. radiodurans* LRS and to methods of rationally designing or selecting novel antibiotics using three-dimensional (3D) atomic structure data obtained via X-ray crystallographic analysis of such crystals.

The mesophilic bacterium *Deinococcus radiodurans* (*D. radiodurans*) is an extremely robust gram-positive eubacterium that shares extensive similarity throughout its genome with *E. coli* and the thermophilic bacterium *Thermus thermophilus* (*T. thermophilus)* (White O. *et al.* (1999) Science 286:1571). *D. radiodurans* was originally identified as a live contaminant of irradiated canned meat and has been found to survive in an extremely broad range of environments ranging from hypo- to hyper-nutritive conditions, in atomic pile wastes, in weathered granite in extremely cold and dry antarctic valleys and as a live contaminant of irradiated medical instruments. This bacterium is the most radiation-resistant organism known, possessing the ability to survive under conditions normally causing lethal levels of DNA damage, such as in the presence of lethal levels of H₂O₂, ionizing radiation or ultraviolet radiation. The extreme adaptability of this organism is likely due to its specialized systems for DNA repair, DNA damage export, desiccation, temperature and starvation shock recovery and genetic redundancy.

The ribosome, the largest known macromolecular enzyme and the focus of intense biochemical research for over four decades, is a universal intracellular ribonucleoprotein complex which translates the genetic code, in the form of mRNA, into proteins (reviewed in Garrett, R. A. *et al.* eds. *The Ribosome Structure, Function, Antibiotics and Cellular Interactions,* (2000) ASM Press, Washington, DC.). Ribosomes of all species display great structural and functional similarities and are composed of two independent subunits, the small ribosomal subunit and the large ribosomal subunit (LRS), that associate upon initiation of protein biosynthesis. In prokaryotes, the small and large ribosomal subunits, which are respectively termed 30S and 50S according to their sedimentation coefficients (forming the 70S ribosomal particle upon association), have a molecular weight of 0.85 and 1.45 MDa, respectively. The 30S subunit consists of one 16S ribosomal RNA (rRNA) chain, composed of about 1500 nucleotides, and about 20 proteins and the LRS consists of two rRNA chains, termed 23S and 5S, and over 30 proteins. The 23S rRNA molecule contains about 3000 nucleotides and is the major component of this subunit. The *D. radiodurans* LRS (D50S) is composed of 5S and 23S rRNA molecules and ribosomal proteins L1-L7, L9-L24, CTC, L27-L36.

The ribosome has three binding sites for transfer RNA (tRNA), designated the P (peptidyl), A- (acceptor or aminoacyl) and E- (exit) sites which are partly located on both the small and large ribosomal subunits. In particular, the aminoacyl-tRNA (aa-tRNA) stem region binds the LRS, where catalysis of peptide bond synthesis, a process that involves addition of amino acids to the nascent polypeptide chain, occurs.

The 30S ribosomal subunit performs the process of decoding genetic information during translation. It initiates mRNA and tRNA anticodon stem loop engagement, governs mRNA and tRNA translocation, and controls fidelity of codon-anticodon interactions by discriminating between corresponding and non-corresponding aa-tRNAs in the A-site during translation of the genetic code. This subunit also functions in conjunction with the LRS to move tRNAs and associated mRNA by precisely one codon with respect to the ribosome, in a process termed translocation. The entire process also depends on several extrinsic protein factors and the hydrolysis of GTP.

The LRS is responsible for catalytic formation of the peptide bond, a vital biochemical process effected by this subunit via its peptidyl transferase center, the detailed mechanism, nor the structural basis of which, has been fully elucidated (Nissen, P. *et al.* Science (2000) 289:920; Polacek, N. *et al.* Nature (2001) 411:498; Thompson, J. *et al*. Proc Natl Acad Sci U S A 2001, 98(16):9002; Barta, A. *et al.* Science (2001) 291:203; Bayfield MA. *et al.* (2001) Proc Natl Acad Sci U S A 98:10096).

Importantly, the ribosomal subunits are the major molecular binding targets for a broad range of natural and synthetic antibiotics which prevent bacterial growth and/or survival by blocking subunit function, thereby preventing protein synthesis. The peptidyl transferase center of the LRS serves as the major binding target for many antibiotics, including chloramphenicol; lincosamides, such as clindamycin; streptogramins B; and substrate analogs, such as puromycin (Spahn, C. M. T. & Prescott, C. D. J Mol Med-Jmm (1996) 74:423).

Although the peptidyl transferase center is known to bind certain antibiotics, the structural basis of such interactions has been, until recently, unknown. Chloramphenicol is known to hamper binding of tRNA to the A-site, thereby inhibiting ribosome function by blocking peptidyl transferase activity (Rodriguez-Fonseca, C. *et al.* (1995) Journal of Molecular Biology 247:224; Moazed, D. and Noller, H. F. (1987) Biochemie 69:879). Furthermore, lincosamides, such as clindamycin-an antibiotic which is bactericidal to many gram-positive aerobic bacteria and many anaerobic microorganisms-inhibit ribosome function by interacting with the A- and P-sites (Kalliaraftopoulos, S. *et al.* (1994) Molecular Pharmacology 46:1009). Puromycin is also known to bind to the active site.

In contrast, macrolides, such as clarithromycin, erythromycin and roxithromycin, do not block peptidyl transferase activity (Vazquez, D. in: *Inhibitors of protein synthesis* (Springer Verlag, Berlin, Germany, 1975)). These antibiotics inhibit ribosome function by binding to the entrance of the protein exit tunnel of the LRS, thereby blocking the tunnel that channels the nascent peptides away from the peptidyl transferase center (Milligan, RA. and Unwin, PN. (1986) Nature 319:693; Nissen, P. *et al.* (2000) Science 289:920; Yonath, A. *et al.* (1987) Science 236:813). The group of erythromycin-derived macrolides, which includes clarithromycin and roxithromycin, are second generation semi-synthetic macrolides characterized by increased acid stability relative to erythromycin, (Steinmetz, W. E. *et al.* (1992) Journal of Medicinal Chemistry 35:4842; Gasc, J. C. *et al.* (1991) Journal of Antibiotics 44:313).

Thus, the ability to generate high resolution 3D atomic structure models of the LRS and of LRS-antibiotic complexes is of great importance since such models can be used to elucidate the mechanisms whereby ribosomes perform the crucial biological process of mRNA translation and the mechanisms whereby antibiotics inhibit ribosome function. Such models would constitute a powerful tool for the rational design or selection of, for example, novel and/or enhanced antibiotics or of ribosomes having enhanced protein production capacities and, as such, would be of significant benefit, for example, in the pharmacological and biomedical fields, the recombinant protein production industry and in the field of scientific research.

The ability to rationally design or identify antibiotics is of paramount medical importance, due the widespread mortality of bacterial diseases and, particularly, due to currently expanding global epidemics of increasing numbers of lethal diseases caused by antibiotic resistant or multi-resistant strains of bacterial pathogens. Such lethal and debilitating diseases include, for example, bacteremia, pneumonia, endocarditis, bone infections, joint infections and nocosomial infections caused by *Staphylococcus aureus* (Bradley SF. Clin Infect Dis. 2002, 34(2):211), and pulmonary infections caused by *Haemophilus influenzae* or *Streptococcus pneumoniae (S. pneumoniae*; Mlynarczyk G. *et al.* Int J Antimicrob Agents 2001, 18(6):497).

Large ribosomal subunit-targeting antibiotics to which bacterial resistance has become problematic include lincosamides, such as clindamycin, an effective antibiotic in the treatment of most infections involving anaerobes and gram-positive cocci (Kasten MJ. (1999) Mayo Clin Proc. 74:825); chloramphenicol, an effective antibiotic in the treatment of a wide variety of bacterial infections, including serious anaerobic infections (Johnson AW. *et al.* (1992) Acta Paediatr. 81:941); and macrolides, antibiotics offering coverage against a broad spectrum of pathogens and to which there has been reported a global increase in resistance among respiratory pathogens, particularly *S. pneumoniae* (Douthwaite S. (2001) Clin Microbiol Infect. Suppl 3:11). Indeed, resistance to macrolides and lincosamides, among other antibiotics, appears in almost all streptococcal species that attack humans (Horaud T. *et al.* (1985) J Antimicrob Chemother. 16 Suppl A:111).

The molecular mechanisms whereby bacteria become antibioticresistant usually involve drug efflux, drug inactivation, or alterations in the antibiotic target site. Although ribosomal proteins can affect the binding and action of ribosome-targeted antibiotics, the primary target of these antibiotics is rRNA (Cundliffe, E. in *The Ribosome: Structure, Function and Evolution* (1990) pp. 479-490, ASM Press, Washington, DC. (eds. Hill, W. E. *et al*.)) and many cases of antibiotic resistance in clinical strains can be linked to alterations of specific nucleotides of the 23S rRNA within the peptidyl transferase center or around the entrance of the exit tunnel of the LRS (Vester, B. and Douthwaite, S. Antimicrobial Agents and Chemotherapy (2001) 45:1).

Another advantage of 3D models of LRS structure at the atomic level is that these represent a significant step towards rational design or identification of ribosomes having desired protein production capacities. This could be of significant benefit for example, for enhancing production of recombinant proteins, such proteins being currently difficult and costly to produce in industrial quantities and being uniquely useful and highly potent in a very broad range of biomedical, pharmacological, industrial and scientific applications.

Numerous prior art approaches have been employed in attempts to generate complete high resolution 3D atomic structure models of ribosomes and of ribosome-antibiotic complexes.

One approach has attempted to crystallize ribosomal components of the eubacterium *E. coli,* the preferred model organism for such studies, for structural analysis thereof by X-ray crystallography. However, this approach has been hindered by the fact that *E. coli* ribosomal components are too fragile to resist deterioration during attempts at satisfactory crystallization thereof.

Another approach utilizing *E. coli* has used cryo-electron microscopy of the 70S ribosomal particle thereof complexed with formyl-methionyl initiator tRNAf(Met) (Gabashvili, I. S. *et al.* (2000) Cell 100:537). This approach, however, failed to yield high resolution structures of the ribosome.

Thus, approaches employing X-ray crystallography of ribosomal subunits of bacterial species adapted to extreme environmental conditions have been employed since such organisms appear to express robust ribosomal components, more easily crystallized, than those of *E. coli.*

One approach has employed X-ray crystallography of the 30S subunit of *T. thermophilus* (T30S) (Schluenzen, F. *et al.* Cell (2000) 102:615; Wimberly BT. *et al.* (2000) Nature 407:327; Clemons WM. *et al.* (2001) J Mol Biol. 310(4):827).

Yet another approach has used X-ray crystallography of a complex of T30S with the initiation factor IF1 (Carter, A.P. *et al.* (2001) Science 291:498).

Still another approach has employed X-ray crystallography of T30S in complex with mRNA and cognate tRNA in the A-site, both in the presence and absence of the antibiotic paromomycin (Ogle, J. M. *et al.* (2001) Science 292:897).

An additional approach has used X-ray crystallography of complexes of T30S with the antibiotics paromomycin, streptomycin or spectinomycin which interfere with decoding and translocation (Carter A.P. *et al.* (2000) Nature 407:340).

Yet an additional approach has employed X-ray crystallography of T30S in complexes with the antibiotics tetracycline, pactamycin or hygromycin (Brodersen D.E. *et al.* (2000) Cell 103:1143).

Still an additional approach has used X-ray crystallography of T30S in complexes with tetracycline, the universal initiation inhibitor edeine or the C-terminal domain of the translation initiation factor IF3 (Pioletti, M. *et al.* (2001) Embo J. 20:1829).

A further approach, similar to that described above utilizing *E. coli* 70S ribosomal particle, has employed X-ray crystallography of *T. thermophilus* 70S ribosomal particle (T70S) complexed with mRNAs and tRNAs (Yusupov MM. *et al.* Science (2001) 292:883).

Still a further approach has utilized X-ray crystallography of the LRS of the halophilic archaea *Haloarcula marismortui* (*H. marismortui;* Ban, N. *et al.* Science (2000) 289:905) and of complexes thereof with substrate analogs (Nissen, P. *et al.* (2000) Science 289:920).

All of the aforementioned prior art approaches, however, suffer from critical disadvantages.

Most significantly, all of the structural features involved in the non-catalytic functional aspects of protein biosynthesis were found to be disordered in the atomic structure of the 50S *H. marismortui* LRS (H50S), the only LRS whose structure has been partially determined at high resolution. Critically, this subunit has not been modeled in complex with a bound antibiotic molecule either. Indeed, since ribosomes of such halophilic bacteria are resistant to antibiotic agents (Mankin AS. and Garrett RA. (1991) J Bacteriol. 173:3559), the ribosomes thereof are less suitable models for generating ribosome-antibiotic complexes. Furthermore, *H. marismortui* is an archaea having eukaryotic properties and hence constitutes a sub-optimal model of ribosomal structure and function since biomedically, pharmacologically and industrially relevant bacterial strains are usually eubacteria which are evolutionarily and biologically divergent organisms (Willumeit R. *et al.* (2001) Biochim Biophys Acta. 1520(1):7).

In the case of approaches in which LRSs were modeled via determining the lower resolution structure of whole 70S ribosomal particles, none were capable of generating high resolution 3D atomic structure models, nor did these provide models of the atomic interactions between the LRS and any antibiotic molecule.

Thus, all prior art approaches have failed to provide adequate high resolution 3D atomic structure models of either the LRS or of a complex thereof with any antibiotic molecule.

There is thus a widely recognized need for, and it would be highly advantageous to have, high resolution 3D atomic structure models of the LRS and of antibiotic-LRS complexes devoid of the above limitation.

### SUMMARY OF THE INVENTION

According to the present invention there is provided composition-of-matter comprising a crystallized complex of an antibiotic bound to a large ribosomal subunit of a eubacterium.

According to another aspect of the present invention there is provided composition-of-matter comprising a crystallized LRS of a eubacterium.

According to yet another aspect of the present invention there is provided a method of identifying a putative antibiotic comprising: (a) obtaining a set of structure coordinates defining a three-dimensional atomic structure of a crystallized antibiotic-binding pocket of a large ribosomal subunit of a eubacterium; and (b) computationally screening a plurality of compounds for a compound capable of specifically binding the antibiotic-binding pocket, thereby identifying the putative antibiotic.

According to still another aspect of the present invention there is provided a computing platform for generating a three-dimensional model of at least a portion of a large ribosomal subunit of a eubacterium, the computing platform comprising: (a) a data-storage device storing data comprising a set of structure coordinates defining at least a portion of a three-dimensional structure of the large ribosomal subunit; and (b) a processing unit being for generating the three-dimensional model from the data stored in the data-storage device.

According to an additional aspect of the present invention there is provided a computing platform for generating a three-dimensional model of at least a portion of a complex of an antibiotic and a large ribosomal subunit of a eubacterium, the computing platform comprising: (a) a data-storage device storing data comprising a set of structure coordinates defining at least a portion of a three-dimensional structure of the complex of an antibiotic and a large ribosomal subunit; and (b) a processing unit being for generating the three-dimensional model from the data stored in the data-storage device.

According to yet an additional aspect of the present invention there is provided a computer generated model representing at least a portion of a large ribosomal subunit of a eubacterium, the computer generated model having a three-dimensional atomic structure defined by a set of structure coordinates corresponding to a set of coordinates set forth in Table 3, the set of atom coordinates set forth in Table 3 being selected from the group consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 61881-62151; atom coordinates 62152-62357; atom coordinates 62358-62555; atom coordinates 2556-62734; atom coordinates 62735-62912; atom coordinates 62913-62965; atom coordinates 62966-63109; atom coordinates 3110-63253; atom coordinates 63254-63386; atom coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to still an additional aspect of the present invention there is provided a computer generated model representing at least a portion of a large ribosomal subunit of a eubacterium, the computer generated model having a three-dimensional atomic structure defined by a set of structure coordinates having a root mean square deviation of not more than 2.0 Å from a set of structure coordinates corresponding to a set of coordinates set forth in Table 3, the set of atom coordinates set forth in Table 3 being selected from the group consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 61881-62151; atom coordinates 62152-62357; atom coordinates 62358-62555; atom coordinates 62556-62734; atom coordinates 62735-62912; atom coordinates 62913-62965; atom coordinates 62966-63109; atom coordinates 63110-63253; atom coordinates 63254-63386; atom coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to a further aspect of the present invention there is provided a computer generated model representing at least a portion of a complex of an antibiotic and a large ribosomal subunit of a eubacterium.

According to yet a further aspect of the present invention there is provided a computer readable medium comprising, in a retrievable format, data including a set of structure coordinates defining at least a portion of a three-dimensional structure of a crystallized large ribosomal subunit of a eubacterium.

According to still a further aspect of the present invention there is provided a computer readable medium comprising, in a retrievable format, data including a set of structure coordinates defining at least a portion of a three-dimensional structure of a crystallized complex of an antibiotic and a large ribosomal subunit of a eubacterium.

According to another aspect of the present invention there is provided a method of crystallizing a large ribosomal subunit of a eubacterium comprising: (a) suspending a purified preparation of the large ribosomal subunit in a crystallization solution, the crystallization solution comprising a buffer component and a volatile component, the volatile component being at a first concentration in the crystallization solution, thereby forming a crystallization mixture; and (b) equilibrating the crystallization mixture with an equilibration solution, the equilibration solution comprising a second buffer component and the volatile component, the volatile component being at a second concentration in the equilibration solution, the second concentration being a fraction of the first concentration, thereby crystallizing the large ribosomal subunit.

According to further features in preferred embodiments of the invention described below, the eubacterium is *D. radiodurans.*

According to still further features in the described preferred embodiments, the antibiotic is clindamycin and whereas the crystallized complex is characterized by unit cell dimensions of a = 170.286 ± 10 Å, b = 410.134 ± 15 Å and c = 697.201 ± 25 Å.

According to still further features in the described preferred embodiments, the antibiotic is erythromycin and whereas the crystallized complex is characterized by unit cell dimensions of a = 169.194 ± 10 Å, b = 409.975 ± 15 Å and c = 695.049 ± 25 Å.

According to still further features in the described preferred embodiments, the antibiotic is clarithromycin and whereas the crystallized complex is characterized by unit cell dimensions of a = 169.871 ± 10 Å, b = 412.705 ± 15 Å and c = 697.008 ± 25 Å.

According to still further features in the described preferred embodiments, the antibiotic is roxithromycin and whereas the crystallized complex is characterized by unit cell dimensions of a = 170.357 ± 10 Å, b = 410.713 ± + 15 Å and c = 694.810 + 25 Å.

According to still further features in the described preferred embodiments, the antibiotic is chloramphenicol and whereas the crystallized complex is characterized by unit cell dimensions of a = 171.066 ± 10 Å, b = 409.312 ± 15 Å and c = 696.946 ± 25 Å.

According to still further features in the described preferred embodiments, the crystallized complex is characterized by having a crystal space group of 1222.

According to still further features in the described preferred embodiments, the antibiotic is selected from the group consisting of chloramphenicol, a lincosamide antibiotic, clindamycin, a macrolide antibiotic, clarithromycin, erythromycin and roxithromycin.

According to still further features in the described preferred embodiments, the antibiotic is chloramphenicol and whereas the large ribosomal subunit comprises a nucleic acid molecule, a segment of which including nucleotides being associated with the chloramphenicol, wherein a three-dimensional atomic structure of the nucleotides is defined by the set of structure coordinates corresponding to nucleotide coordinates 2044, 2430, 2431, 2479 and 2483-2485 set forth in Table 8.

According to still further features in the described preferred embodiments, the antibiotic is chloramphenicol and whereas the large ribosomal subunit comprises a nucleic acid molecule, a segment of which including nucleotides being associated with the chloramphenicol, wherein a three-dimensional atomic structure of the nucleotides is defined by a set of structure coordinates having a root mean square deviation of not more than 2.0 Å from the set of structure coordinates corresponding to nucleotide coordinates 2044, 2430, 2431, 2479 and 2483-2485 set forth in Table 8.

According to still further features in the described preferred embodiments, a three-dimensional atomic structure of the segment is defined by the set of structure coordinates corresponding to nucleotide coordinates 2044-2485 set forth in Table 8.

According to still further features in the described preferred embodiments, a three-dimensional atomic structure of the segment is defined by a set of structure coordinates having a root mean square deviation of not more than 2.0 Å from the set of structure coordinates corresponding to nucleotide coordinates 2044-2485 set forth in Table 8.

According to still further features in the described preferred embodiments, the antibiotic is chloramphenicol and whereas a three-dimensional atomic structure of the chloramphenicol is defined by the set of structure coordinates corresponding to HETATM coordinates 59925-59944 set forth in Table 8.

According to still further features in the described preferred embodiments, the antibiotic is chloramphenicol and whereas a three-dimensional atomic structure of the chloramphenicol is defined by a set of structure coordinates having a root mean square deviation of not more than 2.0 Å from the set of structure coordinates corresponding to HETATM coordinates 59925-59944 set forth in Table 8.

According to still further features in the described preferred embodiments, the antibiotic is clindamycin and whereas the large ribosomal subunit comprises a nucleic acid molecule, a segment of which including nucleotides being associated with the clindamycin, wherein a three-dimensional atomic structure of the nucleotides is defined by the set of structure coordinates corresponding to nucleotide coordinates 2040-2042, 2044, 2482, 2484 and 2590 set forth in Table 9.

According to still further features in the described preferred embodiments, the antibiotic is clindamycin and whereas the large ribosomal subunit comprises a nucleic acid molecule, a segment of which including nucleotides being associated with the clindamycin, wherein a three-dimensional atomic structure of the nucleotides is defined by a set of structure coordinates having a root mean square deviation of not more than 2.0 Å from the set of structure coordinates corresponding to nucleotide coordinates 2040-2042, 2044, 2482, 2484 and 2590 set forth in Table 9.

According to still further features in the described preferred embodiments, a three-dimensional atomic structure of the segment is defined by the set of structure coordinates corresponding to nucleotide coordinates 2040-2590 set forth in Table 9.

According to still further features in the described preferred embodiments, a three-dimensional atomic structure of the segment is defined by a set of structure coordinates having a root mean square deviation of not more than 2.0 Å from the set of structure coordinates corresponding to nucleotide coordinates 2040-2590 set forth in Table 9.

According to still further features in the described preferred embodiments, the antibiotic is clindamycin and whereas a three-dimensional atomic structure of the clindamycin is defined by the set of structure coordinates corresponding to HETATM coordinates 59922-59948 set forth in Table 9.

According to still further features in the described preferred embodiments, the antibiotic is clindamycin and whereas a three-dimensional atomic structure of the clindamycin is defined by a set of structure coordinates having a root mean square deviation of not more than 2.0 Å from the set of structure coordinates corresponding to HETATM coordinates 59922-59948 set forth in Table 9.

According to still further features in the described preferred embodiments, the antibiotic is clarithromycin and whereas the large ribosomal subunit comprises a nucleic acid molecule, a segment of which including nucleotides being associated with the clarithromycin, wherein a three-dimensional atomic structure of the nucleotides is defined by the set of structure coordinates corresponding to nucleotide coordinates 2040-2042, 2045, 2484, 2588 and 2589 set forth in Table 10.

According to still further features in the described preferred embodiments, the antibiotic is clarithromycin and whereas the large ribosomal subunit comprises a nucleic acid molecule, a segment of which including nucleotides being associated with the clarithromycin, wherein a three-dimensional atomic structure of the nucleotides is defined by a set of structure coordinates having a root mean square deviation of not more than 2.0 Å from the set of structure coordinates corresponding to nucleotide coordinates 2040-2042, 2045, 2484, 2588 and 2589 set forth in Table 10.

According to still further features in the described preferred embodiments, a three-dimensional atomic structure of the segment is defined by the set of structure coordinates corresponding to nucleotide coordinates 2040-2589 set forth in Table 10.

According to still further features in the described preferred embodiments, a three-dimensional atomic structure of the segment is defined by a set of structure coordinates having a root mean square deviation of not more than 2.0 Å from the set of structure coordinates corresponding to nucleotide coordinates 2040-2589 set forth in Table 10.

According to still further features in the described preferred embodiments, the antibiotic is clarithromycin and whereas a three-dimensional atomic structure of the clarithromycin is defined by the set of structure coordinates corresponding to HETATM coordinates 59922-59973 set forth in Table 10.

According to still further features in the described preferred embodiments, the antibiotic is clarithromycin and whereas a three-dimensional atomic structure of the clarithromycin is defined by a set of structure coordinates having a root mean square deviation of not more than 2.0 Å from the set of structure coordinates corresponding to HETATM coordinates 59922-59973 set forth in Table 10.

According to still further features in the described preferred embodiments, the antibiotic is erythromycin and whereas the large ribosomal subunit comprises a nucleic acid molecule, a segment of which including nucleotides being associated with the erythromycin, wherein a three-dimensional atomic structure of the nucleotides is defined by the set of structure coordinates corresponding to nucleotide coordinates 2040-2042, 2045, 2484, 2588 and 2589 set forth in Table 11.

According to still further features in the described preferred embodiments, the antibiotic is erythromycin and whereas the large ribosomal subunit comprises a nucleic acid molecule, a segment of which including nucleotides being associated with the erythromycin, wherein a three-dimensional atomic structure of the nucleotides is defined by a set of structure coordinates having a root mean square deviation of not more than 2.0 Å from the set of structure coordinates corresponding to nucleotide coordinates 2040-2042, 2045, 2484, 2588 and 2589 set forth in Table 11.

According to still further features in the described preferred embodiments, a three-dimensional atomic structure of the segment is defined by the set of structure coordinates corresponding to nucleotide coordinates 2040-2589 set forth in Table 11.

According to still further features in the described preferred embodiments, a three-dimensional atomic structure of the segment is defined by a set of structure coordinates having a root mean square deviation of not more than 2.0 Å from the set of structure coordinates corresponding to nucleotide coordinates 2040-2589 set forth in Table 11.

According to still further features in the described preferred embodiments, the antibiotic is erythromycin and whereas a three-dimensional atomic structure of the erythromycin is defined by the set of structure coordinates corresponding to HETATM coordinates 59922-59972 set forth in Table 11.

According to still further features in the described preferred embodiments, the antibiotic is erythromycin and whereas a three-dimensional atomic structure of the erythromycin is defined by a set of structure coordinates having a root mean square deviation of not more than 2.0 Å from the set of structure coordinates corresponding to HETATM coordinates 59922-59972 set forth in Table 11.

According to still further features in the described preferred embodiments, the antibiotic is roxithromycin and whereas the large ribosomal subunit comprises a nucleic acid molecule, a segment of which including nucleotides being associated with the roxithromycin, wherein a three-dimensional atomic structure of the nucleotides is defined by the set of structure coordinates corresponding to nucleotide coordinates 2040-2042, 2045, 2484, 2588 and 2589 set forth in Table 12.

According to still further features in the described preferred embodiments, the antibiotic is roxithromycin and whereas the large ribosomal subunit comprises a nucleic acid molecule, a segment of which including nucleotides being associated with the roxithromycin, wherein a three-dimensional atomic structure of the nucleotides is defined by a set of structure coordinates having a root mean square deviation of not more than 2.0 Å from the set of structure coordinates corresponding to nucleotide coordinates 2040-2042, 2045, 2484, 2588 and 2589 set forth in Table 12.

According to still further features in the described preferred embodiments, a three-dimensional atomic structure of the segment is defined by the set of structure coordinates corresponding to nucleotide coordinates 2040-2589 set forth in Table 12.

According to still further features in the described preferred embodiments, a three-dimensional atomic structure of the segment is defined by a set of structure coordinates having a root mean square deviation of not more than 2.0 Å from the set of structure coordinates corresponding to nucleotide coordinates 2040-2589 set forth in Table 12.

According to still further features in the described preferred embodiments, the antibiotic is roxithromycin and whereas a three-dimensional atomic structure of the roxithromycin is defined by the set of structure coordinates corresponding to HETATM coordinates 59922-59979 set forth in Table 12.

According to still further features in the described preferred embodiments, the antibiotic is roxithromycin and whereas a three-dimensional atomic structure of the roxithromycin is defined by a set of structure coordinates having a root mean square deviation of not more than 2.0 Å from the set of structure coordinates corresponding to HETATM coordinates 59922-59979 set forth in Table 12.

According to still further features in the described preferred embodiments, the crystallized large ribosomal subunit is characterized by unit cell dimensions of a = 170.827 ± 10 Å, b = 409.430 ± 15 Å and c = 695.597 ± 25 Å.

According to still further features in the described preferred embodiments, the crystallized large ribosomal subunit is characterized by having a crystal space group of 1222.

According to still further features in the described preferred embodiments, a three-dimensional atomic structure of at least a portion of the crystallized large ribosomal subunit is defined by a set of structure coordinates corresponding to a set of coordinates set forth in Table 3, the set of coordinates set forth in Table 3 being selected from the group consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 61881-62151; atom coordinates 62152-62357; atom coordinates 62358-62555; atom coordinates 62556-62734; atom coordinates 62735-62912; atom coordinates 62913-62965; atom coordinates 62966-63109; atom coordinates 63110-63253; atom coordinates 63254-63386; atom coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to still further features in the described preferred embodiments, a three-dimensional atomic structure of at least a portion of the crystallized large ribosomal subunit is defined by a set of structure coordinates having a root mean square deviation of not more than 2.0 Å from a set of structure coordinates corresponding to a set of coordinates set forth in Table 3, the set of coordinates set forth in Table 3 being selected from the consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 61881-62151; atom coordinates 62152-62357; atom coordinates 62358-62555; atom coordinates 62556-62734; atom coordinates 62735-62912; atom coordinates 62913-62965; atom coordinates 62966-63109; atom coordinates 63110-63253; atom coordinates 63254-63386; atom coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to still further features in the described preferred embodiments, the crystallized large ribosomal subunit comprises a nucleic acid molecule, a segment of which including nucleotides being capable of specifically associating with an antibiotic selected from the group consisting of chloramphenicol, a lincosamide antibiotic, clindamycin, a macrolide antibiotic, clarithromycin, erythromycin and roxithromycin.

According to still further features in the described preferred embodiments, a three-dimensional atomic structure of the nucleic acid molecule is defined by the set of structure coordinates corresponding to atom coordinates 1-59360 set forth in Table 3.

According to still further features in the described preferred embodiments, the antibiotic is chloramphenicol and whereas a three-dimensional atomic structure of the nucleotides being capable of specifically associating with the chloramphenicol is defined by the set of structure coordinates corresponding to nucleotide coordinates 2044, 2430, 2431, 2479 and 2483-2485 set forth in Table 3.

According to still further features in the described preferred embodiments, the antibiotic is chloramphenicol and whereas a three-dimensional atomic structure of the nucleotides being capable of specifically associating with the chloramphenicol is defined by a set of structure coordinates having a root mean square deviation of not more than 2.0 Å from the set of structure coordinates corresponding to nucleotide coordinates 2044, 2430, 2431, 2479 and 2483-2485 set forth in Table 3.

According to still further features in the described preferred embodiments, the antibiotic is chloramphenicol and whereas a three-dimensional atomic structure of the segment including the nucleotides being capable of specifically associating with the chloramphenicol is defined by the set of structure coordinates corresponding to nucleotide coordinates 2044-2485 set forth in Table 3.

According to still further features in the described preferred embodiments, the antibiotic is chloramphenicol and whereas a three-dimensional atomic structure of the segment including the nucleotides being capable of specifically associating with the chloramphenicol is defined by a set of structure coordinates having a root mean square deviation of not more than 2.0 Å from the set of structure coordinates corresponding to nucleotide coordinates 2044-2485 set forth in Table 3.

According to still further features in the described preferred embodiments, the antibiotic is clindamycin and whereas a three-dimensional atomic structure of the nucleotides being capable of specifically associating with the clindamycin is defined by the set of structure coordinates corresponding to nucleotide coordinates 2040-2042, 2044, 2482, 2484 and 2590 set forth in Table 3.

According to still further features in the described preferred embodiments, the antibiotic is clindamycin and whereas a three-dimensional atomic structure of the nucleotides being capable of specifically associating with the clindamycin is defined by a set of structure coordinates having a root mean square deviation of not more than 2.0 Å from the set of structure coordinates corresponding to nucleotide coordinates 2040-2042, 2044, 2482, 2484 and 2590 set forth in Table 3.

According to still further features in the described preferred embodiments, the antibiotic is clindamycin and whereas a three-dimensional atomic structure of the segment including the nucleotides being capable of specifically associating with the clindamycin is defined by the set of structure coordinates corresponding to nucleotide coordinates 2040-2590 set forth in Table 3.

According to still further features in the described preferred embodiments, the antibiotic is clindamycin and whereas a three-dimensional atomic structure of the segment including the nucleotides being capable of specifically associating with the clindamycin is defined by a set of structure coordinates having a root mean square deviation of not more than 2.0 Å from the set of structure coordinates corresponding to nucleotide coordinates 2040-2590 set forth in Table 3.

According to still further features in the described preferred embodiments, the antibiotic is clarithromycin, erythromycin or roxithromycin, and whereas a three-dimensional atomic structure of the nucleotides being capable of specifically associating with the antibiotic is defined by the set of structure coordinates corresponding to nucleotide coordinates 2040-2042, 2045, 2484, 2588 and 2589 set forth in Table 3.

According to still further features in the described preferred embodiments, the antibiotic is clarithromycin, erythromycin or roxithromycin, and whereas a three-dimensional atomic structure of the nucleotides being capable of specifically associating with the antibiotic is defined by a set of structure coordinates having a root mean square deviation of not more than 2.0 Å from the set of structure coordinates corresponding to nucleotide coordinates 2040-2042, 2045, 2484, 2588 and 2589 set forth in Table 3.

According to still further features in the described preferred embodiments, the antibiotic is clarithromycin, erythromycin or roxithromycin, and whereas a three-dimensional atomic structure of the segment including the nucleotides being capable of specifically associating with the antibiotic is defined by the set of structure coordinates corresponding to nucleotide coordinates 2040-2589 set forth in Table 3.

According to still further features in the described preferred embodiments, the antibiotic is clarithromycin, erythromycin or roxithromycin, and whereas a three-dimensional atomic structure of the segment including the nucleotides being capable of specifically associating with the antibiotic is defined by a set of structure coordinates having a root mean square deviation of not more than 2.0 Å from the set of structure coordinates corresponding to nucleotide coordinates 2040-2589 set forth in Table 3.

According to still further features in the described preferred embodiments, the method of identifying a putative antibiotic further comprising: (i) contacting the putative antibiotic with the antibiotic-binding pocket; and (ii) detecting specific binding of the putative antibiotic to the antibiotic-binding pocket, thereby qualifying the putative antibiotic.

According to still further features in the described preferred embodiments, the method of identifying a putative antibiotic wherein step (a) is effected by co-crystallizing at least the antibiotic-binding pocket with an antibiotic.

According to still further features in the described preferred embodiments, the antibiotic-binding pocket is a clindamycin-binding pocket and whereas the structure coordinates define the three-dimensional atomic structure at a resolution higher than or equal to 3.1 Å.

According to still further features in the described preferred embodiments, the antibiotic-binding pocket is an erythromycin-binding pocket and whereas the structure coordinates define the three-dimensional atomic structure at a resolution higher than or equal to 3.4 Å.

According to still further features in the described preferred embodiments, the antibiotic-binding pocket is a clarithromycin-binding pocket and whereas the structure coordinates define the three-dimensional atomic structure at a resolution higher than or equal to 3.5 Å.

According to still further features in the described preferred embodiments, the antibiotic-binding pocket is a roxithromycin-binding pocket and whereas the structure coordinates define the three-dimensional atomic structure at a resolution higher than or equal to 3.8 Å.

According to still further features in the described preferred embodiments, the antibiotic-binding pocket is a chloramphenicol-binding pocket and whereas the structure coordinates define the three-dimensional atomic structure at a resolution higher than or equal to 3.5 Å.

According to still further features in the described preferred embodiments, the antibiotic-binding pocket is selected from the group consisting of a chloramphenicol-specific antibiotic-binding pocket, a lincosamide-specific antibiotic-binding pocket, a clindamycin-specific antibiotic-binding pocket, a macrolide antibiotic-specific antibiotic-binding pocket, a clarithromycin-specific antibiotic-binding pocket, an erythromycin-specific antibiotic-binding pocket and a roxithromycin-specific antibiotic-binding pocket.

According to still further features in the described preferred embodiments, the antibiotic comprises at least two non-covalently associated molecules.

According to still further features in the described preferred embodiments, the set of structure coordinates define the three-dimensional structure at a resolution higher than or equal to a resolution selected from the group consisting of 5.4 Å, 5.3 Å, 5.2 Å, 5.1 Å, 5.0 Å, 4.9 Å, 4.8 Å, 4.7 Å, 4.6 Å, 4.5 Å, 4.4 Å, 4.3 Å, 4.2 Å, 4.1 Å, 4.0 Å, 3.9 Å, 3.8 Å, 3.7 Å, 3.6 Å, 3.5 Å, 3.4 Å, 3.3 Å, 3.2 Å and 3.1 Å.

According to still further features in the described preferred embodiments, the antibiotic-binding pocket forms a part of a polynucleotide component of the large ribosomal subunit.

According to still further features in the described preferred embodiments, the computing platform for generating a three-dimensional model of at least a portion of a large ribosomal subunit of a eubacterium further comprising a display being for displaying the three-dimensional model generated by the processing unit.

According to still further features in the described preferred embodiments, the set of structure coordinates define the portion of a three-dimensional structure of a large ribosomal subunit at a resolution higher than or equal to a resolution selected from the group consisting of 5.4 Å, 5.3 Å, 5.2 Å, 5.1 Å, 5.0 Å, 4.9 Å, 4.8 Å, 4.7 Å, 4.6 Å, 4.5 Å, 4.4 Å, 4.3 Å, 4.2 Å, 4.1 Å, 4.0 Å, 3.9 Å, 3.8 Å, 3.7 Å, 3.6 Å, 3.5 Å, 3.4 Å, 3.3 Å, 3.2 Å and 3.1 Å.

According to still further features in the described preferred embodiments, the set of structure coordinates define the portion of a three-dimensional structure of the large ribosomal subunit at a resolution higher than or equal to 3.1 Å.

According to still further features in the described preferred embodiments, the set of structure coordinates defining at least a portion of a three-dimensional structure of the large ribosomal subunit is a set of structure coordinates corresponding to a set of coordinates set forth in Table 3, the set of coordinates set forth in Table 3 being selected from the group consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 61881-62151; atom coordinates 62152-62357; atom coordinates 62358-62555; atom coordinates 62556-62734; atom coordinates 62735-62912; atom coordinates 62913-62965; atom coordinates 62966-63109; atom coordinates 63110-63253; atom coordinates 63254-63386; atom - coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to still further features in the described preferred embodiments, the set of structure coordinates defining at least a portion of a three-dimensional structure of the large ribosomal subunit is a set of structure coordinates having a root mean square deviation of not more than 2.0 Å from a set of structure coordinates corresponding to a set of coordinates set forth in Table 3, the set of coordinates set forth in Table 3 being selected from the group consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 61881-62151; atom coordinates 62152-62357; atom coordinates 62358-62555; atom coordinates 62556-62734; atom coordinates 62735-62912; atom coordinates 62913-62965; atom coordinates 62966-63109; atom coordinates 63110-63253; atom coordinates 63254-63386; atom coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to still further features in the described preferred embodiments, computing platform for generating a three-dimensional model of at least a portion of a complex of an antibiotic and a large ribosomal subunit of a eubacterium further comprising a display being for displaying the three-dimensional model generated by the processing unit.

According to still further features in the described preferred embodiments, the antibiotic is clindamycin and whereas the set of structure coordinates define the portion of a three-dimensional structure at a resolution higher than or equal to 3.1 Å.

According to still further features in the described preferred embodiments, the antibiotic is erythromycin and whereas the set of structure coordinates define the portion of a three-dimensional structure at a resolution higher than or equal to of 3.4 Å.

According to still further features in the described preferred embodiments, the antibiotic is clarithromycin and whereas the set of structure coordinates define the portion of a three-dimensional structure at a resolution higher than or equal to 3.5 Å.

According to still further features in the described preferred embodiments, the antibiotic is roxithromycin and whereas the set of structure coordinates define the portion of a three-dimensional structure at a resolution higher than or equal to 3.8 Å.

According to still further features in the described preferred embodiments, the antibiotic is chloramphenicol and whereas the set of structure coordinates define the portion of a three-dimensional structure at a resolution higher than or equal to 3.5 Å.

According to still further features in the described preferred embodiments, he antibiotic is chloramphenicol and whereas the set of structure coordinates defining at least a portion of a three-dimensional structure of the complex of the chloramphenicol and the large ribosomal subunit corresponds to a set of coordinates selected from the group consisting of: nucleotide coordinates 2044, 2430, 2431, 2479 and 2483-2485 set forth in Table 8; nucleotide coordinates 2044-2485 set forth in Table 8; HETATM coordinates 59925-59944 set forth in Table 8; the set of atom coordinates set forth in Table 8; and the set of atom coordinates set forth in Table 13.

According to still further features in the described preferred embodiments, the antibiotic is clindamycin and whereas the set of structure coordinates defining at least a portion of a three-dimensional structure of the complex of the clindamycin and the large ribosomal subunit corresponds to a set of coordinates selected from the group consisting of: nucleotide coordinates 2040-2042, 2044, 2482, 2484 and 2590 set forth in Table 9; nucleotide coordinates 2040-2590 set forth in Table 9; HETATM coordinates 59922-59948 set forth in Table 9; the set of atom coordinates set forth in Table 9; and the set of atom coordinates set forth in Table 14.

According to still further features in the described preferred embodiments, the antibiotic is clarithromycin and whereas the set of structure coordinates defining at least a portion of a three-dimensional structure of the complex of the clarithromycin and the large ribosomal subunit corresponds to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59973 set forth in Table 10; the set of atom coordinates set forth in Table 10; and the set of atom coordinates set forth in Table 15.

According to still further features in the described preferred embodiments, the antibiotic is erythromycin and whereas the set of structure coordinates defining at least a portion of a three-dimensional structure of the complex of the erythromycin and the large ribosomal subunit corresponds to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59972 set forth in Table 11; the set of atom coordinates set forth in Table 11; and the set of atom coordinates set forth in Table 16.

According to still further features in the described preferred embodiments, the antibiotic is roxithromycin and whereas the set of structure coordinates defining at least a portion of a three-dimensional structure of the complex of the roxithromycin and the large ribosomal subunit corresponds to a set of coordinates selected from the group consisting of: nucleotide coordinates 2040-2042, 2045, 2484, 2588 and 2589 set forth in Table 12; nucleotide coordinates 2040-2589 set forth in Table 12; HETATM coordinates 59922-59979 set forth in Table 12; the set of atom coordinates set forth in Table 12; and the set of atom coordinates set forth in Table 17.

According to still further features in the described preferred embodiments, the antibiotic is chloramphenicol and whereas the set of structure coordinates defining at least a portion of a three-dimensional structure of the complex of the chloramphenicol and the large ribosomal subunit is a set of structure coordinates having a root mean square deviation of not more than 2.0 Å from a set of structure coordinates corresponding to a set of coordinates selected from the group consisting of: nucleotide coordinates 2044, 2430, 2431, 2479 and 2483-2485 set forth in Table 8; nucleotide coordinates 2044-2485 set forth in Table 8; HETATM coordinates 59925-59944 set forth in Table 8; the set of atom coordinates set forth in Table 8; and the set of atom coordinates set forth in Table 13.

According to still further features in the described preferred embodiments, the antibiotic is clindamycin and whereas the set of structure coordinates defining at least a portion of a three-dimensional structure of the complex of the clindamycin and the large ribosomal subunit is a set of structure coordinates having a root mean square deviation of not more than 2.0 Å from a set of structure coordinates corresponding to a set of coordinates selected from the group consisting of: nucleotide coordinates 2040-2042, 2044, 2482, 2484 and 2590 set forth in Table 9; nucleotide coordinates 2040-2590 set forth in Table 9; HETATM coordinates 59922-59948 set forth in Table 9; the set of atom coordinates set forth in Table 9; and the set of atom coordinates set forth in Table 14.

According to still further features in the described preferred embodiments, the antibiotic is clarithromycin and whereas the set of structure coordinates defining at least a portion of a three-dimensional structure of the complex of the clarithromycin and the large ribosomal subunit is a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59973 set forth in Table 10; the set of atom coordinates set forth in Table 10; and the set of atom coordinates set forth in Table 15.

According to still further features in the described preferred embodiments, the antibiotic is erythromycin and whereas the set of structure coordinates defining at least a portion of a three-dimensional structure of the complex of the erythromycin and the large ribosomal subunit is a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59972 set forth in Table 11; the set of atom coordinates set forth in Table 11; and the set of atom coordinates set forth in Table 16.

According to still further features in the described preferred embodiments, the antibiotic is roxithromycin and whereas the set of structure coordinates defining at least a portion of a three-dimensional structure of the complex of the roxithromycin and the large ribosomal subunit is a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 12; nucleotide coordinates 2040-2589 set forth in Table 12; HETATM coordinates 59922-59979 set forth in Table 12; the set of atom coordinates set forth in Table 12; and the set of atom coordinates set forth in Table 17.

According to still further features in the described preferred embodiments, the antibiotic is selected from the group consisting of chloramphenicol, a lincosamide antibiotic, clindamycin, a macrolide antibiotic, clarithromycin, erythromycin and roxithromycin.

According to still further features in the described preferred embodiments, the antibiotic is clindamycin and whereas the set of structure coordinates define the three-dimensional structure of the computer generated model at a resolution higher than or equal to 3.1 Å.

According to still further features in the described preferred embodiments, the antibiotic is erythromycin and whereas the set of structure coordinates define the three-dimensional structure of the computer generated model at a resolution higher than or equal to 3.4 Å.

According to still further features in the described preferred embodiments, the antibiotic is clarithromycin and whereas the set of structure coordinates define the three-dimensional structure of the computer generated model at a resolution higher than or equal to 3.5 Å.

According to still further features in the described preferred embodiments, the antibiotic is roxithromycin and whereas the set of structure coordinates define the three-dimensional structure of the computer generated model at a resolution higher than or equal to 3.8 Å.

According to still further features in the described preferred embodiments, the antibiotic is chloramphenicol and whereas the set of structure coordinates define the three-dimensional structure of the computer generated model at a resolution higher than or equal to 3.5 Å.

According to still further features in the described preferred embodiments, the antibiotic is chloramphenicol and whereas a three-dimensional atomic structure of the portion of a complex of the chloramphenicol and the large ribosomal subunit is defined by a set of structure coordinates corresponding to a set of coordinates selected from the group consisting of: nucleotide coordinates 2044, 2430, 2431, 2479 and 2483-2485 set forth in Table 8; nucleotide coordinates 2044-2485 set forth in Table 8; HETATM coordinates 59925-59944 set forth in Table 8; the set of atom coordinates set forth in Table 8; and the set of atom coordinates set forth in Table 13.

According to still further features in the described preferred embodiments, the antibiotic is clindamycin and whereas a three-dimensional atomic structure of the portion of a complex of the clindamycin and the large ribosomal subunit is defined by a set of structure coordinates corresponding to a set of coordinates selected from the group consisting of: nucleotide coordinates 2040-2042, 2044, 2482, 2484 and 2590 set forth in Table 9; nucleotide coordinates 2040-2590 set forth in Table 9; HETATM coordinates 59922-59948 set forth in Table 9; the set of atom coordinates set forth in Table 9; and the set of atom coordinates set forth in Table 14.

According to still further features in the described preferred embodiments, the antibiotic is clarithromycin and whereas a three-dimensional atomic structure of the portion of a complex of the clarithromycin and the large ribosomal subunit is defined by a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59973 set forth in Table 10; the set of atom coordinates set forth in Table 10; and the set of atom coordinates set forth in Table 15.

According to still further features in the described preferred embodiments, the antibiotic is erythromycin and whereas a three-dimensional atomic structure of the portion of a complex of the erythromycin and the large ribosomal subunit is defined by a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59972 set forth in Table 11; the set of atom coordinates set forth in Table 11; and the set of atom coordinates set forth in Table 16.

According to still further features in the described preferred embodiments, the antibiotic is roxithromycin and whereas a three-dimensional atomic structure of the portion of a complex of the roxithromycin and the large ribosomal subunit is defined by a set of structure coordinates corresponding to a set of coordinates selected from the group consisting of: nucleotide coordinates 2040-2042, 2045, 2484, 2588 and 2589 set forth in Table 12; nucleotide coordinates 2040-2589 set forth in Table 12; HETATM coordinates 59922-59979 set forth in Table 12; the set of atom coordinates set forth in Table 12; and the set of atom coordinates set forth in Table 17.

According to still further features in the described preferred embodiments, the antibiotic is chloramphenicol and whereas a three-dimensional atomic structure of the portion of a complex of the chloramphenicol and the large ribosomal subunit is defined by a set of structure coordinates having a root mean square deviation of not more than 2.0 Å from a set of structure coordinates corresponding to a set of coordinates selected from the group consisting of: nucleotide coordinates 2044, 2430, 2431, 2479 and 2483-2485 set forth in Table 8; nucleotide coordinates 2044-2485 set forth in Table 8; HETATM coordinates 59925-59944 set forth in Table 8; the set of atom coordinates set forth in Table 8; and the set of atom coordinates set forth in Table 13.

According to still further features in the described preferred embodiments, the antibiotic is clindamycin and whereas a three-dimensional atomic structure of the portion of a complex of the clindamycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2044, 2482, 2484 and 2590 set forth in Table 9; nucleotide coordinates 2040-2590 set forth in Table 9; HETATM coordinates 59922-59948 set forth in Table 9; the set of atom coordinates set forth in Table 9; and the set of atom coordinates set forth in Table 14.

According to still further features in the described preferred embodiments, the antibiotic is clarithromycin and whereas a three-dimensional atomic structure of the portion of a complex of the clarithromycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59973 set forth in Table 10; the set of atom coordinates set forth in Table 10; and the set of atom coordinates set forth in Table 15.

According to still further features in the described preferred embodiments, the antibiotic is erythromycin and whereas a three-dimensional atomic structure of the portion of a complex of the erythromycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59972 set forth in Table 11; the set of atom coordinates set forth in Table 11; and the set of atom coordinates set forth in Table 16.

According to still further features in the described preferred embodiments, the antibiotic is roxithromycin and whereas a three-dimensional atomic structure of the portion of a complex of the roxithromycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 12; nucleotide coordinates 2040-2589 set forth in Table 12; HETATM coordinates 59922-59979 set forth in Table 12; the set of atom coordinates set forth in Table 12; and the set of atom coordinates set forth in Table 17.

According to still further features in the described preferred embodiments, the set of structure coordinates define the portion of a three-dimensional structure of a crystallized large ribosomal subunit at a resolution higher than or equal to a resolution selected from the group consisting of 5.4 Å, 5.3 Å, 5.2 Å, 5.1 Å, 5.0 Å, 4.9 Å, 4.8 Å, 4.7 Å, 4.6 Å, 4.5 Å, 4.4 Å, 4.3 Å, 4.2 Å, 4.1 Å, 4.0 Å, 3.9 Å, 3.8 Å, 3.7 Å, 3.6 Å, 3.5 Å, 3.4 Å, 3.3 Å, 3.2 Å and 3.1 Å.

According to still further features in the described preferred embodiments, the set of structure coordinates define the portion of a three-dimensional structure of a crystallized large ribosomal subunit at a resolution higher than or equal to 3.1 Å.

According to still further features in the described preferred embodiments, the set of structure coordinates defining at least a portion of a three-dimensional structure of a large ribosomal subunit is a set of structure coordinates corresponding to a set of coordinates set forth in Table 3, the set of coordinates set forth in Table 3 being selected from the group consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 61881-62151; atom coordinates 62152-62357; atom coordinates 62358-62555; atom coordinates 62556-62734; atom coordinates 62735-62912; atom coordinates 62913-62965; atom coordinates 62966-63109; atom coordinates 63110-63253; atom coordinates 63254-63386; atom coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to still further features in the described preferred embodiments, the structure coordinates defining at least a portion of a three-dimensional structure of a crystallized large ribosomal subunit have a root mean square deviation of not more than 2.0 Å from a set of structure coordinates corresponding to a set of coordinates set forth in Table 3, the set of coordinates set forth in Table 3 being selected from the group consisting of: nucleotide coordinates 2044, 2430, 2431, 2479 and 2483-2485; nucleotide coordinates 2044-2485; nucleotide coordinates 2040-2042, 2044, 2482, 2484 and 2590; nucleotide coordinates 2040-2590; nucleotide coordinates 2040-2042, 2045, 2484, 2588 and 2589; nucleotide coordinates 2040-2589; atom coordinates 1-59360; atom coordinates 59361-61880; atom coordinates 1-61880; atom coordinates 6181-62151; atom coordinates 62152-62357; atom coordinates 62358-62555; atom coordinates 62556-62734; atom coordinates 62735-62912; atom coordinates 62913-62965; atom coordinates 62966-63109; atom coordinates 63110-63253; atom coordinates 63254-63386; atom coordinates 63387-63528; atom coordinates 63529-63653; atom coordinates 63654-63768; atom coordinates 63769-63880; atom coordinates 63881-64006; atom coordinates 64007-64122; atom coordinates 64123-64223; atom coordinates 64224-64354; atom coordinates 64355-64448; atom coordinates 64449-64561; atom coordinates 64562-64785; atom coordinates 64786-64872; atom coordinates 64873-64889; atom coordinates 64890-64955; atom coordinates 64956-65011; atom coordinates 65012-65085; atom coordinates 65086-65144; atom coordinates 65145-65198; atom coordinates 65199-65245; atom coordinates 65246-65309; atom coordinates 65310-65345; atom coordinates 61881-65345; and atom coordinates 1-65345.

According to still further features in the described preferred embodiments, the antibiotic is chloramphenicol and whereas the three-dimensional structure of a crystallized complex of an antibiotic and a large ribosomal subunit is defined by a set of structure coordinates corresponding to a set of coordinates selected from the group consisting of: nucleotide coordinates 2044, 2430, 2431, 2479 and 2483-2485 set forth in Table 8; nucleotide coordinates 2044-2485 set forth in Table 8; HETATM coordinates 59925-59944 set forth in Table 8; the set of atom coordinates set forth in Table 8; and the set of atom coordinates set forth in Table 13.

According to still further features in the described preferred embodiments, the antibiotic is clindamycin and whereas the three-dimensional structure of a crystallized complex of an antibiotic and a large ribosomal subunit is defined by a set of structure coordinates corresponding to a set of coordinates selected from the group consisting of: nucleotide coordinates 2040-2042, 2044, 2482, 2484 and 2590 set forth in Table 9; nucleotide coordinates 2040-2590 set forth in Table 9; HETATM coordinates 59922-59948 set forth in Table 9; the set of atom coordinates set forth in Table 9; and the set of atom coordinates set forth in Table 14.

According to still further features in the described preferred embodiments, the antibiotic is clarithromycin and whereas the three-dimensional structure of a crystallized complex of an antibiotic and a large ribosomal subunit is defined by a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59973 set forth in Table 10; the set of atom coordinates set forth in Table 10; and the set of atom coordinates set forth in Table 15.

According to still further features in the described preferred embodiments, the antibiotic is erythromycin and whereas the three-dimensional structure of a crystallized complex of an antibiotic and a large ribosomal subunit is defined by a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59972 set forth in Table 11; the set of atom coordinates set forth in Table 11; and the set of atom coordinates set forth in Table 16.

According to still further features in the described preferred embodiments, the antibiotic is roxithromycin and whereas the three-dimensional structure of a crystallized complex of an antibiotic and a large ribosomal subunit is defined by a set of structure coordinates corresponding to a set of coordinates selected from the group consisting of: nucleotide coordinates 2040-2042, 2045, 2484, 2588 and 2589 set forth in Table 12; nucleotide coordinates 2040-2589 set forth in Table 12; HETATM coordinates 59922-59979 set forth in Table 12; the set of atom coordinates set forth in Table 12; and the set of atom coordinates set forth in Table 17.

According to still further features in the described preferred embodiments, the antibiotic is chloramphenicol and whereas the three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2044, 2430, 2431, 2479 and 2483-2485 set forth in Table 8; nucleotide coordinates 2044-2485 set forth in Table 8; HETATM coordinates 59925-59944 set forth in Table 8; the set of atom coordinates set forth in Table 8; and the set of atom coordinates set forth in Table 13.

According to still further features in the described preferred embodiments, the antibiotic is clindamycin and whereas the three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2044, 2482, 2484 and 2590 set forth in Table 9; nucleotide coordinates 2040-2590 set forth in Table 9; HETATM coordinates 59922-59948 set forth in Table 9; the set of atom coordinates set forth in Table 9; and the set of atom coordinates set forth in Table 14.

According to still further features in the described preferred embodiments, the antibiotic is clarithromycin and whereas the three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59973 set forth in Table 10; the set of atom coordinates set forth in Table 10; and the set of atom coordinates set forth in Table 15.

According to still further features in the described preferred embodiments, the antibiotic is erythromycin and whereas the three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59972 set forth in Table 11; the set of atom coordinates set forth in Table 11; and the set of atom coordinates set forth in Table 16.

According to still further features in the described preferred embodiments, the antibiotic is roxithromycin and whereas the three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 12; nucleotide coordinates 2040-2589 set forth in Table 12; HETATM coordinates 59922-59979 set forth in Table 12; the set of atom coordinates set forth in Table 12; and the set of atom coordinates set forth in T

According to still further features in the described preferred embodiments, the eubacterium is *D. radiodurans.*

According to still further features in the described preferred embodiments, the eubacterium is a gram-positive bacterium.

According to still further features in the described preferred embodiments, the eubacterium is a coccus.

According to still further features in the described preferred embodiments, the eubacterium is a Deinococcus-Thermophilus group bacterium.

According to still further features in the described preferred embodiments, the volatile component is an alcohol component.

According to still further features in the described preferred embodiments, the volatile component comprises at least one monovalent alcohol and at least one polyvalent alcohol.

According to still further features in the described preferred embodiments, the volumetric ratio of the at least one multivalent alcohol to the at least one monovalent alcohol is selected from the range consisting of 1:3.0-1:4.1.

According to still further features in the described preferred embodiments, the volumetric ratio of the at least one multivalent alcohol to the at least one monovalent alcohol is 1:3.5.

According to still further features in the described preferred embodiments, the at least one monovalent alcohol is ethanol.

According to still further features in the described preferred embodiments, the at least one polyvalent alcohol is dimethylhexandiol.

According to still further features in the described preferred embodiments, the first concentration is selected from a range consisting of 0.1-10 % (v/v).

According to still further features in the described preferred embodiments, the fraction is selected from a range consisting of 0.33-0.67.

According to still further features in the described preferred embodiments, the fraction is 0.5.

According to still further features in the described preferred embodiments, the buffer component is an optimal buffer for the functional activity of the large ribosomal subunit.

According to still further features in the described preferred embodiments, the buffer component is an aqueous solution comprising:
MgCl₂ in such a quantity as to yield a final concentration of the MgCl₂ in the crystallization solution, the equilibration solution, or both selected from a range consisting of 3-12 mM;
NH₄Cl in such a quantity as to yield a final concentration of the NH₄Cl in the crystallization solution, the equilibration solution, or both selected from a range consisting of 20-70 mM;
KCl in such a quantity as to yield a final concentration of the KCl in the crystallization solution, the equilibration solution, or both selected from a range consisting of 0-15 mM; and
HEPES in such a quantity as to yield a final concentration of the HEPES in the crystallization solution, the equilibration solution, or both selected from a range consisting of 8-20 mM.

According to still further features in the described preferred embodiments, the crystallization solution, the equilibration solution, or both have a pH selected from the range consisting of 6.0-9.0 pH units.

According to still further features in the described preferred embodiments, the equilibrating is effected by vapor diffusion.

According to still further features in the described preferred embodiments, the equilibrating is effected at a temperature selected from a range consisting of 15-25 °C.

According to still further features in the described preferred embodiments, the equilibrating is effected at a temperature selected from a range consisting of 17-20 °C.

According to still further features in the described preferred embodiments, the equilibrating is effected using a hanging drop of the crystallization mixture.

According to still further features in the described preferred embodiments, the equilibrating is effected using Linbro dishes.

According to still further features in the described preferred embodiments, the crystallization solution, the equilibration solution, or both comprise 10 mM MgCl₂, 60 mM NH₄Cl, 5 mM KCl and 10 mM HEPES..

According to still further features in the described preferred embodiments, the crystallization solution, the equilibration solution, or both, have a pH of 7.8.

According to still further features in the described preferred embodiments, the crystallization solution comprises an antibiotic.

According to still further features in the described preferred embodiments, the antibiotic is selected from the group consisting of chloramphenicol, a lincosamide antibiotic, clindamycin, a macrolide antibiotic, erythromycin and roxithromycin.

According to still further features in the described preferred embodiments, the crystallization solution comprises the antibiotic at a concentration selected from the range consisting of 0.8-3.5 mM.

According to still further features in the described preferred embodiments, the method of crystallizing a large ribosomal subunit of a eubacterium further comprises soaking the crystallized ribosomal subunit in a soaking solution containing an antibiotic.

According to still further features in the described preferred embodiments, the antibiotic is clarithromycin.

According to still further features in the described preferred embodiments, the soaking solution comprises the antibiotic at a concentration selected from the range consisting of 0.004-0.025 mM.

According to still further features in the described preferred embodiments, the soaking solution comprises the antibiotic at a concentration of 0.01 mM.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

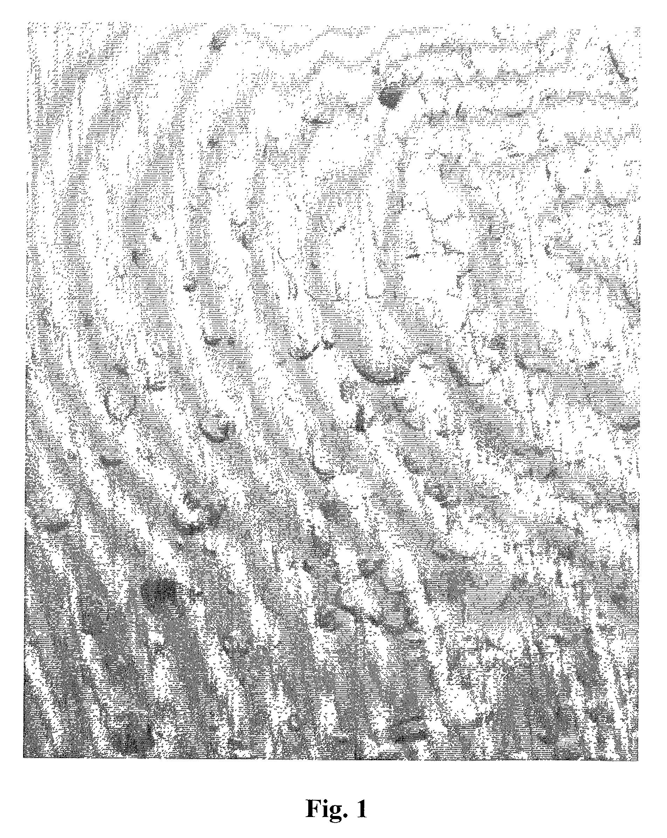

FIG. 1 is a photograph depicting D50S crystals grown according to the teachings of the present invention.

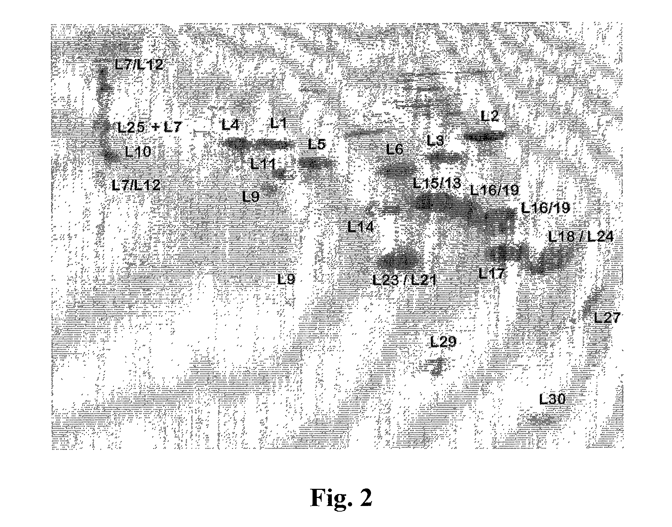

FIG. 2 is a photograph depicting 2D polyacrylamide gel electrophoretic separation and identification of D50S proteins.

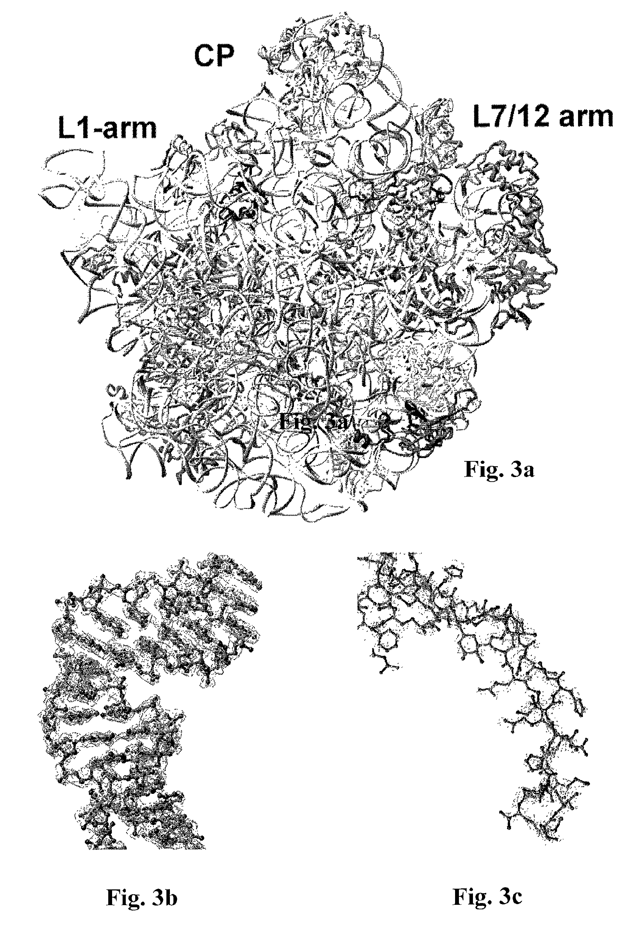

FIGs. 3a-c are atomic structure diagrams depicting a crown view representation of the D50S structure, shown from the side facing the small subunit within the 70S particle (Figure 3a). The RNA chains are shown as ribbons (in cyan) and the proteins main chains in different colors. For orientation, the L12 stalk is on the right, the L1 stalk is on the left, and the central protuberance (CP), including the 5S rRNA is in the middle of the upper part of the particle. Figures 3b and 3c depict typical map segments of rRNA helices and proteins, respectively.

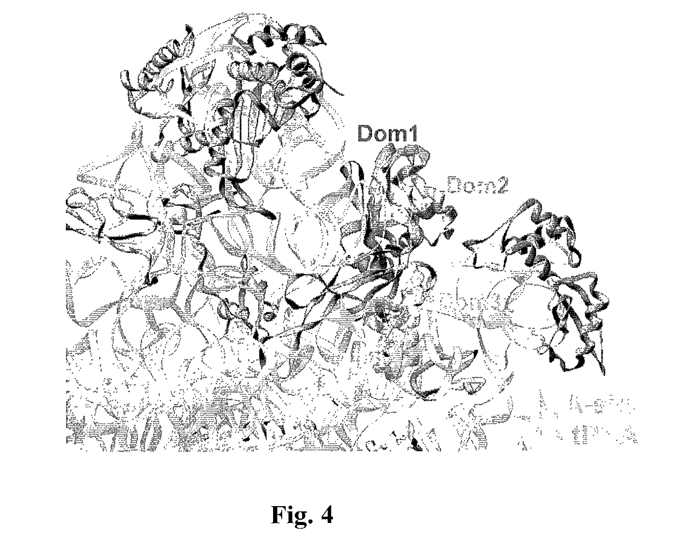

FIG. 4 is an atomic structure diagram depicting the location of protein CTC and its domain organization. CTC is shown in colored ribbons on the upper part of the D50S structure, shown in gray ribbons in the orientation of Figure 3a. The N-terminal domain (Dom1) is located at the solvent side, shown in this figure behind the CP. The middle domain (Dom2) wraps around the CP and fills the gap extending to the L11 arm. The C-terminal domain (Dom3) is located at the rim of the intersubunit interface and reaches the site of docked A-site tRNA position (marked by a star).

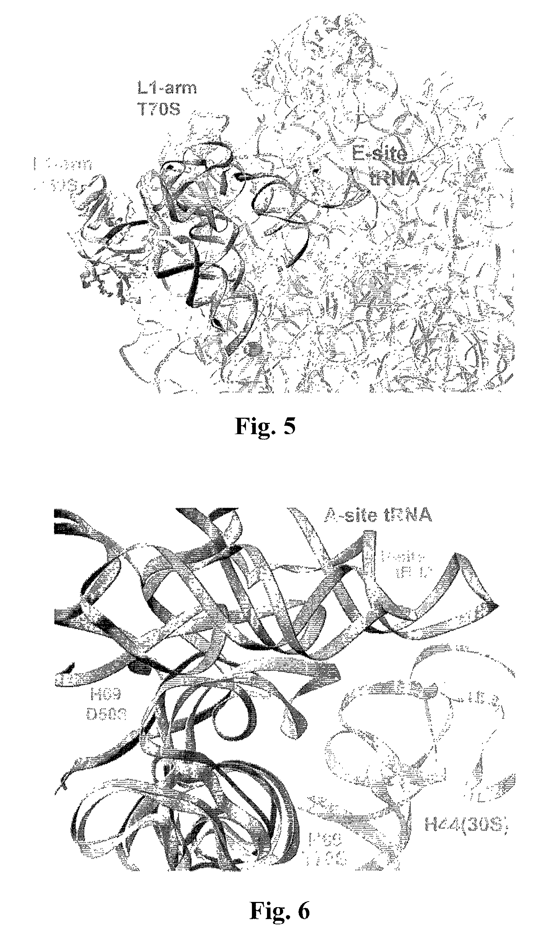

FIG. 5 is an atomic structure diagram depicting the D50S Li-arm and its possible rotation. The adjacent part of the D50S structure is shown in gray, the L1-arm of D50S is highlighted in gold, and also shown is the L1-arm of the T70S structure in green. In T70S the L1-arm and protein L1 block the exit of the E-tRNA (magenta). Whereas, in the D50S structure, the L1-arm is displaced by about 30 Å, and can also rotate around a pivot point (marked by a red dot) by about 30°, thus clearing the E-tRNA exit.
FIG. 6 is an atomic structure diagram depicting the intersubunit bridge to the decoding side of D50S. Shown is an overlay of H69 of D50S (cyan) and the corresponding feature in the structure of the T70S ribosome (gold). The figure indicates the proposed movement of H69 towards the decoding center of H44 (gray) in T30S.

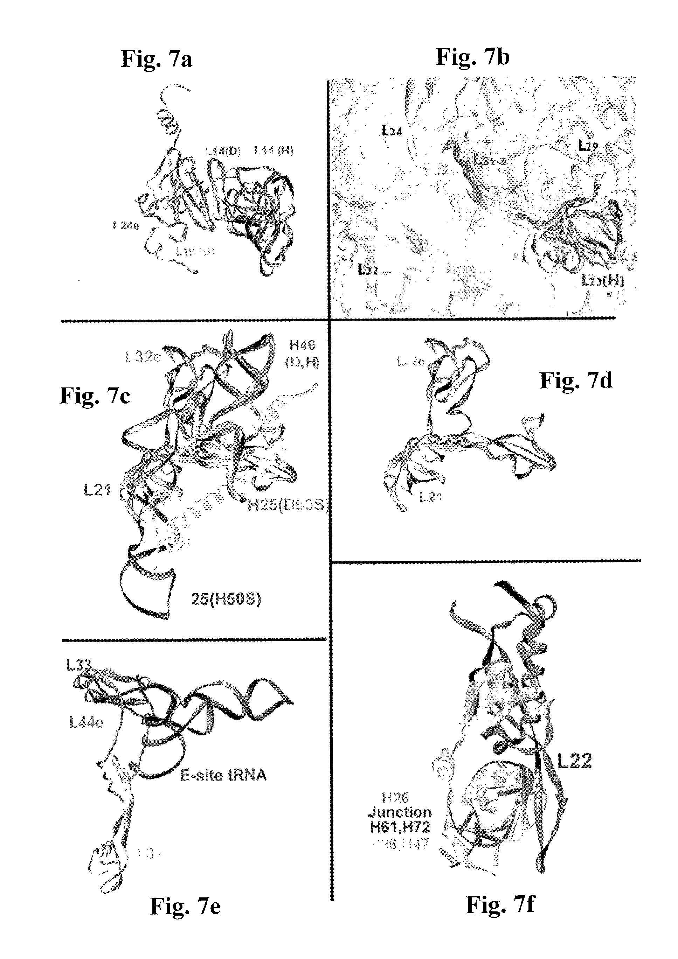

FIGs. 7a-f are atomic structure diagrams depicting novel structural features identified in D50S. Figure 7a depicts the inter-protein β-sheet, made by proteins L14-L19 in D50S, overlaid on the H50S counterparts, L14 and HL24e. Note the differences in structure and size between L19 in D50S to HL24e. Figure 7b depicts the opening of the nascent polypeptide tunnel. The D50S protein L23 (gold) and its substitutes in H50S, L29e (red) and the HL23 (purple), are highlighted. Figure 7c depicts an overlay of H25 in D50S (blue) and in H50S (red). In D50S H25 is significantly shorter, and proteins L20 (yellow) and L21 (green) are attached to it. Their space is occupied by part of the long helix of H25 in H50S. Figure 7d depicts isolated views of L21 (green) and L23e (gray) that are related by an approximate 2-fold and which display similar extensions. Figure 7e depicts an overlay of D50S protein L33 (purple) and H50S protein L44e (green) which shows similar globular domain folds in both proteins, but no extension for L33. Part of the space of the H50S L44e loop is occupied by the extension of D50S L31 (yellow). The E-site tRNA (red) is shown interacting with D50S L33 and H50S L44e and D50S L31 (gold) loop. Figure 7f depicts a tweezers-like structure formed by proteins L32 (gold) and L22 (red), presumably stabilizing a helical structure generated from three RNA domains: H26 (green), the junction H61, H72 (blue) and the junction H26, H47 (cyan).

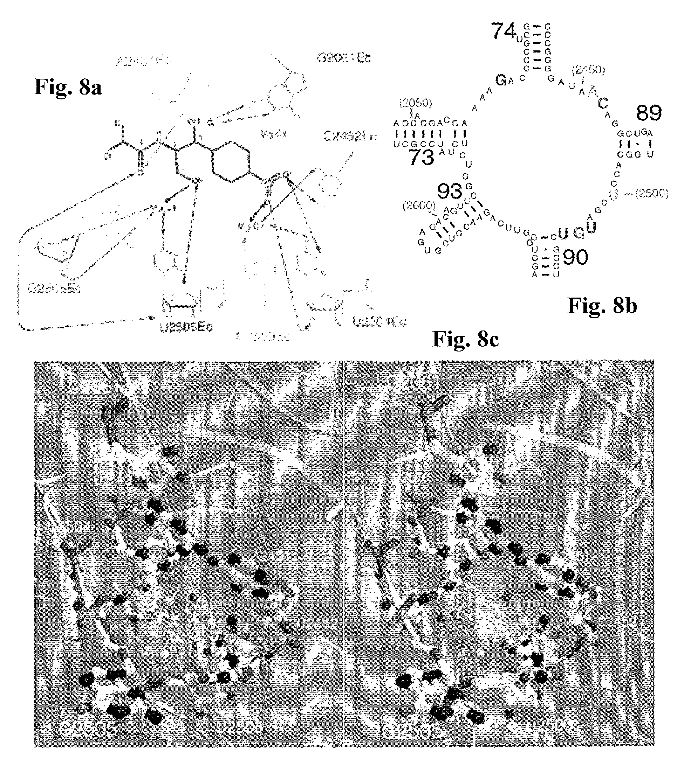

FIGs. 8a-c are structure diagrams depicting interaction of chloramphenicol with the peptidyl transferase cavity of D50S. Figure 8a is a chemical structure diagram depicting interaction of chloramphenicol with 23S rRNA nucleotides in the peptidyl transferase cavity. Arrows depict interacting chemical moieties positioned < 4.5 Å apart. Figure 8b is a diagram depicting the secondary structure of the peptidyl transferase ring of *D. radiodurans* 23S rRNA, showing nucleotides (colored) interacting with chloramphenicol. Matching nucleotide color-coding schemes are used in Figures 8a and 8b.
Figure 8c is a stereo diagram depicting chloramphenicol binding sites in the peptidyl transferase cavity. The difference electron density map (2Fo-Fc) is contoured at 1.2 sigma. Chloramphenicol and portions of 23S rRNA which do not interact therewith are depicted in green and blue, respectively, and 23S rRNA nucleotides interacting with chloramphenicol are shown in the form of chemical structure models. Nucleotide numbering is according to the *E. coli* sequence. Mg²⁺ ions are indicated (Mg).

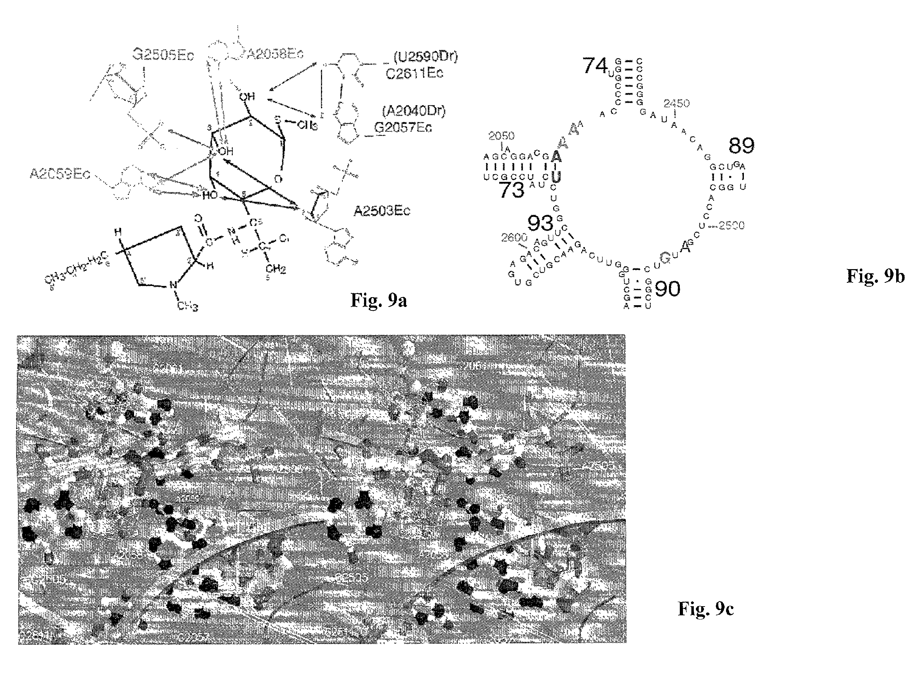

FIGs. 9a-c are structure diagrams depicting interaction of clindamycin with the peptidyl transferase cavity of D50S. Figure 9a is a chemical structure diagram depicting interaction of clindamycin with 23S rRNA nucleotides in the peptidyl transferase cavity. Arrows depict interacting chemical moieties positioned < 4.5 Å apart. Figure 9b is a diagram depicting the secondary structure of the peptidyl transferase ring of D50S 23S rRNA showing nucleotides (colored) interacting with clindamycin. Matching nucleotide color-coding schemes are used in Figures 9a and 9b. Figure 9c is a stereo diagram depicting clindamycin binding sites in the peptidyl transferase cavity. The difference electron density map (2Fo-Fc) is contoured at 1.2 sigma. Clindamycin and portions of 23S rRNA which do not interact therewith are depicted in green and blue, respectively, and 23S rRNA nucleotides interacting with clindamycin are shown as chemical structure models. Nucleotide numbering is according to the *E. coli* sequence.

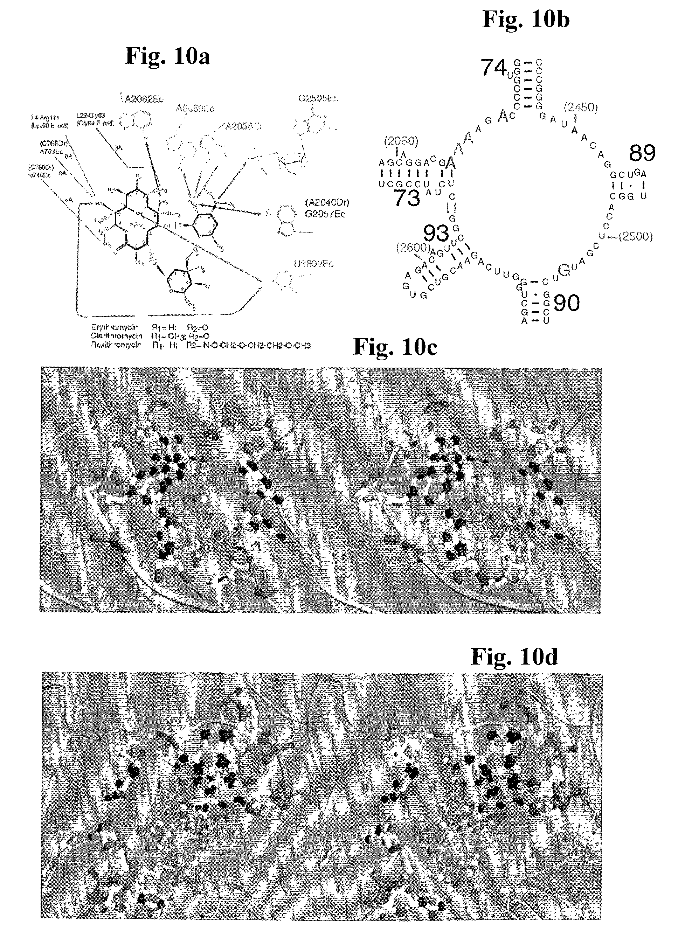

FIGs. 10a-d are structure diagrams depicting interaction of the macrolide antibiotics erythromycin, clarithromycin and roxithromycin with the peptidyl transferase cavity of D50S. Figure 10a is a chemical structure diagram depicting the interactions (colored arrows) of the reactive groups of the macrolides with the nucleotides of the peptidyl transferase cavity (colored). Colored arrows between two chemical moieties indicate that the two groups are less than 4.5 Å apart. Groups previously implicated in antibiotic interactions, namely proteins L4, L22, and domain II of the 23S rRNA are shown in black with their corresponding distances to the macrolide moieties. Figure 10b is a diagram depicting secondary structure of the peptidyl transferase ring of D50S showing the nucleotides involved in the interaction with clindamycin (colored nucleotides). Matching nucleotide color-coding schemes are used in Figures 10a and 10b. Figure 10c is a stereo diagram depicting the erythromycin binding site at the entrance of the tunnel of D50S. The stereo view of clarithromycin is identical to that of erythromycin. Figure 10d is a stereo diagram depicting the roxithromycin binding site at the entrance of the tunnel of D50S. In Figures 10c and 10d, the difference electron density map (2Fo-Fc) is contoured at 1.2 sigma. Green, antibiotic; blue, 23S rRNA; yellow, part of ribosomal protein L4; light green, part of ribosomal protein L22. Nucleotides that interact with the antibiotic are shown with their chemical structure. Nucleotide numbering is according to the *E. coli* sequence.

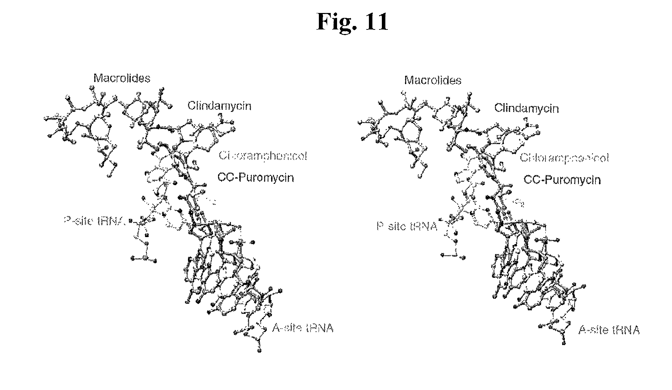

FIG. 11 is a stereo diagram depicting the relative positions of chloramphenicol, clindamycin, and macrolides with respect to CC-puromycin and the 3'-CA end of P-site and A-site tRNAs. The location of CC-puromycin was obtained by docking the previously reported position thereof (Nissen, P. *et al.* (2000) Science 289:920) into the peptidyl transferase center of DS0S The location of the 3'-CA end of P- and A-site tRNAs were obtained by docking the previously reported position (Yusupov, MM. *et al.* (2001) Science 292:883) into the peptidyl transferase center of D50S. Light blue, 3'-CA end of A-site tRNA; light yellow, 3' CA end of P-site tRNA; gray, puromycin; gold, chloramphenicol; green, clindamycin; cyan, macrolides (erythromycin). Oxygen atoms are shown in red and nitrogen atoms in dark blue.

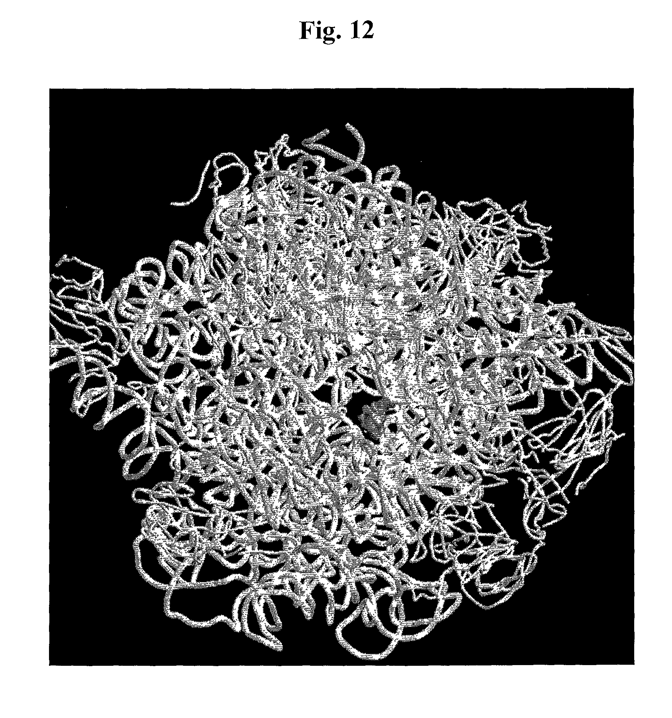

FIG. 12 is an atomic structure diagram depicting the view of D50S from the 30S side showing erythromycin (red) bound at the entrance of the tunnel. Yellow, ribosomal proteins; gray, 23S rRNA; dark gray, 5S rRNA.

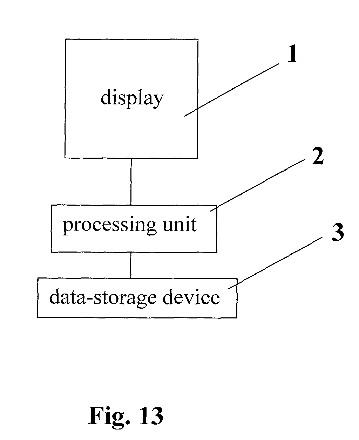

FIG. 13 is a schematic diagram depicting a computing platform for generating a three-dimensional model of at least a portion of a LRS or of a complex of an antibiotic and a LRS.

### DESCRIPTION OF THE PREFERRED EMBODIMENTS

The present invention is of a crystallized large ribosomal subunit (LRS) or a crystallized co-complex of the LRS and an antibiotic, compositions-of-matter comprising such crystals and methods of using structural data derived from such crystals for generating three-dimensional (3D) models of the LRS or LRS-antibiotic complex, which models can be used for rational design or identification of novel antibiotics and LRSs having desired characteristics.

The principles and operation of the present invention may be better understood with reference to the accompanying descriptions.

Before explaining at least one embodiment of the invention in detail, it is to be understood that the invention is not limited in its application to the details of construction and the arrangement of the components set forth in the following description or exemplified in the Examples. The invention is capable of other embodiments or of being practiced or carried out in various ways. Also, it is to be understood that the phraseology and terminology employed herein is for the purpose of description and should not be regarded as limiting.

For the past few years, the rational design or identification of novel antibiotics has gained importance mostly due to the emergence of pathogenic bacterial strains resistant to known antibiotics.

One major binding target of antibiotics is the LRS, the universal and central macromolecular catalyst of protein synthesis. The LRS has been the center of numerous studies due to its pivotal role in protein synthesis and antibiotic therapy.

Various approaches for generating 3D atomic structure models of free or antibiotic complexed ribosomal subunits have been described by the prior art.

For example, attempts to generate structure models of the LRS of the eubacterium *E. coli* have failed since this molecule is too fragile to generate X-ray crystallography grade crystals.

Information derived from attempts to determine the structure of the 30S subunit from the thermophilic bacterium *Thermus thermophilus* (*T. thermophilus*) (T30S) alone or in complex with various combinations of RNA molecules, initiation factors and small ribosomal subunit-specific antibiotics can not be used for modeling free or antibiotic complexed LRSs.

Approaches attempting to determine the structure of the *T. thermophilus* 70S ribosomal particle (T70S) in complex with mRNA and tRNA have failed to yield structures at resolutions higher than 5.5 Å nor have these provided structure models of the LRS in complex with an antibiotic molecule.

Attempts to determine the structure of the LRS of the archaea *Haloarcula marismortui* (*H. marismortui*) have not provided satisfactory coverage of the structural features involved in the non-catalytic functional aspects of protein biosynthesis and have not provided structures of this subunit in complex with a bound antibiotic molecule. Furthermore, there are significant differences between such archaeal LRS and eubacterial LRSs, the latter being of incomparably greater significance, scientifically or industrially, than the former. Archaeal ribosomes have not only bacterial but also eukaryotic properties and are therefore less suitable as eubacterial models.

Thus, all prior art approaches have failed to provide satisfactory 3D atomic structure models of free or antibiotic complexed eubacterial LRSs.

While reducing the present invention to practice, the present inventors have generated an essentially complete high resolution 3D atomic structure model of a eubacterial LRS, and high resolution 3D atomic structure models of the eubacterial LRS in complex with a range of antibiotics.

As used herein, an "essentially complete" structure of a LRS refers to a high resolution structure whose RNA component is at least 96 % complete and which includes the features involved in both catalytic and non-catalytic functional aspects of protein biosynthesis at high resolution.

As used herein, the term "high resolution" refers to a resolution higher than or equal to 5.4 Å.

As described in Example 1 of the Examples section below, an essentially complete 3D atomic structure model of a free LRS at a resolution of 3.1 Å was generated for the first time. Due to its high resolution, this model is superior to all prior art LRS models. Furthermore, this model is also superior to all prior art LRS models in that it represents the most complete bacterial, including archaeal, LRS model generated at a resolution of 3.1 Å, or higher. In addition, as is shown in Example 2 of the Examples section which follows, 3D atomic structure models of the interaction between the LRS and a range of antibiotics were also generated at resolutions as high as 3.1 Å. These represent the first 3D atomic structure models of the interaction between LRSs and antibiotics.

Such novel and highly resolved crystallography data, which were obtained using the crystallography method of the present invention, represents a breakthrough of historical proportions in structure determination of free and antibiotic-complexed LRSs (Examples 1 and 2, respectively) and, as such, these data have been recently published, after the earliest priority date of this application, in both Cell and Nature (Harms J. *et al.* (2001) Cell 107:679; and Schlunzen F. *et al.* (2001) Nature 413:814).

Due to the completeness and highly resolved nature of the data obtained, the models of the present invention constitute a unique and powerful tool capable of greatly facilitating the rational design or identification of LRS-targeting antibiotics or of LRSs having desired characteristics, and of providing profound insights into the crucial and universal mechanisms of protein production which are performed by the ribosome.

Thus, according to one aspect of the present invention there are provided compositions including crystallized eubacterial LRSs.

According to one embodiment, such compositions are crystallized free LRSs.

As used herein, the term "free LRSs" refers to LRSs which are not complexed with an antibiotic.

The crystallized free LRSs of the present invention are suitable for generating, preferably via X-ray crystallography, coordinate data defining the high resolution 3D atomic structure of essentially complete crystallized free LRSs, or portions of crystallized free LRSs, as shown in Example 1 of the Examples section, below.

X-ray crystallography is effected by exposing crystals to an X-ray beam and collecting the resultant X-ray diffraction data. This process usually involves the measurements of many tens of thousands of data points over a period of one to several days depending on the crystal form and the resolution of the data required. The crystals diffract the rays, creating a geometrically precise pattern of spots recorded on photographic film or electronic detectors. The distribution of atoms within the crystal influences the pattern of spots. The quality of protein crystals is determined by the ability of the crystal to scatter X-rays of wavelengths (typically 1.0-1.6 Å) suitable to determine the atomic coordinates of the macromolecule. The measure of the quality is determined as a function of the highest angle of scatter (the ultimate or intrinsic resolution) and according to Bragg's Law: nλ = 2d sinθ (where θ is the angle of incidence of the reflected X-ray beam, d is the distance between atomic layers in a crystal, λ is the wavelength of the incident X-ray beam, and *n* is an integer), *d* may be determined, and represents the resolution of the crystal form in angstroms. Thus, this measurement is routinely used to judge the ultimate usefulness of protein crystals. Group theory shows that there are 230 unique ways in which chemical substances, proteins or otherwise, may assemble in 3D to form crystals. These are called the 230 "space groups." The designation of the space group in addition to the unit cell constants (which define the explicit size and shape of the cell which repeats periodically within the crystal) is routinely used to uniquely identify a crystalline substance. Certain conventions have been established to ensure the proper identification of crystalline materials and these conventions have been set forth and documented in the International Tables for Crystallography, incorporated herein by reference.

The crystallized free LRSs of the present invention can be used to generate coordinate data defining essentially complete 3D atomic structures of crystallized free LRSs, or 3D atomic structures of portions of crystallized free LRSs, at a resolution preferably higher than or equal to 5.4 Å, more preferably higher than or equal to 5.3 Å, more preferably higher than or equal to 5.2 Å, more preferably higher than or equal to 5.1 Å, more preferably higher than or equal to 5.0 Å, more preferably higher than or equal to 4.9 Å, more preferably higher than or equal to 4.8 Å, more preferably higher than or equal to 4.7 Å, more preferably higher than or equal to 4.6 Å, more preferably higher than or equal to 4.5 Å, more preferably higher than or equal to 4.4 Å, more preferably higher than or equal to 4.3 Å, more preferably higher than or equal to 4.2 Å, more preferably higher than or equal to 4.1 Å, more preferably higher than or equal to 4.0 Å, more preferably higher than or equal to 3.9 Å, more preferably higher than or equal to 3.8 Å, more preferably higher than or equal to 3.7 Å, more preferably higher than or equal to 3.6 Å, more preferably higher than or equal to 3.5 Å, more preferably higher than or equal to 3.4 Å, more preferably higher than or equal to 3.3 Å, more preferably higher than or equal to 3.2 Å, and most preferably higher than or equal to 3.1 Å, as shown in Example 1 of the Examples section, below.

Thus, the present invention provides coordinate data which define the 3D atomic structure of essentially whole crystallized free LRSs, or components thereof, at resolutions as high as 3.1 Å.

As used herein, the term "3D atomic structure" refers to the positioning and structure of atoms or groups of atoms, including sets of atoms or sets of groups of atoms which are not directly associated with each other such as, for example, sets of non-contiguous nucleotides from the same polynucleotide molecule.

Those of ordinary skill in the art will understand that a set of atomic structure coordinates is a relative set of points that define a shape in three dimensions. Thus, it is possible that a different set of coordinates, for example a set of coordinates utilizing a different frame of reference and/or different units, could define a similar or identical shape. Moreover, it will be understood that slight variations in the individual coordinates will have little effect on overall shape.

The variations in coordinates discussed above may be generated because of mathematical manipulations of the structure coordinates. For example, structure coordinates can be manipulated by crystallographic permutations of the structure coordinates, fractionalization of the structure coordinates, integer additions or subtractions to sets of the structure coordinates, inversion of the structure coordinates or any combination of the above. Alternatively, modifications in the crystal structure due to mutations, additions, substitutions, or other changes in any of the components that make up the crystal could also account for variations in structure coordinates. If such variations are within an acceptable standard error as compared to the original coordinates, the resulting 3D shape is considered to be the same.

The LRS of *D. radiodurans* is a very large macromolecular complex comprising the following components: 5S and 23S rRNA molecules, and ribosomal proteins L1-L7, L9-L24, CTC, and L27-L36.

As shown in Example 1 of the Examples section below, crystal derived coordinate data can be used to define the 3D atomic structure of such LRS components, of portions of such LRS components, of combinations of such LRS components, or essentially of the entirety of the LRS (refer to Table 3).

While reducing the present invention to practice, the present inventors succeeded, following extensive experimentation, as described in Example 1 of the Examples section below, in crystallizing free eubacterial LRSs.

Thus, according to another aspect of the present invention, there is provided a method of crystallizing eubacterial LRSs.

Preferably, the method of the present invention is used to crystallize LRSs of *Deinococcus radiodurans* (*D. radiodurans*), more preferably of Deinococcus-Thermophilus group bacteria, more preferably of gram-positive bacteria, and most preferably of cocci.

According to the teachings of the present invention, crystallized free LRSs are obtained by isolating LRSs, preferably as previously described (Noll, M. *et al.* (1973) J Mol Biol. 75:281) and suspending them in an aqueous crystallization solution preferably supplemented with 0.1-10 % (v/v) of a volatile component and equilibrating the resulting crystallization mixture, preferably by vapor diffusion, preferably using standard Linbro dishes, preferably at 15-25 °C, most preferably at 17-20 °C, against an equilibration solution supplemented with the aforementioned volatile component at a concentration preferably 0.33-0.67 times, most preferably 0.5 times that thereof in the crystallization solution.

Typically in the vapor diffusion method, a small drop of crystallization mixture containing a macromolecule to be crystallized is placed on a cover slip or glass plate which is inverted over a well of equilibration solution such that the cover slip or glass plate forms a seal over the well. The equilibration solution is initially at a lower volatile component vapor pressure than the crystallization mixture so that evaporation of the volatile component from the crystallization mixture to the equilibration mixture progresses at a rate fixed by the difference in the vapor pressures therebetween and by the distance between the crystallization mixture and the equilibration solution. Thus, as evaporation proceeds, the crystallization mixture becomes supersaturated with the macromolecule to be crystallized and, under the appropriate crystallization mixture conditions-including pH, solute composition and/or concentration, and temperature-crystallization occurs.

Suitable crystallization solutions and equilibration solutions according to the present invention comprise, via the buffer component thereof: MgCl₂, preferably at a concentration of 3-12 mM, most preferably 10 mM; NH₄Cl, preferably at a concentration of 20-70 mM, most preferably 60 mM; KCl, preferably at a concentration of 0-15 mM, most preferably 5 mM; and HEPES, preferably at a concentration of 8-20 mM, most preferably 10 mM.

Preferably crystallization solutions and equilibration solutions are at a pH of 6.0-9.0, most preferably at a pH of 7.8.

Preferably, the buffer component of crystallization solutions and equilibration solutions are optimized for enabling *in vitro* functional activity of LRSs.

According to a preferred embodiment of the present invention, the buffer component of crystallization solutions and equilibration solutions is H-I buffer (10 mM MgCl₂, 60 mM NH₄Cl, 5 mM KCl, 10 mM HEPES pH 7.8).

Preferably, the volatile component is composed of a mixture of multivalent and monovalent alcohols, the multivalent to monovalent alcohol ratio preferably being 1:3.0 to 1:4.1, most preferably 1:3.5, the multivalent alcohol preferably being dimethylhexandiol and the monovalent alcohol preferably being ethanol.

The crystallized free LRSs of the present invention are preferably characterized by unit cell dimensions of approximately a = 170.827 ± 10 Å, b = 409.430 ± 15 Å and c = 695.597 ± 25 Å; more preferably a = 170.827 ± 5 Å, b = 409.430 ± 7.5 Å and c = 695.597 ± 12.5 Å; and most preferably a = 170.827 ± 1 Å, b = 409.430 ± 1.5 Å and c = 695.597 ± 2.5 Å, as shown in Example 1 of the Examples section, which follows.

It will be appreciated by one of ordinary skill in the art that, due to the high level of conservation between LRSs of different eubacteria, the method of crystallizing LRSs of the present invention can be generally applied to crystallizing LRSs of different types/species of eubacteria.

Examples of types/species of eubacteria include *Aquifex,* Thermotogales group bacteria (e.g., *Thermotoga, Fervidobacterium*), Thermodesulfobacterium group bacteria (e.g., *Thermodesulfobacterium*), Green nonsulfur group bacteria (e.g., *Chloroflexus, Herpetosiphon, Thermomicrobium*), Deinococcus-Thermus group bacteria (e.g., *Deinococcus, Thermus*), Thermodesulfovibrio group bacteria (e.g., *Thermodesulfovibrio*), Synergistes group bacteria (e.g. *Synergistes),* low G+C Gram positive group bacteria (e.g. *Bacillus, Clostridium, Eubacterium, Heliobacterium, Lactobacillus, Mycoplasma, Spiroplasma*), high G+C Gram positive group bacteria (e.g., *Bifidobacterium, Mycobacterium, Propionibacterium, Streptomyces),* Cyanobacteria (e.g., *Oscillatoria, Prochlorococcus, Synechococcus,* chloroplasts), Planctomycetales group bacteria (e.g., *Planctomyces*), Chlamydiales group bacteria (e.g., *Chlamydia*), Green sulfur group bacteria (e.g., *Chlorobium*), Cytophaga group bacteria (e.g., *Bacteriodes, Cytophaga, Flexibacter, Flavobacterium, Rhodothermus),* Fibrobacter group bacteria (e.g., *Fibrobacter*), Spirochetes group bacteria (e.g., *Borrelia, Leptonema, Spirochaeta* (including *Spirochaeta* sp. str. Antarctic), *Treponema*), and Proteobacteria group bacteria (e.g., alpha Proteobacteria, beta Proteobacteria, gamma Proteobacteria, delta/epsilon Proteobacteria, *Agrobacterium, Anaplasma, Rhodobacter, Rhodospirillum, Rickettsia,* mitochondria, *Neisseria, Rhodocyclus, Beggiatoa, Chromatium, Escherichia, Haemophilus, Legionella, Pseudomonas, Salmonella, Vibrio, Yersinia, Bdellovibrio, Campylobacter, Desulfovibrio, Helicobacter, Myxococcus*, and *Wolinella*).

Preferably, the method according to this aspect of the present invention is used to crystallize free LRSs of Deinococcus-Thermus group bacteria.

Examples of Deinococcus-Thermus group bacteria include *Thermus,* such as, for example, *T. thermophilus, T. aquaticus,* and *T. flavus*; and *Deinococcus* such as, for example, *D. radiodurans*, *D. geothermalis, D. radiophilus, D. murrayi, D. proteolyticus, D. radiopugnans,* and *D. erythromyxa.*

Most preferably the method of the present invention is used to crystallize *D. radiodurans* free LRSs.

Thus, the present invention also provides a method which can be used to crystallize LRSs in a manner which enables fine resolution of the crystal structure.

Although the method of the present invention is most preferably used to crystallize *D. radiodurans* LRS-antibiotic complexes, as described in Example 2 of the Examples section below, the method is generally suitable for crystallizing antibiotic-LRS complexes.

As mentioned hereinabove, LRSs are one of the main targets for antibiotics. As such, the present crystallization method was also used to crystallize LRS-antibiotic complexes in efforts of gaining insight into LRS-antibiotic interactions.

Thus, according to another aspect of the present invention there are provided compositions including crystallized antibiotic-LRS complexes.

Preferably, the antibiotic is chloramphenicol, a lincosamide antibiotic or a macrolide antibiotic.

Examples of lincosamide antibiotics include lincomycin, pirlimycin and clindamycin.

Preferably, the lincosamide antibiotic is clindamycin.

Examples of macrolide antibiotics include erythromycin, carbomycin, clarithromycin, josamycin, leucomycin, midecamycin, mikamycin, miokamycin, oleandomycin, pristinamycin, rokitamycin, rosaramicin, roxithromycin, spiramycin, tylosin, troleandomycin, virginiamycin and azalides.

Preferably, the macrolide antibiotic is clarithromycin, erythromycin or roxithromycin.

The crystallized antibiotic-LRS complexes of the present invention are suitable for generating, preferably via X-ray crystallography, coordinate data defining high resolution 3D atomic structures of crystallized antibiotic-LRS complexes, or portions thereof comprising antibiotic-binding pockets of LRSs and/or antibiotics. Hence, the crystallized antibiotic-LRS complexes of the present invention are suitable for generating coordinate data defining high resolution 3D atomic structures of the atomic interactions between antibiotic-binding pockets of LRSs and antibiotics.

As used herein, an "antibiotic-binding pocket" is defined as the set of LRS atoms or nucleotides which specifically associate with, or are capable of specifically associating with, an antibiotic.

Thus, the present invention provides coordinate data which define, at a resolution higher than or equal to 3.1 Å, the 3D atomic structure of crystallized antibiotic-LRS complexes, or portions thereof, including portions comprising the antibiotic-binding pocket of the LRS and/or the antibiotic, as demonstrated in Example 2 of the Examples section, below.

As is further described in the Examples section which follows, the present methodology was used to crystallize chloramphenicol-, clindamycin-, erythromycin- and roxithromycin-LRSs complexes.

The crystallized chloramphenicol-LRS complexes of the present invention are preferably characterized by unit cell dimensions of approximately a = 171.066 ± 10 Å, b = 409.312 ± 15 Å, and c = 696.946 ± 25 Å; more preferably a = 171.066 ± 5 Å, b = 409.312 ± 7.5 Å, and c = 696.946 ± 12.5 Å; and most preferably a = 171.066 ± 1 Å, b = 409.312 ± 1.5 Å, and c = 696.946 ± 2.5 Å, as shown in Example 2 of the Examples section, which follows.

The crystallized clindamycin-LRS complexes of the present invention are preferably characterized by unit cell dimensions of approximately: a = 170.286 ± 10 Å, b = 410.134 ± 15 Å and, c = 697.201 ± 25 Å; more preferably a = 170.286 ± 5 Å, b = 410.134 ± 7.5 Å, and c = 697.201 ± 12.5 Å; and most preferably a = 170.286 ± 1 Å, b = 410.134 ± 1.5 Å, and c = 697.201 ± 2.5 Å, as shown in Example 2 of the Examples section, below.

The crystallized clarithromycin-LRS complexes of the present invention are preferably characterized by unit cell dimensions of approximately: a = 169.871 ± 10 Å, b = 412.705 ± 15 Å and c = 697.008 ± 25 Å; more preferably a = 169.871 ± 5 Å, b = 412.705 ± 7.5 Å and c = 697.008 ± 12.5 Å; and most preferably a = 169.871 ± 1 Å, b = 412.705 ± 1.5 Å and c = 697.008 ± 2.5 Å, as shown in Example 2 of the Examples section, which follows.

The crystallized erythromycin-LRS complexes of the present invention are preferably characterized by unit cell dimensions of approximately: a = 169.194 ± 10 Å, b = 409.975 ± 15 Å, and c = 695.049 ± 25 Å; more preferably a = 169.194 ± 5 Å, b = 409.975 ± 7.5 Å, and c = 695.049 ± 12.5 Å; and most preferably a = 169.194 ± 1 Å, b = 409.975 ± 1.5 Å, and c = 695.049 ± 2.5 Å, as shown in Example 2 of the Examples section, below.

The crystallized roxithromycin-LRS complexes of the present invention are preferably characterized by unit cell dimensions of approximately: a = 170.357 ± 10 Å, b = 410.713 ± 15 Å, and c = 694.810 ± 25 Å; more preferably a = 170.357 ± 5 Å, b = 410.713 ± 7.5 Å, and c = 694.810 ± 12.5 Å; and most preferably a = 170.357 ± 1 Å, b = 410.713 ± 1.5 Å, and c = 694.810 ± 2.5 Å, as shown in Example 2 of the Examples section, which follows.

The crystallized antibiotic-LRS complexes of the present invention can be used to generate coordinate data defining 3D atomic structures thereof at characteristic resolutions.

The crystallized antibiotic-LRS complexes of the present invention can be used to generate coordinate data defining 3D atomic structures of crystallized chloramphenicol- or clarithromycin-LRS complexes, including portions thereof comprising the antibiotic-binding pocket of the LRS and/or the antibiotic, preferably at a resolution higher than or equal to 7 Å, more preferably at a resolution higher than or equal to 6 Å, more preferably at a resolution higher than or equal to 5 Å, more preferably at a resolution higher than or equal to 4 Å, and most preferably at a resolution higher than or equal to 3.5 Å.

The crystallized antibiotic-LRS complexes of the present invention are used to generate coordinate data defining 3D atomic structures of crystallized clindamycin-LRS complexes, including portions thereof comprising the antibiotic-binding pocket of the LRS and/or the antibiotic, preferably at a resolution higher than or equal to 6.2 A, more preferably at a resolution higher than or equal to 5 Å, more preferably at a resolution higher than or equal to 4 Å, more preferably at a resolution higher than or equal to 3.5 Å, and most preferably at a resolution higher than or equal to 3.1 Å.

The crystallized antibiotic-LRS complexes of the present invention are used to generate coordinate data defining 3D atomic structures of crystallized erythromycin-LRS complexes, including portions thereof comprising the antibiotic-binding pocket of the LRS and/or the antibiotic, preferably at a resolution higher than or equal to 6.8 Å, more preferably at a resolution higher than or equal to 6 Å, more preferably at a resolution higher than or equal to 5 Å, more preferably at a resolution higher than or equal to 4 Å, and most preferably at a resolution higher than or equal to 3.4 Å.

The crystallized antibiotic-LRS complexes of the present invention are used to generate coordinate data defining 3D atomic structures of crystallized clarithromycin-LRS complexes, including portions thereof comprising the antibiotic-binding pocket of the LRS and/or the antibiotic, preferably at a resolution higher than or equal to 7.4 Å, more preferably at a resolution higher than or equal to 7 Å, more preferably at a resolution higher than or equal to 6 Å, more preferably at a resolution higher than or equal to 5 Å, more preferably at a resolution higher than or equal to 4 Å, and most preferably at a resolution higher than or equal to 3.8 Å, as shown in Example 2 of the Examples section, below.

As shown in Example 2 of the Examples section which follows, atoms of LRSs associated with antibiotic atoms in crystallized antibiotic-LRS complexes are 23S rRNA nucleotide atoms.

As used herein, atoms which are termed "associated" are atoms positioned less than 4.5 Å apart.

As shown in Example 2 of the Examples section below, crystal derived coordinate data can be used to define the 3D atomic structure of portions of a chloramphenicol-LRS complex including, but not limited to, a portion of the chloramphenicol-binding pocket of the LRS and/or a portion of the chloramphenicol molecule (refer to Tables 7 and 12).

As shown in Example 2 of the Examples section below, crystal derived coordinate data can be used to define the 3D atomic structure of portions of a clindamycin-LRS complex including, but not limited to, a portion of the clindamycin-binding pocket of the LRS and/or a portion of the clindamycin molecule (refer to Tables 8 and 13).

As shown in Example 2 of the Examples section below, crystal derived coordinate data can be used to define the 3D atomic structure of portions of a clarithromycin-LRS complex including, but not limited to, a portion of the clarithromycin-binding pocket of the LRS and/or a portion of the clarithromycin molecule (refer to Tables 9 and 14).

As shown in Example 2 of the Examples section below, crystal derived coordinate data can be used to define the 3D atomic structure of portions of a erythromycin-LRS complex including, but not limited to, a portion of the erythromycin-binding pocket of the LRS and/or a portion of the erythromycin molecule (refer to Tables 10 and 15).

As shown in Example 2 of the Examples section below, crystal derived coordinate data can be used to define the 3D atomic structure of portions of a roxithromycin-LRS complex including, but not limited to, a portion of the roxithromycin-binding pocket of the LRS and/or a portion of the roxithromycin molecule (refer to Tables 11 and 16).

Since, as described above, the coordinate data of the present invention can be used to define 3D atomic structures of free LRSs, or portions thereof, such coordinate data can be used to generate models of the 3D atomic structure of free LRSs, or portions thereof, as described in Example 1 of the Examples section below.

Thus, according to still another aspect of the present invention, there are provided models of the 3D atomic structures of free LRSs, or portions thereof.

It will be understood by one of ordinary skill in the art that such models can be used to represent selected 3D structures of the free LRSs of the present invention and to thereby assign particular functions to particular structures represented by such models and to perform comparative structure/function analyses of different LRSs, or portions thereof, or to design or identify a molecule binding a desired portion thereof.

As described hereinabove, the coordinate data of the present invention define the essentially complete structure of crystallized free eubacterial LRSs at a resolution as high as 3.1 Å, whereas the highest prior art such resolution was 5.5 Å, and whereas no satisfactorily complete prior art 3D atomic structures of LRSs of any type have been defined at a resolution of 3.1 Å or higher.

Thus, it will be appreciated that the highly resolved coordinate data of the present invention define significantly more accurately the 3D structure of free LRSs, or portions thereof than the prior art. As such, the free LRS 3D atomic structure models of the present invention are distinctly superior to such prior art models in representing the structure of free LRSs. Thus, the free LRS 3D atomic structure models of the present invention are thereby also distinctly superior relative to such prior art models with regards to enabling elucidation of structural-functional relationships of free LRS. This is abundantly demonstrated by the wealth of novel structural-functional features of free LRSs which can now be described for the first time using the highly resolved data of the present invention, examples of which are described at length in the Examples section below, for example in Tables 4 and 5.

Thus, the models of the present invention can be used to provide novel and far-reaching insights into the crucial and universal mechanisms of protein production which are performed by the ribosome.

The models of the 3D atomic structures of free LRSs, or portions thereof, of the present invention can be exploited in several ways.

For example, data relating to free LRSs uncovered by the present invention can be used to design LRSs having desired characteristics. For example, LRSs could be designed to synthesize high levels of proteins. Such modified LRSs could thus be of value, for example, for enhancing recombinant protein production by bacteria, an area of potentially great economic and scientific benefit. Such functional modification could be achieved, for example, by using the models of the present invention to identify features of the LRS which negatively regulate protein synthesis and designing and modeling desired functional alterations. For example, features which sterically hinder growth of the nascent polypeptide or features which sterically hinder or limit tRNA processes could be altered so as not to cause such steric hindrance, thereby potentially enhancing protein production by ribosomes comprising such modified LRSs.

Coordinate data defining 3D atomic structures of antibiotic-LRS complexes, or portions thereof, at high resolution can be used to generate models of the 3D atomic structure of antibiotic-LRS complexes, or portions thereof, as described in Example 2 of the Examples section below.

Thus, according to a further aspect of the present invention, there are provided models of the 3D atomic structures of antibiotic-LRS complexes, or portions thereof.

Such models, being completely novel and unprecedented in the prior art, enable, for the first time, elucidation of the precise structural-functional basis of the interactions between antibiotics and LRSs, as described in extensive detail in Example 2 of the Examples section below, for a range of antibiotics.

By virtue of illuminating the precise structural-functional interactions between antibiotics and LRSs, the high resolution models of the 3D structure of the antibiotic-LRS complexes of the present invention constitute a unique and highly potent tool enabling the rational design or identification of putative antibiotics.

Thus, according to yet a further aspect of the present invention, there is provided a method of identifying a putative antibiotic.

The method of identifying a putative antibiotic is effected by obtaining a set of structure coordinates defining the 3D atomic structure of a crystallized antibiotic-binding pocket of a free LRS or, more preferably, of a crystallized antibiotic-complexed LRS and, preferably computationally, screening a plurality of compounds for a compound capable of specifically binding the antibiotic-binding pocket, thereby identifying the putative antibiotic.

Preferably, the method further comprises the steps of contacting the putative antibiotic with the antibiotic-binding pocket and detecting specific binding of the putative antibiotic to the antibiotic-binding pocket, thereby qualifying the putative antibiotic.

It will be appreciated, in this case, that qualification of a large number of putative antibiotic compounds can be used to provide data as to particular structural regions thereof which contribute to binding, thus enabling design of compounds which exhibit efficient binding.

Various methods of computationally screening compounds capable of specifically binding a set of atoms whose atomic positioning and structure is modeled, such as the antibiotic-binding pockets of the present invention, are well known to skilled artisans (see, for example, Bugg *et al*., Scientific American (1993) December: 92; West *et al.,* (1995) TIPS. 16:67; Dunbrack *et* *al.,* (1997) Folding and Design 2:27).

For example, potential antibiotic-binding pocket-binding compounds can be examined through the use of computer modeling using a docking program such as GRAM, DOCK, or AUTODOCK (Dunbrack *et al.,* (1997) Folding and Design 2:27). Using such programs, one may predict or calculate the orientation, binding constant or relative affinity of a given compound to an antibiotic-binding pocket, and use that information to design or select compounds of the desired affinity. Using such methods, a database of chemical structures is searched and computational fitting of compounds to LRSs is performed to identify putative antibiotics containing one or more functional groups suitable for the desired interaction with the nucleotides comprising the antibiotic-binding pocket. Compounds having structures which best fit the points of favorable interaction with the 3D structure are thus identified. Thus, these methods ascertain how effectively candidate compounds mimic the binding of antibiotics to antibiotic-binding pockets. Generally the tighter the fit (e.g., the lower the steric hindrance, and/or the greater the attractive force) the more potent the putative antibiotic will be.

Molecular docking programs may also be effectively used in conjunction with structure modeling programs (see hereinbelow). One important advantage of using computational techniques when selecting putative antibiotics is that such techniques can provide antibiotics with high binding specificity which are less likely to interfere with mammalian protein synthesis and/or cause side-effects.

Using computational approaches, compounds can furthermore be systematically modified by molecular modeling programs until promising putative antibiotics are generated. This technique has been shown, for example, to be effective in the development of HIV protease inhibitors (Lam *et al.* (1994) Science 263:380; Wlodawer *et al.* (1993) Ann Rev Biochem. 62:543; Appelt (1993) Perspectives in Drug Discovery and Design 1:23; Erickson (1993) Perspectives in Drug Discovery and Design 1:109) and hence of providing for the first time an apparent cure for AIDS.

Thus, the use of computational screening enables large numbers of compounds to be rapidly screened and produces small numbers of putative antibiotics without the requirement of resorting to the laborious synthesis of large numbers of compounds inherent to chemical synthesis techniques.

Once putative antibiotics are computationally identified they can either be obtained from chemical libraries, such as those held by most large chemical companies, including Merck, Glaxo Welcome, Bristol Meyers Squib, Monsanto/Searle, Eli Lilly, Novartis and Pharmacia UpJohn. Alternatively, putative antibiotics may be synthesized *de novo,* a feasible practice in rational drug design due to the small numbers of promising compounds produced via computer modeling.

Putative antibiotics can be tested for their ability to bind antibiotic-binding pockets in any standard, preferably high throughput, binding assay, via contact with target LRSs or portions thereof comprising antibiotic-binding pockets. Alternatively putative antibiotics can be functionally qualified for antibiotic activity, for example, via testing of their ability to inhibit growth of target bacterial strains *in vitro* or *in vivo* or to inhibit protein synthesis by target LRSs *in vitro.* When suitable putative antibiotics are identified, further NMR structural analysis can optionally be performed on binding complexes formed between the antibiotic-binding pocket and the putative antibiotic.

For all of the putative antibiotic screening assays described herein, further necessary refinements to the structure of the putative antibiotic may be performed by successive iteration of any and/or all of the steps provided by the particular screening assay.

Putative antibiotics may also be generated *in vitro,* for example, by screening random peptide libraries produced by recombinant bacteriophages (Scott and Smith (1990) Science 249:386; Cwirla *et al.* (1990) Proc Natl Acad Sci U S A. 87:6378; Devlin *et al*. (1990) Science 249:404) or chemical libraries.

Phage libraries for screening have been constructed such that when infected therewith, host *E. coli* produce large numbers of random peptide sequences of about 10-15 amino acids (Parmley and Smith (1988) Gene 73:305, Scott and Smith (1990) Science 249:386). In one such method, phages are mixed at low dilution with permissive *E. coli* strains in low melting point LB agar which is then overlayed on LB agar plates. Following incubation at 37 °C, small clear plaques in a lawn of *E. coli* form representing active phage growth. These phages are then adsorbed in their original positions onto nylon filters which are placed in washing solutions to block any remaining adsorbent sites. The filters can then be placed in a solution containing, for example, a radiolabelled LRS, or portion thereof, comprising an antibiotic-binding pocket. Following incubation, filters are thoroughly washed and developed for autoradiography. Plaques containing phages that bind to radiolabelled LRSs can then be conveniently identified and the phages further cloned and retested for LRS binding capacity. Following isolation and purification of LRS-binding phages, amino acid sequences of putative antibiotics can be deduced via DNA sequencing and employed to produce synthetic peptides.

Promising putative antibiotic peptides can then be easily synthesized in large quantities for clinical use. It is a significant advantage of this method that synthetic peptide production is relatively non-labor intensive, facile, and easily quality-controlled, such that large quantities of peptide antibiotics could be produced economically (see, for example, Patarroyo (1990) Vaccine, 10:175).

In another screening assay, a LRS, or portion thereof, comprising an antibiotic-binding pocket is bound to a solid support, for example, via biotin-avidin linkage and a candidate compound is allowed to equilibrate therewith to test for binding thereto. Generally, the solid support is washed and compounds that are retained are selected as putative antibiotics. In order to facilitate visualization, compounds may be labeled, for example, by radiolabeling or with fluorescent markers.

Another highly effective means of testing binding interactions is via surface plasmon resonance analysis, using, for example, commercially available BIAcore chips (Pharmacia). Such chips may be coated with either the LRS, or portion thereof comprising an antibiotic-binding pocket, or with the putative antibiotic, and changes in surface conductivity are then measured as a function of binding affinity upon exposure of one member of the putative binding pair to the other member of the pair.

It will be understood by one of ordinary skill in the art that rational design of LRSs can be easily effected, for example, via recombinant DNA technology or via chemical techniques.

Thus, the antibiotic-LRS complex 3D structure models of the present invention can be efficiently used by one of ordinary skill in the art to obtain novel antibiotics.

As well as providing a means whereby novel antibiotics can be obtained, the antibiotic-LRS complex 3D structure models of the present invention provide novel information illuminating the mechanisms of LRS function *per se,* since antibiotics function as inhibitors of, and hence as probes of LRS function, as described in Example 2 of the Examples section below.

The models of the present invention also serve as a valuable tool for solving related atomic structures.

In particular, the models of the 3D atomic structure of free LRSs and of antibiotic-LRS complexes of the present invention can be utilized, respectively, to facilitate solution of the 3D structures of free LRSs or antibiotic-LRS complexes, or portions thereof, which are similar to those of the present invention.

This can be effected, preferably computationally via molecular replacement. In molecular replacement, all or part of a model of a free LRSs or of an antibiotic-LRS complex of the present invention is used to determine the structure of a crystallized macromolecule or macromolecular complex having a closely related but unknown structure. This method is more rapid and efficient than attempting to determine such information *ab initio.* Solution of an unknown structure by molecular replacement involves obtaining X-ray diffraction data for crystals of the macromolecule or macromolecular complex for which one wishes to determine the 3D structure. The 3D structure of a macromolecule or macromolecular complex whose structure is unknown is obtained by analyzing X-ray diffraction data derived therefrom using molecular replacement techniques with reference to the structural coordinates of the present invention as a starting point to model the structure thereof, as described in U.S. Pat. No. 5,353,236, for instance. The molecular replacement technique is based on the principle that two macromolecules which have similar structures, orientations and positions in the unit cell diffract similarly. Molecular replacement involves positioning the known structure in the unit cell in the same location and orientation as the unknown structure. Once positioned, the atoms of the known structure in the unit cell are used to calculate the structure factors that would result from a hypothetical diffraction experiment. This involves rotating the known structure in the six dimensions (three angular and three spatial dimensions) until alignment of the known structure with the experimental data is achieved. This approximate structure can be fine-tuned to yield a more accurate and often higher resolution structure using various refinement techniques. For instance, the resultant model for the structure defined by the experimental data may be subjected to rigid body refinement in which the model is subjected to limited additional rotation in the six dimensions yielding positioning shifts of under about 5%. The refined model may then be further refined using other known refinement methods.

According to one embodiment, the coordinates of the present invention can be used to model atomic structures defined by a set of structure coordinates having a root mean square deviation of not more than 2.0 Å, more preferably of not more than 1.0 Å, and most preferably of not more than 0.5 Å from a set of structure coordinates of the present invention.

Supplementary utilities of the models of the present invention include computational identification or rational design of potentially antibiotic resistant forms of LRSs followed by identification or rational design of putative antibiotics effective against such modified LRSs, thereby providing banks of antibiotics potentially useful against future outbreaks of bacterial pathogens bearing such antibiotic resistant LRSs.

The 3D atomic structure models of the present invention, or portions thereof, can further be utilized to computationally identify RNA bases or amino acids within the 3D structure thereof, preferably within or adjacent to an antibiotic-binding pocket; to generate and visualize a molecular surface, such as a water-accessible surface or a surface comprising the space-filling van der Waals surface of all atoms; to calculate and visualize the size and shape of surface features of free LRSs or antibiotic-LRS complexes, to locate potential H-bond donors and acceptors within the 3D structure, preferably within or adjacent to an antibiotic-binding pocket; to calculate regions of hydrophobicity and hydrophilicity within the 3D structure, preferably within or adjacent to an antibiotic-binding pocket; and to calculate and visualize regions on or adjacent to the protein surface of favorable interaction energies with respect to selected functional groups of interest (e.g., amino, hydroxyl, carboxyl, methylene, alkyl, alkenyl, aromatic carbon, aromatic rings, heteroaromatic rings, substituted and unsubstituted phosphates, substituted and unsubstituted phosphonates, substituted and unsubstituted fluoro and difluorophosphonates; etc.).

The 3D atomic structure models of the present invention are preferably generated by a computing platform **20** (Figure 13) which generates a graphic output of the models via display **22**. The computing platform generates graphic representations of atomic structure models via processing unit **24** which processes structure coordinate data stored in a retrievable format in data storage device **26**. Examples of computer readable media which can be used to store coordinate data include conventional computer hard drives, floppy disks, DAT tape, CD-ROM, and other magnetic, magneto-optical, optical, floptical, and other media which may be adapted for use with computing platform **20**.

Suitable software applications, well known to those of skill in the art, which may be used by processing unit **24** to process structure coordinate data so as to provide a graphic output of 3D structure models generated therewith via display **22** include RIBBONS (Carson, M. (1997) Methods in Enzymology 277: 25), O (Jones, TA. *et al*. (1991) Acta Crystallogr A47:110), DINO (DINO: Visualizing Structural Biology (2001) http://www.dino3d.org); and QUANTA, CHARMM, INSIGHT, SYBYL, MACROMODE, ICM, MOLMOL, RASMOL and GRASP (reviewed in Kraulis, J. (1991) Appl Crystallogr. 24:946).

Preferably, the software application used to process coordinate data is RIBBONS.

Preferably the structure coordinates of the present invention are in PDB format for convenient processing by such software applications. Most or all of these software applications, and others as well, are downloadable from the World Wide Web.

Thus, the present invention provides novel and highly resolved 3D structural data of a bacterial LRS, thereby providing for the first time, tools enabling the design and testing of novel antibiotic compounds as well as tools which can be used to predict the effect of changes in LRS structure on antibiotic-binding efficiencies and the like.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J-, ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization -A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### Growth of D. radiodurans LRS crystals and solution of the complete 3D atomic structure of the eubacterial LRS at a 3.1 Å resolution

The ability to generate 3D models of bacterial LRS structure and function at the atomic level would be extremely useful since the ribosome is responsible for the central biological process of protein production and serves as the main binding target for a broad range of antibiotics. Three-dimensional atomic structure models of the LRS could be of significant utility for elucidating mechanisms of ribosome function. Such models could constitute a powerful tool for the rational design or identification of antibiotics, a vital need in light of expanding epidemics of diseases caused by antibiotic resistant microorganisms. Furthermore these models could be employed to rationally design or select ribosomes having desired characteristics, such as, for example, enhanced protein production capacity when expressed in bacterial strains which would be of great benefit, for example, for improving recombinant protein production. All prior art approaches, however, have failed to produce satisfactory 3D atomic models of LRS such as, for example, for drug design or drug improvement. In order to fulfill these important needs, therefore, high resolution 3D atomic structure models of bacterial LRSs were generated while reducing the present invention to practice, as follows.

### Materials and Methods:

***Cell culture:*** *D. radiodurans* cells were cultured as recommended by the American Tissue-Type Culture Collection (ATCC), using ATCC medium 679 with minor modifications.

***Amino acid sequencing of D. radiodurans large ribosomal subunit proteins:*** *D. radiodurans* LRS proteins were separated by 2D polyacrylamide gel electrophoresis and identified via sequencing analysis of their five N-terminal amino acids.

***Determination of D. radiodurans 23S and 5S rRNA secondary structure:*** Secondary structure diagrams were constructed for the 23S and 5S rRNA chains of D50S, guided by their sequences and by the available diagram for the RNA of the 50S subunit from *T. thermophilus* (T50S, Gutell, R. (1996) In *Ribosomal RNA: Structure, Evolution, Processing and Function in Protein Biosynthesis., A.* E. Z. Dahlberg, R. A., eds, ed. (FL, USA: CRC Press, Boca Raton), pp. 111-128).

***Growth of D50S crystals:*** Ribosomes and their subunits were prepared as previously described (Noll, M. *et al.* (1973) J Mol Biol. 75:281) and suspended in solutions containing H-I buffer (10 mM MgCl₂, 60 mM NH₄Cl, 5 mM KCl, 10 mM HEPES pH 7.8), a buffer optimized for testing *in vitro* functional activity of ribosomes, supplemented with 0.1-1 % (v/v) of a solution comprising monovalent and multivalent alcohols (typically comprising the monovalent and multivalent alcohols dimethylhexandiol and ethanol, respectively, at a ratio dimethylhexandiol to ethanol of 1:3.5) as a precipitant. Ribosome suspensions were equilibrated against equilibration solutions comprising the same buffer components as the solutions in which the ribosomes were suspended but containing half the amount of alcohols thereof, as previously described (Yonath A. *et al.* (1983) FEBS Lett. 163:69; Yonath, *A. et al.* (1982) Journal of Cellular Biochemistry 19:145). Crystals were grown in hanging drops using standard Linbro dishes using vapor diffusion at 18 °C. For optimizing crystal growth, it was necessary to determine the exact conditions for every preparation individually. The same, or similar, divalent alcohols (e.g. ethyleneglycol) were used as cryoprotectants for flash freezing of the crystals in liquid propane.

Heavy-atom derivatives of D50S were prepared by soaking crystals in 1-2 mM of iridium pentamide or K₅H(PW₁₂O₄₀)12H₂O for 24 hours.

***Collection of X-ray diffraction data:*** Experimental MIRAS phases were obtained from anomalous data using heavy atom derivatives of D50S crystals. The tungsten and iridium sites were obtained from difference Patterson, residual and difference Fourier maps. To remove potential bias of W₁₂, the density modification procedure was altered to gradually include the low-resolution terms.

X-ray diffraction data was collected at 95 K with well-collimated X-ray beams at high brightness synchrotron (SR) stations (ID14/European Synchrotron Radiation Facility (ESRF)/European Molecular Biology Laboratory (EMBO) and ID19/Argonne Photon Source (APS)). Data was recorded on image-plates (MAR 345) or CCD (Mar, Quantum 4, or APS2), processed with DENZO and reduced with SCALEPACK (Otwinowski, Z. and Minor, W. (1997) Macromolecular Crystallography, Pt A 276:307) and the CCP4 package (Bailey, S. (1994) Acta Cryst D. 50:760). Crystals were screened at BW6/MPG and BW7/EMBL at Deutsches Elektronen-Synchrotron (DESY).

***Phase determination:*** The initial electron density maps were obtained by molecular replacement searches using AmoRe (Navazza J. (1994) Acta Crystallogr. A50:57), using the structure of H50S determined by us and others (Yonath *et al.* (1998) Acta Crystallogr. A, 54:945; Ban, N. *et al.* (2000) Science 289:905) as a basis for the search model. The phases obtained from the MR solution (correlation coefficient, calculated using intensities = 45.8 % and contrast to next solution = 1.5) were subjected to several cycles of density modification using SOLOMON (Abrahams, J.P. and Leslie, A.G.W. (1996) Acta Cryst. D. 52:30). The resulting map was sufficiently clear to model a significant part of D50S but MIRAS phasing by heavy metal atoms was still required for tracing of the RNA chains and of the proteins. The inclusion of phase information obtained from the two heavy-atom derivatives substantially improved the quality of the electron density.

Some of the D50S proteins were localized using structural homology with the available high resolution structure of H50S as a guide. The high level of homology existing between *D. radiodurans* and *E. coli* LRSs and existing knowledge concerning their relative positions (Wittmann, H. G. (1983) Annu Rev Biochem. 52:35; Walleczek, J. *et al* (1989) Biochemistry 28:4099) were used for initial placement of most of the proteins, including L7, L10, L11, L17, CTC (L25 in *E. coli*), L27, L28 and L31-L36 that do not exist in H50S and to model their interactions with rRNA (Ostergaard, P. *et al.* (1998) J Mol Biol. 284:227). The coordinates determined for the isolated proteins (Golden, B. L. *et al.* (1993) Embo J. 12:4901; Wimberly, B. T. *et al*. (1999) Cell 97:491; GuhaThakurta, D., and Draper, D. E. (2000) J Mol Biol. 295:569; Fedorov, R. *et al*. (1999) Acta Cryst D. 55:1827; Fedorov, R. *et al.* (2001) Acta Cryst D. 57:968; Worbs, M. *et al.* (2000) Embo J. 19:807; Unge, J. *et al*. (1998) Structure 6:1577; Nikonov, S. *et al.* (1996) Embo J. 15:1350; Hoffman, D. W. *et al.* (1996) J Mol Biol. 264:1058; Davies, C. *et al.* (1996) Structure 4:55; Wahl, M. C. *et al.* (2000) Embo J. 19:174; Hard, T. *et al.* (2000) J Mol Biol. 296:169) were also used.

***Docking procedure:*** The A-, P- and E-site tRNA molecules were positioned on the LRS as previously described (Schluenzen, F. *et al.* (2000) Cell 102:615) in the same relative orientation as was observed in the 5.5 Å resolution structure of the T70S ribosome (Yusupov MM. (2001) Science 292:883).

### Experimental Results:

***Generation of D50S crystals:*** D50S crystals, having an ovo-discoidal shape were grown (Figure 1).

***Amino acid sequences of D. radiodurans large ribosomal subunit proteins:*** Ribosomal proteins identified via 2D PAGE and by sequencing the five N-terminal amino acids of each protein are shown in Figure 2. Upon comparison to reported sequences in the TIGR database (White, O. *et al.* (1999) Science 286:1571), two discrepancies were identified; one in the sequence of protein CTC starting at amino acid residue 19 and the other in protein L6 starting at amino acid residue 30 of the predicted sequence. These results constituted the first such amino acid-based sequencing of D. *radiodurans* LRS proteins.

***D50S structure determination:*** The 3D atomic structure of D50S was determined and refined to 3.1 Å by generating structural coordinates using data derived from X-ray crystallography of native D50S (Table 1) and heavy atom derivatives thereof (Table 2).

**Table 1.**

| ***Native D50S crystallographic data.*** | | | | | |
|---|---|---|---|---|---|
| Data set | Resolution (Å) | No. of unique reflections | Rsym (%) | Completeness (%) | <I/sig (I)> |
| 1 | 50-3.0 | 178685 | 11.8 (69.6) | 49.9 (42.1) | 4.5 (1.5) |
| 2 | 50-3.1 | 400658 | 15.9 (44.4) | 92.3 (77.3) | 12.0(1.5) |

**Table 2.**

| ***D50S heavy atom derivative crystallographic data.*** | | | | | | |
|---|---|---|---|---|---|---|
| Heavy atom | No. of sites | Resolution (Å) | Unit cell dimensions (Å) | Rsym (%) | Completeness (%) | <I/sig (I)> |
| Penta-Ir | 56 | 50-4.0 | 170.24 × 410.54 × 696.52 | 14.5 (47.2) | 93.9(90.9) | 5.8 (1.9) |
| W12 | 4 × 12 | 30-6.0 | 170.15 × 408.65 × 696.52 | 8.1 (12.9) | 71.9 (69.2) | 14.1 (5.0) |
| FOM = 0.58 post-phasing (SHARP), and 0.78 post-density modification with SOLOMON. | | | | | | |
| Space group = I222; Unit cell dimensions: 170.827 × 409.430 × 695.597 Å | | | | | | |
| R_{factor}/R_{free} = 25.6 %/28.3 % | | | | | | |
| Highest resolution bins are in parentheses | | | | | | |

The 3D atomic structure of portions, or combination of portions, of crystallized D50S are defined by atom coordinates (*D. radiodurans* numbering system) set forth in Table 3 (refer to enclosed CD-ROM), as follows:
D50S: 1-65345;
23S rRNA: 1-59360;
5S rRNA: 59361-61880;
ribosomal protein L2: 61881-62151;
ribosomal protein L3: 62152-62357;
ribosomal protein L4: 62358-62555;
ribosomal protein L5: 62556-62734;
ribosomal protein L6: 62735-62912;
ribosomal protein L9: 62913-62965;
ribosomal protein L11: 62966-63109;
ribosomal protein L13: 63110-63253;
ribosomal protein L14: 63254-63386;
ribosomal protein L15: 63387-63528;
ribosomal protein L16: 63529-63653;
ribosomal protein L17: 63654-63768;
ribosomal protein L18: 63769-63880;
ribosomal protein L19: 63881-64006,;
ribosomal protein L20: 64007-64122;
ribosomal protein L21: 64123-64223;
ribosomal protein L22: 64224-64354;
ribosomal protein L23: 64355-64448;
ribosomal protein L24: 64449-64561;
ribosomal protein CTC: 64562-64785;
ribosomal protein L27: 64786-64872;
ribosomal protein L28: 64873-64889;
ribosomal protein L29: 64890-64955;
ribosomal protein L30: 64956-65011;
ribosomal protein L31: 65012-65085;
ribosomal protein L32: 65086-65144;
ribosomal protein L33: 65145-65198;
ribosomal protein L34: 65199-65245;
ribosomal protein L35: 65246-65309; and
ribosomal protein L36: 65310-65345.

Atomic coordinates defining the 3D atomic structure of D50S were deposited in the protein data bank (PDB) under accession code 1KPJ.

The fold of the 23S and 5S rRNA chains manually traced in electron density maps was found to be in remarkable agreement to that predicted. Only in a few instances did the base-pairing system deviate from the predicted scheme, one example being the predicted base pair near the loop of H81 (C2243-G2255, in the *D. radiodurans* numbering system) which was found to be flipped out in the 3D structure of D50S.

Essentially all (96 %) of the 23S rRNA nucleotides and 30 of the 33 D50S proteins were traced in the electron density map and found to be mostly ordered. The remaining three proteins were successfully placed, and portions thereof resolved, and the locations of several metals and hydrated Mg²⁺ ions were identified.

***Overall structure of D50S:*** The traditional shape of the LRS, as seen by electron microscopy, contains a massive core with a central elongated feature and two lateral protuberances, termed the L1 and L7/L12 stalks. The view referred to as the "crown view", has the appearance of a halved pear with two protuberances. Its "flat" surface, facing the viewer in Figure 3, is adjacent to the small subunit in the 70S ribosome and its globular distal side faces the solvent. The overall shape of D50S is generally similar to this consensus view. Typical map segments of an rRNA helix and of a protein chain are shown in Figures 1b and 1c, respectively.

As in all other models of the LRS, the 23S rRNA molecule forms the bulk of the structure and the small 5S rRNA molecule forms most of an elongated feature in the center of the "crown". At the secondary structure level the two RNA chains form seven domains and, although each of the domains has a unique three-dimensional shape, together they produce a compact single intertwined core, in contrast to the domain-like design of the 30S subunit (Schluenzen, F. *et al.* (2000) Cell 102:615; Wimberly, B.T. *et al*. (2000) Nature 407:327).

Superimposition of the D50S structure onto that of H50S (Ban, N. *et al.* (2000) Science 289:905) and onto that of the 50S subunit within the 5.5 Å structure of the T70S ribosome (Yusupov, M. M. *et al.* (2001) Science 292:883) revealed similarities between the rRNA folds thereof, as previously observed between all known 50S structures. Despite these similarities significant structural differences were detected. It was discovered that the central regions of all three structures were similar in all structures, however, even within conserved regions structural differences were detected in RNA bulges, hairpin loops, structural motifs such as the loop-E (Leontis, N. B. and Westhof, E. (1998) J Mol Biol. 283:571) and the base-pairing scheme, which cannot be explained by the expected phylogenetic variations. Some of these features are located at strategic locations and cause significant changes in the local architecture which may propagate towards the periphery. Thus, several surface regions in D50S appear to be unique relative to other 50S structures, even when the local environment of individual features is similar. In order to pinpoint the meaningful differences, the model was divided into individual structural elements, each containing a few neighboring helices and junctions, and the environment of each element was analyzed separately, both visually and by computing the internal distances separating features therein (Tables 4 and 5). In this way, significant differences unrelated to the sequences were discovered, of which some can be related to ribosomal function.

**Table 4.**

| ***Characterization of D50S proteins*** | | | | | | | | | |
|---|---|---|---|---|---|---|---|---|---|
| D50S protein | DNA^{»} seq. length (bp) | General fold^{@} | Contacts with rRNA and docked tRNA (< 4.5 Å) | Proteins separated by < 4.5 Å | H50S counterpart | Comparison to H50S proteins | | | |
| | | | | | | D¹ | E² | Ct³ | Nt° |
| L2 | 275 | αβ + Ct | II, III, IV, V | - | L2 | + | | ± | |
| L3 | 211 | αβ + ext. | II, III, IV, V, VI | L13, L14, L17,L19 | L3 | + | + | | - |
| L4 | 205 | αβ+ext. | I, II, III, IV, V | L15, L20, L34 | L4 | + | ± | | |
| L5 | 180 | αβ + small ext. | V, 5S, P-tRNA | - | L5 | ± | - | | |
| L6 | 212 | 2αβ + Ct | V, VI | L36 | L6 | + | | - | |
| L9 | 146 | αβ | I, V | L31 | | | | | |
| L11 | 144 | 2αβ | II | CTC | | | | | |
| L13 | 174 | αβ + Nt + ext. | II, V, VI | L3, L20 | L13 | + | - | | - |
| L14 | 134 | β barrel, αβ | III, IV, V, VI | L3, L19 | L14 | + | - | | |
| L15 | 156 | α + Nt | I, II, III, V | L4, L21, L35 | L15 | + | | - | ± |
| L16 | 142 | αβ | 5S, II, V, A and P-tRNA | CTC, L27 | L10e | + | | | |
| L17 | 116 | αβ + Nt | III, IV, VI | L22, L32, L3 | L31e | - | - | | - |
| L18 | 114 | αβ | 5S,V | L27 | L18 | + | | - | - |
| L19 | 166 | β barrel + Ct + Nt | IV, VI | L3, L14 | L24e | - | | - | - |
| L20 | 118 | extended α-helix | I, II, IV | L13, L21, L4 - | | | | | |
| L21 | 169 | B barrel + bhl | II | L15,L20 | - | | | | |
| L22 | 134 | αβ + bhl + Nt | I, II, III, IV | L17,L32 | L22 | + | ± | | - |
| L23 | 95 | αβ + bhl | I, III | L29 | L23 | + | - | | |
| L24 | 115 | B barrel + ext. + Nt + C ext | I | - | L24 | + | D longer | - | - |
| CTC | 253 | B barrel + αβ + α | II, V, 5S, A-tRNA | L16, L11 | - | | | | |
| L27 | 91 | B + Nt + C ext. | II, V, 5S, P-tRNA | L18,L16 | L21e | - | | - | - |
| L28 | 81 | extended α-helix | I | - | - | | | | |
| L29 | 67 | A (leucine zipper) | I | L23 | L29 | + | | | |
| L30 | 55 | αβ | II, 5S | - | L30 | ± | | | |
| L31 | 73 | αβ + bhl-N-ext. | I, III, IV, V, E-tRNA | L9 | L15e | - | | - | - |
| L32 | 60 | B Zn-finger motif + Nt | I, II, IV, VI | L17, L22 | - | | | | |
| L33 | 55 | B + Ct | II, V, E-tRNA | L35 | L44e | + | | | - |
| L34 | 47 | A+Nt | I, II, III | L4 | L37e | - | | - | ± |
| L35 | 66 | A+ext. | I, II, V | L15,L33 | - | | | | |
| L36 | 37 | B Zn-finger motif | II, V, VI | L6 | - | | | | |

| | | | | | | | | | |
|---|---|---|---|---|---|---|---|---|---|
| ^{»}According to (White *et al.,* 1999) @ Folds are: α - alpha helical domain. β - beta sheet globular domain. αβ- mixed alpha helical and beta sheet, bhl- β hairpin loop;. ext- extended loop. N-ext, C-ext: extended loops at the C-terminus or N-terminus, N-t, C-t- extended tails at the N-terminus or C-terminus. ¹D = domain similarity | | | | | | | | | |
| ²E = extended loop similarity | | | | | | | | | |
| ³Ct = C-terminal tail similarity, °Nt = N-terminal tail similarity ± In globular domains: some similarity. In extended loops: one ext. is similar while the other is different. In tails: one tails is different in length, the other less or no difference. | | | | | | | | | |

**Table 5.**

| ***Characterization of D50S RNA features*** | | |
|---|---|---|
| Helix # | Characteristics in D50S | Comparisons with other 50S subunits |
| 1 | located between H94 and L13, coaxial with H2 | disordered in Hm |
| 9 | loop shorter than Tt therefore no contact to H54 minor groove | |
| Junc 11/12 | bends toward the lower part of H1 | no bend in Tt, interacts with Junc H4/H14 |
| 12 | smaller loop due to sequence difference, no equivalence to nt 197 in Hm; interacts with H22 via flipped out U206 | C197 in Hm flips out |
| 18 | second bulge interacts with minor groove of H7 | Hm: minor/minor interactions with H4 |
| 21 | larger loop than in Tt and Hm; folds backwards, connecting U400 via Mg; G399 flips out and contacts H11 (U177) and H13 (G225) | |
| 25 | all are similar in the lower part, Tt and Dr are shorter than Hm; Dr loop contacts minor groove of H46 | Tt's loop does not contact H46 minor groove |
| 28 | A624 and A625 flip out | Hm: counterpart (A674) interacts with H4 |
| 38 | loop nt 942-946 points backwards; nt 893-908 disordered | Hm: counterpart (1027-1033) interact with H45; nt 971-998 disordered |
| 39 | U969 flips out towards loop H4 (in 5S rRNA) backbone | |
| June 41/42 | Nt 1036-1037 similar to Tt; different from Hm (1123 -1230). | Hm: bulge contacts H39, 40, 72 |
| 43 | angular separation between H43-44 wider than in Tt | Hm: H43/44 disordered. Tt: loop E motif |
| Junc 26/47 | no similarity to Tt and Hm; Dr shorter than Hm | Hm: connection to H96 and H61 |
| 57 | similar to Tt, loop interacts with H101 backbone | |
| 58 | Dr is similar to Tt from bulge onward but different from Hm; interacts with H54 bulge | Hm: contacts H56 minor groove. Loop interacts with H34 |
| 59 | slightly rotated compared to Tt; facing the solvent | does not exist in Hm |
| 61 | No equivalent to extra nt U1722 in Hm; Dr: L17(Ala6) occupies place of G1730 in Hm | nt U1722 in Hm interacts with helix 59a |
| 63 | shorter but similar to Hm | Tt: minor/minor with H56 |
| 68 | longer loop than Hm, interacts with base-pairs in H22, H88 | |
| 69 | interacts with H71, lies on interface | Hm: disordered; Tt: contacts H44 of 30S |
| 73 | U2591 different orientation than Hm equivalent (C2647); contacts H35 backbone Dr: L32-His4 occupies position of Hm C2647 | |
| 77-78 | arm displaced by 30° angle relative to its position in Tt | Hm: disordered; Tt: blocks E-RNA passage |
| 79 | longer than Hm, Tt; loop contacts H52 and loop H58 | |
| 84 | | Nt 2339-2343 disordered |
| Junc 89/90 | U2483 flips out and contacts G2044 at Junc 73/4 | equivalent position of A2551 in Dr is taken by a pyrimidine (U2607) |
| 96 | C2669 faces its third bulge; located opposite to U2727 in H, whose place is occupied by G2847 in Dr | |
| Junc 99/1 | Nt 2866-70 face the lower part of 1198, contacts H94 | Tt: face the backbone of H98, points towards H2 |
| Dr = *D. radiodurans*, Hm = H. *marismortui,* Tt = T. *thermophilus;* Junc = junction in the secondary map of D50S. nt = nucleotide | | |

Several structural studies have been performed on isolated LRS fragments, among them two NMR studies of the vicinity of H80 (Puglisi, E. *V. et al.* (1997) Nature Struct. Biol. 4:775) and of H92 (Blanchard, S.C. and Puglisi, J.D. (2001) Proc Natl Acad Sci U S A. 98:3720). The results of the first study do not fit the *in situ* conformation within D50S, most likely because of the lack of supporting interactions with neighboring features. The second, however, fits rather well, since most of the structure forms a helix. Nevertheless, even in this case, the curvature of the helix and the shape of the stem loop differ from our observations.

The sarcin/ricin loop (SRL) is a feature that interacts with G domains of elongation factors and which has been found to be essential for elongation factor binding. This loop is located near protein L14 and the site assigned for A-tRNA in our docking experiments that were based on the structure of the 70S ribosome complex (Yusupov, M. M. *et al.* (2001) Science 292:883). Although conformational dynamics of the sarcin-ricin loop are believed to be involved in factor binding, the high resolution structure of this region determined in isolation (Correll, C. C. *et al.* (1997) Cell 91:705) matches that seen in the structure D50S.

The conformation of the 5S rRNA in D50S is slightly different from those determined for it and its complexes in isolation (Correll, C. C. *et al.* (1997) Cell 91:705; Nakashima, T. *et al.* (2001) RNA 7:692; Lu, M. and Steitz, T. A. (2000) Proc Natl Acad Sci U S A. 97:2023; Fedorov, R. *et al.* (2001) Acta Cryst D. 57:968). Two of its binding proteins, called L5 and L25 in *E. coli,* have been extensively studied in isolation (Nakashima *et al.,* 2001) and in complex with RNA fragments representing their binding sites to the 5S molecule (Lu, M. and Steitz, T. A. (2000) Proc Natl Acad Sci U S A 97; Fedorov, R. *et al.* (2001) Acta Cryst D. 57:968). We found that the conformations determined for L5 from *Bacillus stearothermophilus (B. stearothermophilus)* in isolation (Nakashima, T. *et al.* (2001) RNA 7:692) does resemble that seen in D50S (for more detail, see below).

D50S contains several proteins and RNA features that do not appear in H50S and T50S (Tables 4 and 5), including two Zn-fingers proteins, and proteins L32 and L36. Almost all of the globular domains of the D50S proteins are peripheral and, as in H50S and T30S, most of them have tails and extended loops that permeate the subunit's core. Analysis of the general modes of the RNA-protein interactions within D50S did not reveal striking differences from what was reported for the other ribosomal particles. However, many of the D50S proteins that have counterparts in H50S show significantly different conformations.

***Mutations within a single ribosomal protein potentially mediate adaptation from mild to stressful conditions:*** In D50S, CTC (named after a general shock protein) replaces the 5S rRNA binding proteins L25 in *E. coli* and its homologue TL5 in T50S. H50S contains neither L25 nor any of its homologues. Within the known members of the CTC protein family, that of *D. radiodurans* is the longest, containing 253 residues and thus being about 150 residues longer than *E. coli* L25 and 60 residues longer than *T. thermophilus* TL5.

The structure of complexes of *T. thermophilus* TL5 (Fedorov, R. *et al.* (2001) Acta Cryst D. 57:968) and *E. coli* L25 (Lu, M. and Steitz, T. A. (2000) Proc Natl Acad Sci U S A 97) with RNA fragments corresponding to their 5S rRNA binding regions (40 and 18 nucleotides long, respectively) have been determined at high resolution. Comparisons between them showed that the structure of the N-terminal domain of TL5 is similar to that of the entire L25. Protein CTC has three domains; the N-terminus is similar to the entire L25 and to the N-terminus of TL5 and the middle domain is similar to the C-terminal domain of TL5. However, the relative orientation of the N-terminal and of the middle CTC domains differs from that determined for the two domains of TL5 in isolation. The third domain of CTC, the C-terminal, is composed of three long α-helices connected by a pointed end, bearing some resemblance to structural motif seen in some small ribosomal subunit proteins.

As mentioned above, the N-terminal domain of CTC is located on the solvent side of D50S (Figure 4), at the presumed position of L25 in *E. coli.* The middle domain fills the space between the 5S and the L11 arm, and interacts with H38, the helix that forms the intersubunit bridge called B 1a (see below). The interactions with H38 and the partial wrapping of the central protuberance (CP) of the subunit, are likely to provide additional stability, consistent with the fact that these two domains are almost identical to the substitute for protein L25 (protein TL5) in the ribosome of *T. thermophilus.*

The C-terminal domain is placed at the rim of the intersubunit interface. Docking the tRNA molecules, as seen in the 5.5 Å structure of the T70S complex (Yusupov, M. M. *et al.* (2001) Science 292:883), showed that the C-terminal domain of CTC reaches the A-site and restricts the space available for the tRNA molecules. The somewhat lower quality of the electron density map of this domain hints at its inherent flexibility and indicates that it may serve as an A-site regulator and may also have some influence on the processing of mRNA. In addition, the C-terminal domain of CTC interacts with the A-finger. This interaction, the manipulation of the binding of tRNA at the A-site, the influence on the mRNA progression and the enhanced stability of the CP caused by CTC may be parts of the mechanisms that *D. radiodurans* developed for survival under extremely stressful conditions.

### Features involved in ribosomal functions:

***The L1 stalk:*** The L1 stalk includes helices H75-H78 and protein L1, a feature that was identified as a translational receptor binding mRNA (Nikonov, S. *et al.* (1996) Embo J. 15:1350). Its absence has a negative effect on the rate of protein synthesis (Subramanian, A.R., and Dabbs, E.R. (1980) Eur J Biochem 112:425). In the complex of T70S with all three tRNA molecules, the L 1 stalk interacts with the elbow of E-tRNA and the exit path for the E-tRNA is blocked by proteins L1 from the large subunit and S7 from the small one (Yusupov, M. M. *et al.* (2001) Science 292:883). Consequently it was suggested that the release of the deacylated tRNA requires that one or both of these features move. Movement of the L1 arm was also associated with the binding of EF2 in yeast (Gomez-Lorenzo, M. G. (2000) Embo J 19:2710). In H50S, the entire L1 arm is disordered and therefore could not be traced in the electron density map (Ban, N. *et al.* (2000) Science 289:905), an additional hint of its inherent flexibility.

In D50S the L1 arm is tilted about 30° away from it corresponding position in T70S, so that the distance between the outermost surface points of the L1 arm in the two positions is over 30 Å (Figure 5). The orientation of the L1 arm in D50S allows the location of protein L1 so that it does not block the presumed exit path of the E-site tRNA. Hence, it is likely that the mobility of the L1 arm is utilized for facilitating the release of E-site tRNA. Superposition of the structure of D50S on the LRS of the T70S ribosome suggests definition of a pivot point for a possible rotation of the L1 arm.

It has been suggested that a head-platform concerted motion in the small subunit may assist the exit of the E-site tRNA as well as the translocated mRNA (Pioletti, M. *et al.* (2001) Embo J. 20:1829). The present analysis adds to this putative mechanism the exiting E-tRNA and a swinging intersubunit bridge. The 16S RNA and the 30S proteins do not block this path and the movements of the head and the platform may assist the progression of the mRNA together with the E-site tRNA towards the exit site. Density was identified in the current map of D50S that may accommodate a large part of protein L1.

***The L7*/*L12 arm and the GTPase center*:** A major protruding region of domain II, that connects the solvent region with the front surface of the LRS consists of helices H42, H43 and H44 and the internal complex of L12 and L10. This stalk is involved in the contacts with the translocational factors and in factor-dependent GTPase activity (Chandra Sanyal S. and Liljas A. (2000) Curr Opin Struct Biol 10:633). Like other functionally important features, the entire L7/12 stalk is disordered in the H50S structure (Ban, N. *et al.* (2000) Science 289:905; Cundliffe, E. in *The Ribosome: Structure, Function and Evolution* (eds. Hill, W. E. *et al*.) 479-490 (ASM, Washington, DC, 1990)) but is well ordered in T70S (Yusupov, M. M. *et al.* (2001) Science 292:883). In D50S the RNA portion of this domain appears to be ordered, but the two proteins less so, although density that can host a large part of them has been identified. The location of part of this arm (H43) within D50S is somewhat shifted (by 3-4 Å) compared to its position within T70S. Consequently, the conformation of the entire arm in D50S is slightly less compact than its conformation in T70S.

One of the proteins associated with the L7/L12 arm, protein L11, appears in the structure of D50S. L11 together with the 23S rRNA stretch that binds it (the end of H42, H43 and H44), are associated with elongation factor and GTPase activities (Cundliffe, E. *et al.* (1979) J Mol Biol 132:235). This highly conserved region is the target for the antibiotic thiostrepton, and it has been shown that cells acquire resistance to this antibiotic by deleting protein L11 from their ribosomes. Large ribosomal subunits lacking protein L11 do not undergo major conformational changes (Franceschi, F. *et al.* (1994) Syst. & App. Microbiology 16:697), but cease to bind thiostrepton. Complexes containing L11, or one of its domains together with fragments mimicking the RNA stretch binding it, were subjected to crystallographic and NMR studies (Wimberly, B. T. *et al*. (1999) Cell 97:491; GuhaThakurta, D., and Draper, D. E. (2000) J Mol Biol. 295:569). Interestingly, whereas the structures of protein L11 determined in these studies were similar to that found within the ribosomal particle, there is less resemblance between the structures of the RNA fragments and that determined *in situ.*

***Analysis of disorder in intersubunit bridges of unbound 50S subunit:*** Several features, known to form intersubunit bridges are exceptionally well resolved in the map of D50S. These include helices H38 and H69, and proteins L5, L14 and L19. Helix H38, the longest stem in the large subunit, has a significant functional relevance as its upper half is the sole component forming the intersubunit bridge termed "B1a bridge" or "A-finger". In addition, one of its conserved internal loops interacts with the D- and T-loops of A-tRNA (Yusupov, M. M. *et al*. (2001) Science 292:883). In D50S it originates on the solvent side of the LRS, makes a sharp bend, and emerges between domain V and the 5S rRNA at the interface surface. Its location and orientation allow its contacts with protein S13.

The orientation of H69 with its universally conserved stem-loop in D50S is somewhat different than that seen in T70S. Both lie on the surface of the intersubunit interface but, in the 70S ribosome, H69 stretches towards the small subunit whereas in the free 50S subunit it makes more contacts with the large subunit (H71) so that the distance between the tips of their stem-loops is about 13.5 Å

Comparison of the two orientations of H69 (Figure 6) suggested that a small rotation of H69 in the free 50S subunit is sufficient for turning this helix into bridging position so that it can interact with the small subunit near the decoding center in Helix H44. In this position H69 can also contact the A-and P-site tRNA molecules and be proximal to elongation factor EF-G in the post-translocation state (Yusupov, M. M. *et al.* (2001) Science 292:883). Although it seems that H69 undergoes only subtle conformational rearrangements between the free and the bound orientations, it is clear that the displacement and the rotation of a massive helix such as H69 requires a high level of inherent flexibility. This may explain why in the high resolution structure of H50S, which was determined at far from physiological conditions, a distinct disadvantage, H69 is disordered (Ban, N. *et al.* (2000) Science 289:905).

Proteins L14 and L19 form an extended inter-protein beta sheet composed of two β-hairpin loops of L14 and two of L19 (Figure 7a). In H50S, there is no L19 but, L24e, although different in shape and smaller in size, is located at the same position and forms a similar β-sheet element. Both L14 and L19 are directly involved in intersubunit bridges. L19 is known to make contacts with the penultimate stem of the small subunit, at bridge B6. L14 contacts helix H14 of the 16S RNA to form bridge B8. It is likely, therefore, that the structural element produced by L14 and its counterpart (L 19 or L24e) has functional relevance in the construction of these two bridges. In D50S, these proteins, together with protein L3, form one of the two intimately connected protein clusters, consistent with the large number of such crosslinks reported (Walleczek, J. *et al* (1989) Biochemistry 28:4099). This clustering may enhance the stability of the structural features required for the intersubunit bridges.

Protein L5, together with S13 which is located in the head of the small subunit, form the only intersubunit bridge (B1b) which is composed exclusively of proteins, (Yusupov, M. M. *et al.* (2001) Science 292:883). The entire domain of L5 which is involved in this bridge, like almost all of the RNA features forming bridges with the small subunit, that appear to be fully ordered in T70S and almost so in D50S, are missing in H50S. Additional RNA features that are involved in intersubunit contacts are helices H62, H64, H69 and the lateral arm composed of H68-H71. All are present in the D50S structure in a fashion that allows their interactions with the small subunit and have similar conformations as those seen in T70S (Yusupov, M. M. *et al*. (2001) Science 292:883).

Based on the disorder observed in almost all functional features of H50S, it was assumed that most of the features involved in subunit function and in mediating intersubunit contacts are disordered in free LRSs and become stabilized in the 70S ribosome, (Yusupov, M. M. *et al.* (2001) Science 292:883). The finding that many of the disordered features in H50S are ordered in D50S indicates that the H50S crystal structure contain features that flex more than those in that of D50S. Thus, it is likely that structures derived from crystallized H50S subunits (Franceschi, F. *et al.* (1994) Syst. & App. Microbiology 16:697) represent conformations attained under environmental conditions close to those suitable for selective detachment of the proteins missing in this structure and hence that structural information obtained according to the present invention is significantly more accurate than that obtained for H50S.

### Evolutionary implications:

***The nascent-protein exit tunnel become tighter with evolution:*** More than three decades ago biochemical studies showed that the most newly synthesized amino acids of nascent proteins are masked by the ribosome (Malkin, L. I., and Rich, A. (1967) J Mol Biol. 26:329; Sabatini, D. D. and Blobel, G. (1970) J Cell Biol 45:146). A feature which may account for this masking was first seen as a narrow elongated region in images reconstructed at very low resolution (60 A) in 80S ribosomes from chick embryos (Milligan, RA. and Unwin PN. (1986) Nature: 693) and at 45 Å in images of 50S subunits of B. *stearothermophilus* (Yonath, A. *et al.* (1987) Science 236:813). Despite such low resolutions, these studies showed that this tunnel spans the large subunit from the location assumed to be the peptidyl transferase site to its lower part, and that it is about 100 Å in length and 15 Å in diameter, as subsequently confirmed at high resolution in H50S (Nissen, P. *et al*. (2000) Science 289:920) and in D50S.

The structural features building the walls of the tunnel, their chemical composition and their "nonstick" character in H50S are described in (Nissen, P. *et al.* (2000) Science 289:920). Although the same gross characteristics were identified in D50S, namely a lack of well-defined structural motifs, large patches of hydrophobic surfaces and low polarity, on average, the tunnel in D50S was strikingly wider than in H50S. This widening effect is caused by missing segments, such as the loop (residues 72-77) of protein L4 that in H50S penetrates into the tunnel and by several nucleotides that flip into the tunnel, or by the lower exposure of nucleotides, such as A2581 in D50S, compared to A2637, counterpart thereof, in H50S.

The exit of the tunnel is located at the bottom of the ribosomal particle. The tunnel in D50S is composed of domains I and III, and several proteins including L4, L22-L24 and L29. In H50S, however, L31e and L39e, two proteins that do not exist in D50S, are also part of the lower section of the tunnel and cause its tightening. Of interest is L39e, a small protein having an extended non-globular conformation, which penetrates into the RNA features lining the walls of the tunnel in that region. This protein replaces L23 in D50S and, since it is built of an extended tail, it can penetrate deeper into the tunnel walls than the loop of L23 in D50S. The globular domain of protein L23 in D50S, is similar to that of L23 in H50S, and both are positioned in the same location. However, the halophilic L23 has a very short loop compared to H23 in D50S and, in H50S, protein L39e occupies the space taken by the extended tail of L23 in D50S.

L39e is present in archaea and eukaryotes, but not in eubacteria. Thus, it seems that with the increase in cellular complication, and perhaps as a consequence of the high salinity, a tighter control on the tunnel's exit was required, and two proteins, HL23 and L39e, replace single one. So far there are no indications for a connection between this replacement and evolution. Nevertheless, it is evident that a protein in this delicate position may mediate interaction between the ribosome and other cellular components, evolving further to act as a hook for the ribosome on the ER membrane. A high resolution structure of a eukaryotic ribosome, bound to the ER membrane, should provide an answer to these open questions.

### Evolving structural elements:

***Helix H25:*** Helix H25 displays the greatest sequence diversity among eubacterial and halophilic large subunits. It contains 27 nucleotides in D50S and 74 in H50S (Figure 7c). It lies on the solvent side of the subunit and, in D50S, the region that is occupied by this helix in H50S hosts proteins L20 and L21. These two proteins exist in many eubacterial ribosomes but not in that of *H. marismortui* which evolved later than *D. radiodurans.* Protein L21 has a small β-barrel-like domain that is connected to an extended loop. Protein L20, in contrast, is built of a long α-helical extension with hardly any globular domain. Its shape and location make it a perfect candidate for its being a protein having a role in RNA organization. This may explain why L20 is one of the early assembly proteins and why can it take over the role of L24 in mutants lacking the latter.

The replacement of proteins by an RNA helix should be rather surprising, since in this way the ribosome could have lost two strong structure-stabilizing elements. However, in this case, regardless of the effects of the extension of helix H25 in archaea and eukaryotes, this did not reduce stabilization of this region since protein L32e has looped tails having sufficient length to compensate for many of the contacts made by the tail of L20 and the loop of L21 (Figures 7c-d). It is therefore likely that the loop of L32e organizes the RNA environment in H50S in a fashion similar to the loop of L21 in D50S. The globular domains of proteins L32e and L21 appear to be similar and it is likely that L21 and L32e are indeed evolutionarily related. The globular domain of L32e is rotated by 180°, relative to that of L21, around an axis defined by its tail, and the unoccupied space in H50S corresponding to the location of the globular domain of L21 is occupied by the extension of H25.

***The "protein-tweezers" motif:*** Among the novel protein structures of D50S are two Zn-finger proteins; L32 and L36 that do not exist in H50S and have no replacements or counterparts therein. The position occupied by L32 in D50S overlaps that hosting the loop of L22 in H50S and, in D50S, L32 and L22 form a tweezers-like motif possibly clamping interactions between domains II, III and IV (junctions H26/H47 and H61/H72) (Fig 7f). These two proteins interact extensively with protein L17, an additional novel protein that occupies the location of L31e in H50S, and the entire region seems to be highly stabilized. The question, still to be answered is: why, with evolution, was a protein replaced by a loop of another one even though this replacement seems to cause partial loss of stability of a well organized structural motif.

***The E-site tRNA:*** The E-site tRNA may interact, in D50S to the end of the extended loop of protein L31. In H50S, the region interacting with E-site RNA is provided by the extended loop of L44e. These two proteins are located at opposite sides of the location of the E-site tRNA, yet the interactions occur at approximately the same place, via their extended loops (Figure 7e). In D50S, protein L33, which has no extended loop, occupies the space taken by the globular domain of L44e in H50S, and the globular domains of both are rather similar. These complicated rearrangements may indicate that with evolution the ribosome preserved the configurations and locations of the features involved in the peptide bond formation.

***Helix H30b:*** Helix H30b, which does not exist in D50S, is located on the solvent surface in H50S and makes extensive contacts with protein L18e, a protein which does not exist in D50S, and with the lower part of H38. Protein L18e, in turn, connects H30b to H27 and to the loop of H45 and interacts with proteins L4 and L15. This RNA-protein network seems to be rather rigid and its strategic location may indicate that it protects the ribosomal surface from the increasing complexity of the environment.

***Concluding remarks:*** The LRS has a compact structure, its core is built of well-packed interwoven RNA features and it is known to have less conformational variability than the small subunit. Nevertheless, it assumes conformations which can be correlated to the functional activity of the ribosome. Analysis of results obtained while reducing the present invention to practice support linkage of the functional activity of the ribosome and the flexibility of its features. Based on comparison between the structures of free D50S and that of bound T50S, it is suggested that the ribosome utilizes the inherent flexibility of its features for facilitating specific tasks. Remarkable examples of such characteristics are displayed by helix H69, which creates the 50S hook in the decoding region of the small subunit, and the entire L1 arm, which produces the revolving gate for exiting tRNA molecules.

The striking difference between the conservation of the rRNA fold and the significant diversity of ribosomal proteins indicates that the latter do not only have roles in stabilizing rRNA conformation, but also play a role in binding of factors and substrates and in enhancing intersubunit association. The extended protein termini and the long protein loops are mainly buried within the ribosomal particle and thus are trapped in distinct conformations. However, those which are pointing outside, such as protein S18 in the small subunit (Pioletti, M. *et al.* (2001) Embo J. 20:1829), the loop of L5 and the N-terminus of L27, maintain a high level of flexibility and are available to interact, to bind and to enhance the placement of factors and substrates. It is therefore conceivable that in these cases the diversity of ribosomal proteins is linked to the evolution of the interacting components.

Remarkable preservation of structural motifs was observed in ribosomal proteins despite their overall conformational and sequence differences. The L14/L19 inter-protein β-sheet (Figure 6) shows how functional requirements can be satisfied in evolving ribosomes. Similarly, addition of a protein for creating a tighter tunnel opening was identified as the mechanism employed for reducing freedom of movement of nascent proteins in the increasingly complex environment of higher organisms.

The unique three-domain structure of CTC and the topology of these domains in D50S (Figure 4) may indicate that ribosomes of a stress-resistant bacterium control the incorporation of amino acids into growing chains by restricting the space allocated for the A-site tRNA. The positioning of a two-domain homologous protein (TL5) in T50S suggests a mechanism for stabilizing ribosomal function elevated temperatures. In addition, *D. radiodurans* has evolved a third domain in its LRS enabling it to survive under extreme conditions.

***Summary:*** The present results describe the 3.0 Å resolution structure of the large ribosomal subunit of the gram-positive mesophilic bacterium *D. radiodurans* (D50S). The RNA folds of D50S and 50S from *Haloarcula marismortui* (H50S) are similar, yet the functionally relevant features of D50S are ordered, in contrast to the disorder observed, presumably due to crystal environment, in the structure of unbound H50S. Analysis revealed replacement of a single D50S protein by two H50S proteins, while tightening the nascent protein tunnel, and indicated strategies that may partially account for survival under stressful conditions. The present analysis confirms that utilization of inherent flexibility for functional tasks is a common ribosomal strategy, and suggest how the L1-arm facilitates the exit of tRNA and how H69 creates the intersubunit bridge to the decoding center.

Thus, while reducing the present invention invention to practice, the present inventors have generated the first essentially complete model of the high resolution 3D atomic structure of a bacterial LRS which also represents the first such model of a eubacterial LRS. Such a model, therefore, constitutes a dramatic breakthrough in the art, representing the culmination of decades of intensive research aimed at elucidating the extremely complex 3D atomic structure and vital functional mechanisms of the ribosome. As such, the present ribosomal structure model is far superior to all prior art ribosome structure models and provides a critical and potent tool for enabling the rational design or identification of bacterial antibiotics, an urgent medical imperative, particularly in light of the current global epidemics of diseases associated with antibiotic resistant strains of bacteria. The present model also constitutes a potent means enabling the rational design or identification of LRSs having desired characteristics, such as, for example, conferring enhanced protein production capacity for example, for production of recombinant proteins. Also, importantly, the present model constitutes a powerful means for facilitating the elucidation of the vital and universal biological process of protein translation performed by the ribosome.

### EXAMPLE 2

### Growth of antibiotic-LRS complex crystals and high resolution 3D atomic structure models of the interaction of antibiotics with the large ribosomal subunit

The LRS is the functional binding target for a wide range of antibiotics. As such, models of the structural and functional atomic interactions between antibiotics and the LRS are urgently required since, for example, these would constitute an indispensable and powerful tool for the rational design or selection of antibiotics or of ribosomes having desired characteristics, as described above. In particular, the ability to rationally design or select antibiotics is of paramount medical importance due to currently expanding global epidemics of increasing numbers of lethal diseases caused by antibiotic resistant strains of pathogenic microorganisms. However, all prior art approaches have failed to produce satisfactory high resolution 3D atomic models of the structural and functional interactions between antibiotics and the LRS. Thus, in order to fulfill this urgent need, high resolution 3D atomic structure models of the LRS complexed to the antibiotics chloramphenicol, clindamycin, clarithromycin, erythromycin and roxithromycin were generated while reducing this aspect of the present invention to practice, as follows.

### Materials and Methods:

***Base Numbering:*** Bases of the 23S rRNA sequence of D. *radiodurans* and of the corresponding *E. coli* sequence are numbered with "Dr" and "Ec" appended as a suffix to the base number for respective identification thereof.

***Cell culture:*** *D. radiodurans* cells were cultured as recommended by the American Tissue Type Culture Collection (ATCC), using ATCC medium 679 with minor modifications.

***Growth of D50S crystals:*** *D. radiodurans* LRSs were isolated, and crystals of D50S belonging to the space-group I222 were grown as described in Example 1, above. Co-crystallization of D50S with antibiotics was carried out in the presence of 0.8-3.5 mM of the antibiotics chloramphenicol, clindamycin, erythromycin, and roxithromycin. Co-crystallization of D50S with clarithromycin was achieved by soaking D50S crystals in solutions containing 0.01 mM of this antibiotic.

***X-ray diffraction:*** Data were collected at 85 K from shock-frozen crystals with a bright SR beam at ID19 at APS/ANL, ID14/2 and 4 at ESRF/EMBL, and at BW6 at DESY. Data were recorded on MAR345, Quantum 4, or APS-CCD detectors and processed with HKL2000 (Otwinowski, Z. and Minor, W. (1997) Macromolecular Crystallography, Pt A 276:307).

***Placements and refinement:*** The 3.1 Å structure of D50S described in Example 1, above, was refined against the structure factor amplitudes of each of the antibiotic-D50S complexes, using rigid body refinement as implemented in CNS (Brunger, A. T. *et al.* (1998) Acta Crystallographica Section D-Biological Crystallography 54:905). SigmaA-weighted difference maps were used for the initial manual placement of the antibiotics. Each of the D50S-antibiotic models was further refined in Refmac (Murshudov, G. N. *et al.* (1999) Acta Cryst. section D55, 247). For the calculation of free R-factor a subset of reflections (10 % of the data) was omitted from the refinement. The structure coordinates of the antibiotic-D50S complexes were submitted to the PDB under accession numbers 1JZX, 1JZY, 1JZZ, 1K00, and 1K01.

***Coordinates and figures:*** Coordinates of chloramphenicol, clindamycin, erythromycin, roxithromycin, and clarithromycin were taken from Cambridge Structural Database and antibiotic structures were modeled into the difference density based on their crystal structure. Figures were produced using RIBBONS (Carson, M. (1997) Macromolecular Crystallography, Pt B 277:493) or MOLSCRIPT (Kraulis, P. J. (1991) Journal of Applied Crystallography 24:946) software.

### Experimental Results:

Crystallographic data for antibiotic-D50S complexes were generated and are listed in Table 6.

**Table 6.**

| ***Antibiotic-D50S complex crystallographic data.*** | | | | | | |
|---|---|---|---|---|---|---|
| Antibiotic in complex | Resolution (Å) | Rsym (%) | Completeness (%) | <I/sig(I)> | Unit cell dimensions (Å) | R/R_{free} (%) |
| clindamycin | 35-3.1 | 11.8 (57.0) | 94.4 (82.2) | 6.5 (2.1) | 170.286 × 410.134 × 697.201 | 27.1/33.9 |
| erythromycin | 35-3.4 | 14.4 (51.7) | 67.0(64.1) | 6.2 (1.5) | 169.194 × 409.975 × 695.049 | 29.6/33.1 |
| clarithromycin | 50-3.5 | 9.8 (50.6) | 85.2 (78.1) | 8.1 (2.1) | 169.871 × 412.705 × 697.008 | 29.1/33.3 |
| roxithromycin | 50-3.8 8 | 10.7 (24.4) | 64.8 (66.8) | 6.2 (2.2) | 170.357 × 410.713 × 694.810 | 24.7/32.0 |
| chloramphenicol | 25-3.5 | 13.9 (60.7) | 62.8 (54.5) | 7.5 (2.0) | 171.066 × 409.312 × 696.946 | 29.1/33.1 |

***Three-dimensional atomic structures of antibiotic-LRS interactions:*** The 3D atomic structure of portions of the LRSs in crystallized chloramphenicol-, clindamycin-, clarithromycin-, erythromycin-, and roxithromycin-LRS complexes are defined by atomic structure coordinates (D. *radiodurans* numbering system) set forth in Tables 7, 8, 9, 10 and 11, respectively (refer to enclosed CD-ROM for Tables), as follows:
23S rRNA: atom coordinates 1-59533;
ribosomal protein L4: atom coordinates 59534-59731;
ribosomal protein L22: atom coordinates 59535-59862; and
ribosomal protein L32: atom coordinates 59863-59921.

The 3D atomic structure of the antibiotics, or portions thereof, in crystallized chloramphenicol-, clindamycin-, clarithromycin-, erythromycin-, and roxithromycin-LRS complexes are defined by HETATM coordinates 59925-59944 set forth in Table 7, HETATM coordinates 59922-59948 set forth in Table 8, HETATM coordinates 59922-59973 set forth in Table 9, HETATM coordinates 59922-59972 set forth in Table 10, and HETATM coordinates 59922-59979 set forth in Table 11, respectively (refer to enclosed CD-ROM for Tables).

The 3D atomic positioning of Mg²⁺ ions associated with crystallized chloramphenicol-, clindamycin-, clarithromycin-, erythromycin-, and roxithromycin-LRS complexes are defined by HETATM coordinates 59922-59924 set forth in Table 7, HETATM coordinates 59949-59950 set forth in Table 8, HETATM coordinates 59974-59975 set forth in Table 9, HETATM coordinates 59973-59974 set forth in Table 10, and HETATM coordinates 59980-59981 set forth in Table 11, respectively (refer to enclosed CD-ROM for Tables).

The 3D atomic structures of the portions of antibiotic-LRS complexes comprising the antibiotic and the 23S rRNA atoms located within 20 Å of at least one atom of the antibiotic in crystallized chloramphenicol-, clindamycin-, clarithromycin-, erythromycin-, and roxithromycin-LRS complexes are defined by the structural coordinates *(D. radiodurans* numbering system) set forth in Tables 12, 13, 14, 15, and 16, respectively (refer to enclosed CD-ROM for Tables). The HETATM coordinates in Tables 12-16 define the 3D atomic structures of their respective antibiotics, as described above, and the non-HETATM coordinates in these tables define the 3D atomic structure of the 23S rRNA atoms located within 20 Å of at least one atom of the antibiotic.

The electron density maps of the antibiotic-LRS complexes enabled unambiguous determination of the binding sites of the five antibiotics. The chemical nature of the antibiotic-D50S interactions could largely be deduced from the mode of binding. Based on these maps and on the available biochemical and functional data, the structural basis for the modes of action of the antibiotics chloramphenicol, clindamycin, erythromycin, clarithromycin, and roxithromycin is proposed.

All of the antibiotics analyzed were found to target D50S subunit at the peptidyl transferase cavity and were found to interact exclusively with specific nucleotides that have been assigned to a multi-branched loop of domain V of the 23S rRNA in the 2D structure. Nucleotides of 23S rRNA interacting with chloramphenicol (nucleotides 2044, 2430, 2431, 2479 and 2483-2485), clindamycin (nucleotides 2040-2042, 2044, 2482, 2484 and 2590) and all three macrolides (nucleotides 2040-2042, 2045, 2484, 2588 and 2589) are shown in Figures 8a, 9a and 10a, respectively. These findings explain previous mutational and footprinting data (reviewed in Spahn, C. M. T. and Prescott, C. D. (1996) Journal of Molecular Medicine-Jmm 74:423; Vester, B. and Garrett, R. A. (1988) EMBO Journal 7:3577; Polacek, N. in *RNA-Binding Antibiotics* (eds. Schroeder, R. & Wallis, M. G.) (Eurekah.Com, Incorporated, Georgetown., 2000)). None of the antibiotics examined showed any direct interaction with ribosomal proteins. Furthermore, binding of these antibiotics did not result in any significant conformational change of the peptidyl transferase cavity.

***Chloramphenicol:*** At its single binding site, chloramphenicol targets the peptidyl transferase center mainly via hydrogen bond interactions. Chloramphenicol contains several reactive groups capable of forming hydrogen bonds, including the oxygen atoms of the para-nitro (p-NO₂) group, the 1OH and 3OH groups and the 4' carboxyl group.

One of the oxygen atoms of the p-NO₂ group of chloramphenicol is in a position to form hydrogen bonds with N1 of U2483Dr (U2504Ec) and N4 of C2431Dr (C2452Ec) which have been shown to be involved in chloramphenicol resistance (Vester, B. and Garrett, R. A. (1988) EMBO Journal 7:3577). The other oxygen atom of the p-NO₂ group interacts with O2' of U2483Dr (U2504Ec) (Figures 8a-b).

The 1OH group of chloramphenicol is located at hydrogen bonding distance (about 4 Å) from N1 and N2 of G2044Dr (G2061Ec) of the 23S rRNA. This nucleotide has been implicated in chloramphenicol resistance in rat mitochondria (Vester, B. and Garrett, R. A. (1988) EMBO Journal 7:3577) and a mutation of the neighboring nucleotide, A2062Ec, confers resistance to chloramphenicol in *H. halobium* (Mankin, A. S. and Garrett, R. A. (1991) J Bacteriol. 173:3559).

The 3OH group of chloramphenicol is fundamental for its activity. The most common chloramphenicol resistance mechanisms involve either acetylation or phosphorylation of this OH group, a modification which renders chloramphenicol inactive (Izard, T. and Ellis, J. (2000) Embo Journal 19, 2690; Shaw, W. V. and Leslie, A. G. W. (1991) Annu. Rev. Biophys. Biophys. Chem. 20:363). The 3OH group is within hydrogen bonding distance to 4'O of U2485Dr (U2506Ec). The 3OH group of chloramphenicol is also involved in interactions coordinated via a hydrated Mg²⁺ ion (Mg-C1, see following section).

The 4' carbonyl group of chloramphenicol could potentially form a hydrogen bond with the 2'OH of U2485Dr (U2506Ec) and the 2'OH of A2430Dr (A2451Ec), as it is located at a distance of about 4.3 Å from these positions (see Figure 8a). These findings are consistent with mutations of A2430Dr (A2451Ec) resulting in chloramphenicol resistance (Thompson, J. *et al*. (2001) Proc Natl Acad Sci U S A. 98:9002; Vester, B. and Garrett, R. A. (1988) EMBO Journal 7:3577). A stereo view showing the chloramphenicol binding site at the peptidyl transferase cavity of D50S is shown in Figure 8c.

***Mg***^{***2+***}***-antibiotic interactions:*** In addition to the hydrogen-bonds between chloramphenicol and 23S rRNA residues, two hydrated Mg²⁺ ions are involved in chloramphenicol binding, Mg-C1 and Mg-C2, which are not present in the native D50S structure, nor in the complexes of D50S with the other analyzed antibiotics. Thus, their presence at these particular locations depends on chloramphenicol binding.

Mg-C1 mediates the interaction of the 3OH group of chloramphenicol with the 04 atom of U2485Dr (U2506Ec) and with the 2'OH group and 4'O atom of G2484Dr (G2505Ec). Studies have suggested that both of these nucleotides are protected by chloramphenicol (Rodriguez-Fonseca, C., Amils, R. and Garrett, R. (1995) Journal of Molecular Biology 247:224). Mg-C2 mediates the interaction of one of the oxygen atoms of the p-NO₂ group with the 02 of U2479Dr (U2500Ec), 04 U2483Dr (U2504Ec), and 02 of C2431Dr (C2452Ec) via a salt bridge. This interaction further stabilizes the interaction of chloramphenicol with the peptidyl transferase cavity. The presence of Mg²⁺ appears crucial for its interaction with and inhibition of the ribosome.

Interestingly, the Mg²⁺ ion (Mg101) found overlapping with the chloramphenicol location in the native structure is not observed in the chloramphenicol-50S complex, suggesting that the coordinating effect of chloramphenicol is sufficient to maintain the local structure of the 50S subunit in the absence of Mg101. The displacement of Mg101 by chloramphenicol and the coordinating effects of Mg-C1 and Mg-C2 in the presence of chloramphenicol, could provide a partial explanation as to why chloramphenicol, in spite of being a relatively small molecule, has been chemically footprinted to many different positions on the peptidyl transferase ring.

Generation of new metal ion binding sites due to antibiotic-binding as observed for chloramphenicol, may explain the mode of action and/or binding of other drugs as well and may be used as a tool in rational drug design.

***Clindamycin:*** Although the binding site for the lincosamide clindamycin in the peptidyl transferase center is different from that of chloramphenicol, it appears to be partially overlapping (Figures 9a and 11). Novel Mg²⁺ ions involved in the binding of clindamycin were not identified, however, as observed for chloramphenicol, the binding of clindamycin displaced Mg 101.

Clindamycin has three hydroxyl groups in its sugar moiety that can participate in hydrogen bond formation (see Figures 9a and 9b). The 2OH of clindamycin appears to form a hydrogen bond with N6 of A2041Dr (A2058Ec). A2041Dr (A2058Ec) is the pivotal nucleotide for the binding of lincosamide antibiotics (Douthwaite, S. (1992) Nucleic Acids Research 20:4717). Although the 2OH group is less than 4.5 Å away from N6 of A2040Dr (G2057Ec) and 04 of U2590Dr (C2611Ec), additional hydrogen bonds to these nucleotides are unlikely because mutations of A2040Dr (G2057Ec) and U2590Dr (C2611Ec) are thought to alter only the conformation of the 23S rRNA and thus affect nucleotide A2041Dr (G2058Ec) (see Figure 9b). A stereo view showing the clindamycin binding site at the peptidyl transferase cavity of *D. radiodurans* is shown in Figure 9c.

The 3OH group interacts with N6 of nucleotide A2041Dr (A2058Ec) and non-bridging phosphate-oxygens of G2484Dr (G2505Ec). The distances from these moieties to the 3OH group are compatible with hydrogen bond formation. Thus, N6 of A2041Dr (A2058Ec) can interact with both the 2OH and 3OH groups of clindamycin. These structural data explain in the most straightforward way why A2041Dr (A2058Ec) mutations confer resistance. The hydrogen bond to N6 of nucleotide A2041Dr (A2058Ec) can also explain why the dimethylation of the N6 group, which disrupts the hydrogen bonds, causes resistance to lincosamides (Ross, J. I. *et al.* (1997) Antimicrobial Agents & Chemotherapy 41:1162). The 3OH group of clindamycin can additionally interact with N1 of A2041Dr (A2058Ec), N6 of A2042 (A2059Ec), and the 2OH of A2482Dr (A2503Ec).

The 4OH group of clindamycin is close enough to the 2'OH of A2482Dr (A2503Ec) and to N6 and N1 of A2042 (A2059Ec) to form hydrogen bonds. This interaction explains why mutations in A2042 (A2059Ec) cause clindamycin resistance in several bacterial pathogens (Ross, J. I. *et al.* (1997) Antimicrobial Agents & Chemotherapy 41:1162).

The 8' carbon of clindamycin points towards the puromycin binding site, and is located about 2.5 Å from the N3 of C2431Dr (C2452Ec). The sulfur atom of clindamycin is located about 3 Å from base G2484Dr (G2505Ec) of the 23S rRNA. Although both of these groups cannot form hydrogen bonds, possible interactions, such as van der Waals or hydrophobic interactions, between these groups and nucleotides of the 23S rRNA may be expected.

***Macrolide antibiotics (erythromycin, clarithromycin, roxithromycin):*** As was found to be the case for chloramphenicol and clindamycin, the binding site of the macrolides is composed exclusively of 23S rRNA and does not involve any interactions with ribosomal proteins (Figures 10a-d). The three macrolides analyzed were found to bind to a single site, at the entrance of the tunnel in D50S. The erythromycin and clarithromycin binding sites in the D50S peptidyl transferase cavity were found to be identical (Figure 10c). The roxithromycin binding site at the peptidyl transferase cavity of D50S is shown in Figure 10d. Their binding contacts clearly differ from those of chloramphenicol, but overlap to a large extent with those of clindamycin (see Figure 11).

Most of the 14-member ring macrolides, which includes erythromycin and its related compounds, have three structural components: the lactone ring, the desosamine sugar, and the cladinose sugar. The reactive groups of the desosamine sugar and the lactone ring mediate all the hydrogen-bond interactions of erythromycin, clarithromycin, and roxithromycin with the peptidyl transferase cavity.

In all the macrolides examined, the 2'OH group of the desosamine sugar appears to form hydrogen bonds with three positions: N6 and N1 of A2041Dr (A2058Ec) and N6 of A2042Dr (A2059Ec).

These structural results explain not only the genetic studies involving 23S rRNA mutations in macrolide resistance, but also most of the results of macrolide modification in structure activity relation (SAR) studies. The hydrogen bonds between the 2'OH and N1 and N6 of A2041Dr (A2058Ec) explain why this nucleotide is essential for macrolide binding and also shed light on the two most common ribosomal resistance mechanisms against macrolides; the N6 di-methylation of A2041Dr (A2058Ec) by Erm-family methylases (reviewed in Weisblum, B. (1995) Antimicrobial Agents & Chemotherapy 39:577) and the product of rRNA mutations changing nucleotide identity at this position (Sigmund, C. D. *et al.* (1984) Nucleic Acids Research 12:4653). The di-methylation of the N6 group would not only add a bulky substituent causing steric hindrance for the binding, but would prevent the formation of hydrogen bonds to the 2'OH group. The mode of interactions proposed by the present structure implies that a mutation at A2041Dr (A2058Ec) to a nucleotide other than adenine would disrupt the hydrogen bonding pattern, therefore impairing binding and rendering bacteria antibioticresistant. A2041Dr (A2058Ec) is one of the few nucleotides of the peptidyl transferase ring which is not conserved among all phylogenetic domains. Sequence comparisons show that mitochondrial and cytoplasmic rRNAs of higher eukaryotes have a guanosine at position 2058Ec of the LRS RNA (Bottger, E. C. *et al.* (2001) Embo Reports 2:318). Therefore, the proposed mode of interaction explains the selectivity of macrolides for bacterial ribosomes.

These results also explain the importance of the 2'OH group of the desosamine sugar for erythromycin binding known from SAR studies (Mao, J. C.-H. and Puttermann, M. (1969) Journal of Molecular Biology 44:347) since changing the 2'OH group will not allow the suggested mode of interaction to take place. The 2'OH of the desosamine sugar is located about 4 Å away from N6 of A2040Dr (G2057Ec), a nucleotide forming the last base pair of helix 73. Although an interaction with this group cannot be ruled out, this base pair is likely engaged in maintaining the proper conformation of A2041Dr (A2058Ec), rather than interacting directly with the macrolides.

The dimethylamino group of the desosamine sugar exists in both protonated (> 96 % to ≤ 98 %) and neutral (2-4 %) form. This group, if protonated, could interact via ionic interactions in a pH dependent manner with the backbone oxygen of G2484Dr (G2505Ec) (see Figure 10a). At physiological pH, both species appear to be able to bind ribosomes with the same kinetics (Goldman, R. C. *et al*. (1990) Antimicrobial Agents & Chemotherapy 34:426). However, the only study that revealed a strong correlation between pH and potency of inhibition of *in vitro* protein synthesis for the macrolide erythromycin concludes that the neutral macrolide form was the inhibitory species. If the neutral macrolide form is in fact the inhibitory species, a hydrogen-bond between the dimethylamine of the macrolides and the nucleotide G2484Dr (G2505Ec) would not be formed.

The structural information presented herein suggest that it would be of interest to derivatize macrolides at the dimethylamine position. Such modifications could improve binding and in addition, could result in inhibition of peptidyl transferase activity. One of the few macrolides able to inhibit peptidyl transferase is tylosin. Tylosin, which contains a mycarose moiety, has been shown to protect A2506Ec, the neighboring nucleotide of G2484Dr (G2505Ec), from chemical modification (Poulsen, S. M., Kofoed, C. and Vester, B. (2000) Journal of Molecular Biology 304:471). Similarly, modification of the dimethylamine group to a reactive group that would interact with G2484Dr (G2505Ec) in a pH independent manner may lead to more effective macrolides and structurally related compounds.

Only three of the hydroxyl moieties of the lactone ring are within hydrogen bonding distance to the 23S rRNA. The 6-OH group is within hydrogen bonding distance to N6 of A2045Dr (A2062Ec) and likely interacts with it. Although this group has been substituted by a metoxy group to improve acid stability in clarithromycin, the oxygen of the metoxy group is still at hydrogen bonding distance to N6 of A2045Dr (A2062Ec). The presently disclosed structure explains why the chemical footprinting experiments implicate this nucleotide in macrolide binding (Hansen, L. H., Mauvais, P. and Douthwaite, S. (1999) Molecular Microbiology 31:623).

The 11-OH and 12-OH groups of the lactone group may hydrogen-bond with the 04 of U2588Dr (U2609Ec). Hydrogen bonds at these positions could explain why substitutions of any of these two hydroxyl groups cause a moderate decrease in binding, as would be expected for groups hydrogen-bonding with the same 23S rRNA nucleotide (Mao, J. C.-H. and Puttermann, M. (1969) Journal of Molecular Biology 44:347).

The reactive groups of the cladinose sugar are not involved in hydrogen bond interactions with the 23S rRNA. Cladinose dispensability was confirmed by SAR studies (Mao, J. C.-H. and Puttermann, M. (1969) Journal of Molecular Biology 44:347), showing that the 4"-OH is dispensable for the binding. A closely related group of antibiotics, the ketolides, which bind more tightly than macrolides to ribosomes, do not have a cladinose sugar. Moreover, the Kd of RU56006, a derivative of erythromycin lacking the cladinose sugar, is of the same order of magnitude as that of erythromycin (Hansen, L. H., Mauvais, P. and Douthwaite, S. (1999) Molecular Microbiology 31:623).

Studies have shown that the tip of helix 35 in domain II of 23S rRNA has been implicated in the binding of erythromycin (Hansen, L. H., Mauvais, P. and Douthwaite, S. (1999) Molecular Microbiology 31:623; Xiong, L. Q., Shah, S., Mauvais, P. and Mankin, A. S. (1999) Molecular Microbiology 31:633). Although the presently disclosed structure does not suggest direct interaction between helix 35 and erythromycin, the distance between the OH-11 of the lactone ring and the N4 of C765Dr (A752Ec), a nucleotide proposed to interact with erythromycin, is 8.5 Å (Hansen, L. H., Mauvais, P. and Douthwaite, S. (1999) Molecular Microbiology 31:623; Xiong, L. Q., Shah, S., Mauvais, P. and Mankin, A. S. (1999) Molecular Microbiology 31:633), whereas the CH3 group branching from position 13 of the lactone ring is located about 4.5 Å from the 02 of C759Dr (G745Ec) (see Figure 10a). Therefore, hydrophobic, van der Waals, or ion coordinated interactions between the lactone ring of macrolides and this loop of the 23S rRNA cannot be ruled out. In this case, footprinting effects at A752Ec might be of an allosteric nature.

The two ribosomal proteins that have been implicated in erythromycin resistance are L4 and L22 (Wittmann, H. G. *et al.* (1973) Molecular & General Genetics 127:175). The closest distance of erythromycin (12-OH) to L4 (Arg1 11Dr/Lys90Ec) is 8 Å, whereas the closest distance from (8-CH₃) to L22 (Gly63Dr/Gly64Ec) is 9 Å. These distances are more than would be expected for any meaningful chemical interaction. Therefore, the macrolide resistance acquired by mutations in these two proteins is probably the product of an indirect effect that is produced by a perturbation of the 23S rRNA due to the mutated proteins (Gregory, S. T. & Dahlberg, A. E. (1999) Journal of Molecular Biology 289:827).

The principal difference between the three macrolides analyzed in this study is seen at the 9 keto group of the lactone ring. Although there is no difference in the binding mode of erythromycin and clarithromycin, there is a difference in roxithromycin where an etheroxime chain substitutes the 9 keto group. The present electron density map clearly shows a small part of this etheroxime chain pointing towards the inside of the tunnel. This part of the chain is not involved in the interaction between roxithromycin and 23S rRNA or ribosomal proteins (see Figure 10c).

***Overlapping binding sites:*** The present results show that a subset of the 23S rRNA nucleotides involved in the hydrogen bond interactions with chloramphenicol is also involved in interactions with clindamycin. This subset consists of G2044Dr (G2061Ec) and G2484Dr (G2505Ec), whose contact with chloramphenicol is mediated via a Mg²⁺ ion. Both nucleotides have previously been shown by chemical footprinting to be protected by binding of chloramphenicol or clindamycin (Polacek, N. in *RNA-Binding Antibiotics* (eds. Schroeder, R. & Wallis, M. G.) (Eurekah.Com, Incorporated, Georgetown., 2000)) and a mutation at position G2061Ec has also been reported to confer resistance to chloramphenicol (Vester, B. & Garrett, R. A. (1988) EMBO Journal 7:3577).

The interaction of the 23S rRNA with clindamycin and with the macrolides also involves some common nucleotide moieties. These are N6 of A2041Dr (A2058Ec), N6 of A2042Dr (A2059Ec), and the non-bridging phosphate oxygen of U2484Dr (U2505Ec). These positions are targeted by the sugar moiety of clindamycin and the desosamine sugar of the macrolides. These two sugars are located at almost identical locations in the structure of the 50S-clindamycin and 50S-macrolide complexes. The overlapping of binding sites may explain why clindamycin and macrolides bind competitively to the ribosome and why most RNA mutations conferring resistance to macrolides also confer resistance to lincosamides (Fournet, M. P. *et al.* (1987) *J. Pharm. Pharmacol.* 39:319).

Nucleotide G2484Dr (G2505Ec) is targeted by all antibiotics tested in this study. The importance of this nucleotide position has been previously established (Saarma, U. *et al.* (1998) Rna-a Publication of the Rna Society 4:189). Although this nucleotide has been shown to be protected from chemical modification upon chloramphenicol, lincosamide, or macrolide binding (Rodriguez-Fonseca, C. *et al.* (1995) Journal of Molecular Biology 247:224; Moazed, D. and Noller, H. F. (1987) Biochemie 69:879; Douthwaite, S. (1992) Nucleic Acids Research 20:4717), no mutations of G2484Dr (G2505Ec) conferring resistance to these antibiotics have been reported. In addition, G2484Dr (G2505Ec) is also one of the nucleotides protected upon binding of peptidyl-tRNA (Moazed, D. and Noller, H. F. (1991) Proc Natl Acad Sci U S A. 88:3725). The identity of this nucleotide is important for protein synthesis, albeit not for ribosome-antibiotic interactions where the position of its backbone oxygen or the 2'OH of the sugar appear to be the essential requirement.

Thus, the characterization of overlapping binding sites provided by the present studies indicates the essential 23S rRNA nucleotides which must be targeted by antibiotics in order to inhibit ribosomal function. Such characterization is extremely valuable for the rational design of combined antibiotic therapies and, moreover, can open the door for the design of hybrid antibiotics comprising novel combinations of ribosomal binding sites.

***Mechanism of antibiotic action:* The relative binding sites for** chloramphenicol, clindamycin, and erythromycin in 23S rRNA with respect to the A-site substrate analog CC-puromycin (Nissen, P. *et al.* (2000) Science 289:920) and the 3'end of P- and A-site tRNAs (Yusupov, M. M. *et al.* (2001) Science 292:883) docked in the present structure are shown in Figure 11. From these sites, it can be seen that chloramphenicol acts as a competitor of puromycin and thus, as an A-site inhibitor, consistent with previous findings (Vazquez, D. *Inhibitors of protein synthesis* (Springer Verlag, Berlin, Germany, 1975)). In contrast to puromycin, which acts as a structural analog of the 3'-end aminoacyl tRNA, the location of chloramphenicol in the present structure suggests that this drug may act by interfering with the positioning of the aminoacyl moiety in the A-site. Thus, chloramphenicol may physically prevent the formation of the transition state during peptide bond formation. In addition, the dichloromethyl moiety of chloramphenicol, a moiety shown to be important for chloramphenicol activity (Vince, R. *et al.* (1975) Antimicrobial Agents & Chemotherapy 8:439), is close enough to the amino acceptor group of CC-puromycin (Figure 11). The presence of such an electronegative group in the neighborhood of the amino acceptor could also hamper peptide bond formation.

Clindamycin clearly bridges the binding site of chloramphenicol and that of the macrolides (Figure 11) and overlaps directly with the A- and P-sites. Thus, its binding position provides a structural basis for its hybrid A-site and P-site specificity (Kalliaraftopoulos, S. *et al*. (1994) Molecular Pharmacology 46:1009). Furthermore, clindamycin has the capacity to interfere with the positioning of the aminoacyl group at the A-site and the peptidyl group at the P-site while also sterically blocking progression of the nascent peptide towards the tunnel.

Macrolides, on the other hand, are thought to block the progression of the nascent peptide within the tunnel (Vazquez, D. *Inhibitors of protein synthesis* (Springer Verlag, Berlin, Germany, 1975)) and, indeed, the present structure shows erythromycin as being located at the entrance of the tunnel (Figure 12). The macrolide binding site is located at a position that can allow the formation of 6-8 peptide bonds before the nascent protein chain reaches the macrolide binding site. Once macrolides are bound, they reduce the diameter of the tunnel from the original 18-19 Å to < 10 Å. However, since the space not occupied by erythromycin hosts a hydrated Mg²⁺ ion, the passage available for the nascent protein is effectively reduced to 6-7 Å in diameter. Moreover, in order to reach this narrow passage the nascent peptide needs to progress in a diagonal direction, thus imposing further limitations on the growing protein chain. These structural results are consistent with previous biochemical findings, showing that peptides of up to 8 residues can be produced by erythromycin-bound ribosomes (Tenson, T. *et al.* (1996) Proc NatI Acad Sci U S A. 93:5641). It is possible that ribosomes could escape the inhibitory effects of macrolides if proteins with leader sequences specifically capable of threading their way through the narrowed tunnel are translated.

Overall, the binding sites of the antibiotics analyzed in the present study suggest that their inhibitory action is not only determined by their interaction with specific nucleotides, some of them shown to be essential for peptidyl transferase activity and/or A- and/or P-tRNA binding. These antibiotics could also inhibit peptidyl transferase activity by interfering with the proper positioning and movement of the tRNAs in the peptidyl transferase cavity. This steric hindrance may be direct, as in the case of chloramphenicol or indirect as in the case of the three macrolides. In addition to causing steric hindrance, antibiotic-binding may physically link regions known to be essential for the proper positioning of the A- and P-tRNAs and thus prevent the conformational flexibility needed for protein biosynthesis.

The overall interaction of each analyzed antibiotic, together with the lack of any major conformational changes upon antibiotic-binding to the ribosome, supports the theory whereby the peptidyl transferase center evolved as a template for the proper binding of the activated substrates, the A-tRNA and the P-tRNA (Polacek, N. *et al.* (2001) Nature 411:498). However, the presence of a catalytic nucleotide cannot be completely excluded.

***Conclusion:*** The present analyses reveal at atomic resolution, for the first time, the structural and functional interactions involved in the binding of chloramphenicol, clindamycin, and the three macrolides erythromycin, clarithromycin, and roxithromycin to a bacterial ribosome and provide a wealth of novel and valuable information regarding the functional and structural basis of antibiotic resistance. As such, the present 3D atomic structure models are very clearly advantageous over prior art models of antibiotic-ribosome interaction. Such models can be used to elucidate the mechanisms whereby ribosomes perform the crucial biological process of protein translation and the mechanisms whereby antibiotics inhibit ribosome function. Crucially, these models constitute a powerful tool for the rational design or selection of novel antibiotics. This is of critical importance, particularly due to current epidemics of diseases caused by antibiotic resistant or multi-resistant strains of bacterial pathogens. Furthermore, the models of the present invention provide much novel and valuable information regarding the mechanisms of ribosome function *per se.* As such, these models can therefore be utilized to rationally design or select ribosomes having desired characteristics, such as enhanced protein production capacity, which can be used to enhance recombinant protein production. Thus, the unique antibiotic-LRS complex models of the present invention can be advantageously applied in a broad range of biomedical, pharmacological, industrial and scientific applications.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, and patent applications mentioned in this specification are herein incorporated in their entirety by reference into the specification, to the same extent as if each individual publication, patent, or patent application was specifically and individually indicated to be incorporated herein by reference. In addition, citation or identification of any reference in this application shall not be construed as an admission that such reference is available as prior art to the present invention.

## Claims

1. A composition-of-matter comprising a crystallized complex of an antibiotic bound to a large ribosomal subunit of a eubacterium.

2. The composition-of-matter of claim 1, wherein said eubacterium is *D. radiodurans.*

3. The composition-of-matter of claim 1, wherein said eubacterium is a gram-positive bacterium.

4. The composition-of-matter of claim 1, wherein said eubacterium is a coccus.

5. The composition-of-matter of claim 1, wherein said eubacterium is a Deinococcus-Thermophilus group bacterium.

6. The composition-of-matter of claim 1, wherein said antibiotic is clindamycin and whereas said crystallized complex is **characterized by** unit cell dimensions of a = 170.286 ± 10 Å, b = 410.134 ± 15 Å and c = 697.201 ± 25 Å.

7. The composition-of-matter of claim 1, wherein said antibiotic is erythromycin and whereas said crystallized complex is **characterized by** unit cell dimensions of a = 169.194 ± 10 Å, b = 409.975 ± 15 Å and c = 695.049 ± 25 Å.

8. The composition-of-matter of claim 1, wherein said antibiotic is clarithromycin and whereas said crystallized complex is **characterized by** unit cell dimensions of a = 169.871 ± 10 Å, b = 412.705 ± 15 Å and c = 697.008 ± 25 Å.

9. The composition-of-matter of claim 1, wherein said antibiotic is roxithromycin and whereas said crystallized complex is **characterized by** unit cell dimensions of a = 170.357 ± 10 Å, b = 410.713 ± 15 Å and c = 694.810 ± 25 Å.

10. The composition-of-matter of claim 1, wherein said antibiotic is chloramphenicol and whereas said crystallized complex is **characterized by** unit cell dimensions of a = 171.066 ± 10 Å, b = 409.312 ± 15 Å and c = 696.946 ± 25 Å.

11. The composition-of-matter of claim 1, wherein said crystallized complex is **characterized by** having a crystal space group of I222.

12. The composition-of-matter of claim 1, wherein said antibiotic is selected from the group consisting of chloramphenicol, a lincosamide antibiotic, clindamycin, a macrolide antibiotic, clarithromycin, erythromycin and roxithromycin.

13. The composition-of-matter of claim 1, wherein said antibiotic is chloramphenicol and whereas said large ribosomal subunit comprises a nucleic acid molecule, a segment of which including nucleotides being associated with said chloramphenicol, wherein a three-dimensional atomic structure of said nucleotides is defined by the set of structure coordinates corresponding to nucleotide coordinates 2044, 2430, 2431, 2479 and 2483-2485 set forth in Table 7.

14. The composition-of-matter of claim 1, wherein said antibiotic is chloramphenicol and whereas said large ribosomal subunit comprises a nucleic acid molecule, a segment of which including nucleotides being associated with said chloramphenicol, wherein a three-dimensional atomic structure of said nucleotides is defined by a set of structure coordinates having a root mean square deviation of not more than 2.0 Å from the set of structure coordinates corresponding to nucleotide coordinates 2044, 2430, 2431, 2479 and 2483-2485 set forth in Table 7.

15. The composition-of-matter of claim 13, wherein a three-dimensional atomic structure of said segment is defined by the set of structure coordinates corresponding to nucleotide coordinates 2044-2485 set forth in Table 7.

16. The composition-of-matter of claim 14, wherein a three-dimensional atomic structure of said segment is defined by a set of structure coordinates having a root mean square deviation of not more than 2.0 Å from the set of structure coordinates corresponding to nucleotide coordinates 2044-2485 set forth in Table 7.

17. The composition-of-matter of claim 1, wherein said antibiotic is chloramphenicol and whereas a three-dimensional atomic structure of said chloramphenicol is defined by the set of structure coordinates corresponding to HETATM coordinates 59925-59944 set forth in Table 7.

18. The composition-of-matter of claim 1, wherein said antibiotic is chloramphenicol and whereas a three-dimensional atomic structure of said chloramphenicol is defined by a set of structure coordinates having a root mean square deviation of not more than 2.0 Å from the set of structure coordinates corresponding to HETATM coordinates 59925-59944 set forth in Table 7.

19. The composition-of-matter of claim 1, wherein said antibiotic is clindamycin and whereas said large ribosomal subunit comprises a nucleic acid molecule, a segment of which including nucleotides being associated with said clindamycin, wherein a three-dimensional atomic structure of said nucleotides is defined by the set of structure coordinates corresponding to nucleotide coordinates 2040-2042, 2044, 2482, 2484 and 2590 set forth in Table 8.

20. The composition-of-matter of claim 1, wherein said antibiotic is clindamycin and whereas said large ribosomal subunit comprises a nucleic acid molecule, a segment of which including nucleotides being associated with said clindamycin, wherein a three-dimensional atomic structure of said nucleotides is defined by a set of structure coordinates having a root mean square deviation of not more than 2.0 Å from the set of structure coordinates corresponding to nucleotide coordinates 2040-2042, 2044, 2482, 2484 and 2590 set forth in Table 8.

21. The composition-of-matter of claim 19, wherein a three-dimensional atomic structure of said segment is defined by the set of structure coordinates corresponding to nucleotide coordinates 2040-2590 set forth in Table 8.

22. The composition-of-matter of claim 20, wherein a three-dimensional atomic structure of said segment is defined by a set of structure coordinates having a root mean square deviation of not more than 2.0 Å from the set of structure coordinates corresponding to nucleotide coordinates 2040-2590 set forth in Table 8.

23. The composition-of-matter of claim 1, wherein said antibiotic is clindamycin and whereas a three-dimensional atomic structure of said clindamycin is defined by the set of structure coordinates corresponding to HETATM coordinates 59922-59948 set forth in Table 8.

24. The composition-of-matter of claim 1, wherein said antibiotic is clindamycin and whereas a three-dimensional atomic structure of said clindamycin is defined by a set of structure coordinates having a root mean square deviation of not more than 2.0 Å from the set of structure coordinates corresponding to HETATM coordinates 59922-59948 set forth in Table 8.

25. The composition-of-matter of claim 1, wherein said antibiotic is clarithromycin and whereas said large ribosomal subunit comprises a nucleic acid molecule, a segment of which including nucleotides being associated with said clarithromycin, wherein a three-dimensional atomic structure of said nucleotides is defined by the set of structure coordinates corresponding to nucleotide coordinates 2040-2042, 2045, 2484, 2588 and 2589 set forth in Table 9.

26. The composition-of-matter of claim 1, wherein said antibiotic is clarithromycin and whereas said large ribosomal subunit comprises a nucleic acid molecule, a segment of which including nucleotides being associated with said clarithromycin, wherein a three-dimensional atomic structure of said nucleotides is defined by a set of structure coordinates having a root mean square deviation of not more than 2.0 Å from the set of structure coordinates corresponding to nucleotide coordinates 2040-2042, 2045, 2484, 2588 and 2589 set forth in Table 9.

27. The composition-of-matter of claim 25, wherein a three-dimensional atomic structure of said segment is defined by the set of structure coordinates corresponding to nucleotide coordinates 2040-2589 set forth in Table 9.

28. The composition-of-matter of claim 26, wherein a three-dimensional atomic structure of said segment is defined by a set of structure coordinates having a root mean square deviation of not more than 2.0 Å from the set of structure coordinates corresponding to nucleotide coordinates 2040-2589 set forth in Table 9.

29. The composition-of-matter of claim 1, wherein said antibiotic is clarithromycin and whereas a three-dimensional atomic structure of said clarithromycin is defined by the set of structure coordinates corresponding to HETATM coordinates 59922-59973 set forth in Table 9.

30. The composition-of-matter of claim 1, wherein said antibiotic is clarithromycin and whereas a three-dimensional atomic structure of said clarithromycin is defined by a set of structure coordinates having a root mean square deviation of not more than 2.0 Å from the set of structure coordinates corresponding to HETATM coordinates 59922-59973 set forth in Table 9.

31. The composition-of-matter of claim 1, wherein said antibiotic is erythromycin and whereas said large ribosomal subunit comprises a nucleic acid molecule, a segment of which including nucleotides being associated with said erythromycin, wherein a three-dimensional atomic structure of said nucleotides is defined by the set of structure coordinates corresponding to nucleotide coordinates 2040-2042, 2045, 2484, 2588 and 2589 set forth in Table 10.

32. The composition-of-matter of claim 1, wherein said antibiotic is erythromycin and whereas said large ribosomal subunit comprises a nucleic acid molecule, a segment of which including nucleotides being associated with said erythromycin, wherein a three-dimensional atomic structure of said nucleotides is defined by a set of structure coordinates having a root mean square deviation of not more than 2.0 Å from the set of structure coordinates corresponding to nucleotide coordinates 2040-2042, 2045, 2484, 2588 and 2589 set forth in Table 10.

33. The composition-of-matter of claim 31, wherein a three-dimensional atomic structure of said segment is defined by the set of structure coordinates corresponding to nucleotide coordinates 2040-2589 set forth in Table 10.

34. The composition-of-matter of claim 32, wherein a three-dimensional atomic structure of said segment is defined by a set of structure coordinates having a root mean square deviation of not more than 2.0 Å from the set of structure coordinates corresponding to nucleotide coordinates 2040-2589 set forth in Table 10.

35. The composition-of-matter of claim 1, wherein said antibiotic is erythromycin and whereas a three-dimensional atomic structure of said erythromycin is defined by the set of structure coordinates corresponding to HETATM coordinates 59922-59972 set forth in Table 10.

36. The composition-of-matter of claim 1, wherein said antibiotic is erythromycin and whereas a three-dimensional atomic structure of said erythromycin is defined by a set of structure coordinates having a root mean square deviation of not more than 2.0 Å from the set of structure coordinates corresponding to HETATM coordinates 59922-59972 set forth in Table 10.

37. The composition-of-matter of claim 1, wherein said antibiotic is roxithromycin and whereas said large ribosomal subunit comprises a nucleic acid molecule, a segment of which including nucleotides being associated with said roxithromycin, wherein a three-dimensional atomic structure of said nucleotides is defined by the set of structure coordinates corresponding to nucleotide coordinates 2040-2042, 2045, 2484, 2588 and 2589 set forth in Table 11.

38. The composition-of-matter of claim 1, wherein said antibiotic is roxithromycin and whereas said large ribosomal subunit comprises a nucleic acid molecule, a segment of which including nucleotides being associated with said roxithromycin, wherein a three-dimensional atomic structure of said nucleotides is defined by a set of structure coordinates having a root mean square deviation of not more than 2.0 Å from the set of structure coordinates corresponding to nucleotide coordinates 2040-2042, 2045, 2484, 2588 and 2589 set forth in Table 11.

39. The composition-of-matter of claim 37, wherein a three-dimensional atomic structure of said segment is defined by the set of structure coordinates corresponding to nucleotide coordinates 2040-2589 set forth in Table 11.

40. The composition-of-matter of claim 38, wherein a three-dimensional atomic structure of said segment is defined by a set of structure coordinates having a root mean square deviation of not more than 2.0 Å from the set of structure coordinates corresponding to nucleotide coordinates 2040-25 89 set forth in Table 11.

41. The composition-of-matter of claim 1, wherein said antibiotic is roxithromycin and whereas a three-dimensional atomic structure of said roxithromycin is defined by the set of structure coordinates corresponding to HETATM coordinates 59922-59979 set forth in Table 11.

42. The composition-of-matter of claim 1, wherein said antibiotic is roxithromycin and whereas a three-dimensional atomic structure of said roxithromycin is defined by a set of structure coordinates having a root mean square deviation of not more than 2.0 Å from the set of structure coordinates corresponding to HETATM coordinates 59922-59979 set forth in Table 11.

43. A composition-of-matter comprising a crystallized LRS of a eubacterium.

44. The composition-of-matter of claim 43, wherein said eubacterium is *D. radiodurans.*

45. The composition-of-matter of claim 43, wherein said eubacterium is a gram-positive bacterium.

46. The composition-of-matter of claim 43, wherein said eubacterium is a coccus.

47. The composition-of-matter of claim 43, wherein said eubacterium is a Deinococcus-Thermophilus group bacterium.

48. The composition-of-matter of claim 43, wherein said crystallized large ribosomal subunit is **characterized by** unit cell dimensions of a = 170.827 ± 10 Å, b = 409.430 ± 15 Å and c = 695.597 ± 25 Å.

49. The composition-of-matter of claim 43, wherein said crystallized large ribosomal subunit is **characterized by** having a crystal space group of I222.

50. The composition-of-matter of claim 43, wherein a three-dimensional atomic structure of at least a portion of said crystallized large ribosomal subunit is defined by a set of structure coordinates corresponding to a set of coordinates set forth in Table 3, said set of coordinates set forth in Table 3 being selected from the group consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881-62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

51. The composition-of-matter of claim 43, wherein a three-dimensional atomic structure of at least a portion of said crystallized large ribosomal subunit is defined by a set of structure coordinates having a root mean square deviation of not more than 2.0 Å from a set of structure coordinates corresponding to a set of coordinates set forth in Table 3, said set of coordinates set forth in Table 3 being selected from the consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881-62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

52. The composition-of-matter of claim 43, wherein said crystallized large ribosomal subunit comprises a nucleic acid molecule, a segment of which including nucleotides being capable of specifically associating with an antibiotic selected from the group consisting of chloramphenicol, a lincosamide antibiotic, clindamycin, a macrolide antibiotic, clarithromycin, erythromycin and roxithromycin.

53. The composition-of-matter of claim 52, wherein a three-dimensional atomic structure of said nucleic acid molecule is defined by the set of structure coordinates corresponding to atom coordinates 1-59360 set forth in Table 3.

54. The composition-of-matter of claim 52, wherein said antibiotic is chloramphenicol and whereas a three-dimensional atomic structure of said nucleotides being capable of specifically associating with said chloramphenicol is defined by the set of structure coordinates corresponding to nucleotide coordinates 2044, 2430, 2431, 2479 and 2483-2485 set forth in Table 3.

55. The composition-of-matter of claim 52, wherein said antibiotic is chloramphenicol and whereas a three-dimensional atomic structure of said nucleotides being capable of specifically associating with said chloramphenicol is defined by a set of structure coordinates having a root mean square deviation of not more than 2.0 Å from the set of structure coordinates corresponding to nucleotide coordinates 2044, 2430, 2431, 2479 and 2483-2485 set forth in Table 3.

56. The composition-of-matter of claim 52, wherein said antibiotic is chloramphenicol and whereas a three-dimensional atomic structure of said segment including said nucleotides being capable of specifically associating with said chloramphenicol is defined by the set of structure coordinates corresponding to nucleotide coordinates 2044-2485 set forth in Table 3.

57. The composition-of-matter of claim 52, wherein said antibiotic is chloramphenicol and whereas a three-dimensional atomic structure of said segment including said nucleotides being capable of specifically associating with said chloramphenicol is defined by a set of structure coordinates having a root mean square deviation of not more than 2.0 Å from the set of structure coordinates corresponding to nucleotide coordinates 2044-2485 set forth in Table 3.

58. The composition-of-matter of claim 52, wherein said antibiotic is clindamycin and whereas a three-dimensional atomic structure of said nucleotides being capable of specifically associating with said clindamycin is defined by the set of structure coordinates corresponding to nucleotide coordinates 2040-2042, 2044, 2482, 2484 and 2590 set forth in Table 3.

59. The composition-of-matter of claim 52, wherein said antibiotic is clindamycin and whereas a three-dimensional atomic structure of said nucleotides being capable of specifically associating with said clindamycin is defined by a set of structure coordinates having a root mean square deviation of not more than 2.0 Å from the set of structure coordinates corresponding to nucleotide coordinates 2040-2042, 2044, 2482, 2484 and 2590 set forth in Table 3.

60. The composition-of-matter of claim 52, wherein said antibiotic is clindamycin and whereas a three-dimensional atomic structure of said segment including said nucleotides being capable of specifically associating with said clindamycin is defined by the set of structure coordinates corresponding to nucleotide coordinates 2040-2590 set forth in Table 3.

61. The composition-of-matter of claim 52, wherein said antibiotic is clindamycin and whereas a three-dimensional atomic structure of said segment including said nucleotides being capable of specifically associating with said clindamycin is defined by a set of structure coordinates having a root mean square deviation of not more than 2.0 Å from the set of structure coordinates corresponding to nucleotide coordinates 2040-2590 set forth in Table 3.

62. The composition-of-matter of claim 52, wherein said antibiotic is clarithromycin, erythromycin or roxithromycin, and whereas a three-dimensional atomic structure of said nucleotides being capable of specifically associating with said antibiotic is defined by the set of structure coordinates corresponding to nucleotide coordinates 2040-2042, 2045, 2484, 2588 and 2589 set forth in Table 3.

63. The composition-of-matter of claim 52, wherein said antibiotic is clarithromycin, erythromycin or roxithromycin, and whereas a three-dimensional atomic structure of said nucleotides being capable of specifically associating with said antibiotic is defined by a set of structure coordinates having a root mean square deviation of not more than 2.0 Å from the set of structure coordinates corresponding to nucleotide coordinates 2040-2042, 2045, 2484, 2588 and 2589 set forth in Table 3.

64. The composition-of-matter of claim 52, wherein said antibiotic is clarithromycin, erythromycin or roxithromycin, and whereas a three-dimensional atomic structure of said segment including said nucleotides being capable of specifically associating with said antibiotic is defined by the set of structure coordinates corresponding to nucleotide coordinates 2040-2589 set forth in Table 3.

65. The composition-of-matter of claim 52, wherein said antibiotic is clarithromycin, erythromycin or roxithromycin, and whereas a three-dimensional atomic structure of said segment including said nucleotides being capable of specifically associating with said antibiotic is defined by a set of structure coordinates having a root mean square deviation of not more than 2.0 Å from the set of structure coordinates corresponding to nucleotide coordinates 2040-2589 set forth in Table 3.

66. A method of identifying a putative antibiotic comprising:
(a) obtaining a set of structure coordinates defining a three-dimensional atomic structure of a crystallized antibiotic-binding pocket of a large ribosomal subunit of a eubacterium; and
(b) computationally screening a plurality of compounds for a compound capable of specifically binding said antibiotic-binding pocket,
thereby identifying the putative antibiotic.

67. The method of claim 66, further comprising:
(i) contacting the putative antibiotic with said antibiotic-binding pocket; and
(ii) detecting specific binding of the putative antibiotic to said antibiotic-binding pocket, thereby qualifying the putative antibiotic.

68. The method of claim 66, wherein step (a) is effected by co-crystallizing at least said antibiotic-binding pocket with an antibiotic.

69. The method of claim 66, wherein said eubacterium is *D. radiodurans.*

70. The method of claim 66, wherein said eubacterium is a gram-positive bacterium.

71. The method of claim 66, wherein said eubacterium is a coccus.

72. The method of claim 66, wherein said eubacterium is a Deinococcus-Thermophilus group bacterium.

73. The method of claim 66, wherein said antibiotic-binding pocket is a clindamycin-binding pocket and whereas said structure coordinates define said three-dimensional atomic structure at a resolution higher than or equal to 3.1 Å.

74. The method of claim 66, wherein said antibiotic-binding pocket is an erythromycin-binding pocket and whereas said structure coordinates define said three-dimensional atomic structure at a resolution higher than or equal to 3.4 Å.

75. The method of claim 66, wherein said antibiotic-binding pocket is a clarithromycin-binding pocket and whereas said structure coordinates define said three-dimensional atomic structure at a resolution higher than or equal to 3.5 Å.

76. The method of claim 66, wherein said antibiotic-binding pocket is a roxithromycin-binding pocket and whereas said structure coordinates define said three-dimensional atomic structure at a resolution higher than or equal to 3.8 Å.

77. The method of claim 66, wherein said antibiotic-binding pocket is a chloramphenicol-binding pocket and whereas said structure coordinates define said three-dimensional atomic structure at a resolution higher than or equal to 3.5 Å.

78. The method of claim 66, wherein said antibiotic-binding pocket is selected from the group consisting of a chloramphenicol-specific antibiotic-binding pocket, a lincosamide-specific antibiotic-binding pocket, a clindamycin-specific antibiotic-binding pocket, a macrolide antibiotic-specific antibiotic-binding pocket, a clarithromycin-specific antibiotic-binding pocket, an erythromycin-specific antibiotic-binding pocket and a roxithromycin-specific antibiotic-binding pocket.

79. The method of claim 66, wherein the antibiotic comprises at least two non-covalently associated molecules.

80. The method of claim 66, wherein said set of structure coordinates define said three-dimensional structure at a resolution higher than or equal to a resolution selected from the group consisting of 5.4 Å, 5.3 Å, 5.2 Å, 5.1 Å, 5.0 Å, 4.9 Å, 4.8 Å, 4.7 Å, 4.6 Å, 4.5 Å, 4.4 Å, 4.3 Å, 4.2 Å, 4.1 Å, 4.0 Å, 3.9 Å, 3.8 Å, 3.7 Å, 3.6 Å, 3.5 Å, 3.4 Å, 3.3 Å, 3.2 Å and 3.1 Å.

81. The method of claim 66, wherein said antibiotic-binding pocket forms a part of a polynucleotide component of said large ribosomal subunit.

82. A computing platform for generating a three-dimensional model of at least a portion of a large ribosomal subunit of a eubacterium, the computing platform comprising:
(a) a data-storage device storing data comprising a set of structure coordinates defining at least a portion of a three-dimensional structure of the large ribosomal subunit; and
(b) a processing unit being for generating the three-dimensional model from said data stored in said data-storage device.

83. The computing platform of claim 82, further comprising a display being for displaying the three-dimensional model generated by said processing unit.

84. The computing platform of claim 82, wherein the eubacterium is *D. radiodurans.*

85. The computing platform of claim 82, wherein the eubacterium is a gram-positive bacterium.

86. The computing platform of claim 82, wherein the eubacterium is a coccus.

87. The computing platform of claim 82, wherein the eubacterium is a Deinococcus-Thermophilus group bacterium.

88. The computing platform of claim 82, wherein said set of structure coordinates define said portion of a three-dimensional structure of a large ribosomal subunit at a resolution higher than or equal to a resolution selected from the group consisting of 5.4 Å, 5.3 Å, 5.2 Å, 5.1 Å, 5.0 Å, 4.9 Å, 4.8 Å, 4.7 Å, 4.6 Å, 4.5 Å, 4.4 Å, 4.3 Å, 4.2 Å, 4.1 Å, 4.0 Å, 3.9 Å, 3.8 Å, 3.7 Å, 3.6 Å, 3.5 Å, 3.4 Å, 3.3 Å, 3.2 Å and 3.1 Å.

89. The computing platform of claim 82, wherein said set of structure coordinates define said portion of a three-dimensional structure of the large ribosomal subunit at a resolution higher than or equal to 3.1 Å.

90. The computing platform of claim 82, wherein said set of structure coordinates defining at least a portion of a three-dimensional structure of the large ribosomal subunit is a set of structure coordinates corresponding to a set of coordinates set forth in Table 3, said set of coordinates set forth in Table 3 being selected from the group consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881 -62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

91. The computing platform of claim 82, wherein said set of structure coordinates defining at least a portion of a three-dimensional structure of the large ribosomal subunit is a set of structure coordinates having a root mean square deviation of not more than 2.0 Å from a set of structure coordinates corresponding to a set of coordinates set forth in Table 3, said set of coordinates set forth in Table 3 being selected from the group consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881-62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

92. A computing platform for generating a three-dimensional model of at least a portion of a complex of an antibiotic and a large ribosomal subunit of a eubacterium, the computing platform comprising:
(a) a data-storage device storing data comprising a set of structure coordinates defining at least a portion of a three-dimensional structure of the complex of an antibiotic and a large ribosomal subunit; and
(b) a processing unit being for generating the three-dimensional model from said data stored in said data-storage device.

93. The computing platform of claim 92, further comprising a display being for displaying the three-dimensional model generated by said

94. The computing platform of claim 92, wherein the eubacterium is *D. radiodurans.*

95. The computing platform of claim 92, wherein the eubacterium is a gram-positive bacterium.

96. The computing platform of claim 92, wherein the eubacterium is a coccus.

97. The computing platform of claim 92, wherein the eubacterium is a Deinococcus-Thermophilus group bacterium.

98. The computing platform of claim 92, wherein the antibiotic is clindamycin and whereas said set of structure coordinates define said portion of a three-dimensional structure at a resolution higher than or equal to 3.1 Å.

99. The computing platform of claim 92, wherein the antibiotic is erythromycin and whereas said set of structure coordinates define said portion of a three-dimensional structure at a resolution higher than or equal to of 3.4 Å.

100. The computing platform of claim 92, wherein the antibiotic is clarithromycin and whereas said set of structure coordinates define said portion of a three-dimensional structure at a resolution higher than or equal to 3.5 Å.

101. The computing platform of claim 92, wherein the antibiotic is roxithromycin and whereas said set of structure coordinates define said portion of a three-dimensional structure at a resolution higher than or equal to 3.8 Å.

102. The computing platform of claim 92, wherein the antibiotic is chloramphenicol and whereas said set of structure coordinates define said portion of a three-dimensional structure at a resolution higher than or equal to 3.5 Å.

103. The computing platform of claim 92, wherein the antibiotic is selected from the group consisting of chloramphenicol, a lincosamide, clindamycin, a macrolide antibiotic, clarithromycin, erythromycin and roxithromycin.

104. The computing platform of claim 92, wherein the antibiotic is chloramphenicol and whereas said set of structure coordinates defining at least a portion of a three-dimensional structure of the complex of said chloramphenicol and said large ribosomal subunit corresponds to a set of coordinates selected from the group consisting of: nucleotide coordinates 2044, 2430, 2431, 2479 and 2483-2485 set forth in Table 7; nucleotide coordinates 2044-2485 set forth in Table 7; HETATM coordinates 59925-59944 set forth in Table 7; the set of atom coordinates set forth in Table 7; and the set of atom coordinates set forth in Table 12.

105. The computing platform of claim 92, wherein the antibiotic is clindamycin and whereas said set of structure coordinates defining at least a portion of a three-dimensional structure of the complex of said clindamycin and said large ribosomal subunit corresponds to a set of coordinates selected from the group consisting of: nucleotide coordinates 2040-2042, 2044, 2482, 2484 and 2590 set forth in Table 8; nucleotide coordinates 2040-2590 set forth in Table 8; HETATM coordinates 59922-59948 set forth in Table 8; the set of atom coordinates set forth in Table 8; and the set of atom coordinates set forth in Table 13.

106. The computing platform of claim 92, wherein the antibiotic is clarithromycin and whereas said set of structure coordinates defining at least a portion of a three-dimensional structure of the complex of said clarithromycin and said large ribosomal subunit corresponds to a set of coordinates selected from the group consisting of: nucleotide coordinates 2040-2042, 2045, 2484, 2588 and 2589 set forth in Table 9; nucleotide coordinates 2040-2589 set forth in Table 9; HETATM coordinates 59922-59973 set forth in Table 9; the set of atom coordinates set forth in Table 9; and the set of atom coordinates set forth in Table 14.

107. The computing platform of claim 92, wherein the antibiotic is erythromycin and whereas said set of structure coordinates defining at least a portion of a three-dimensional structure of the complex of said erythromycin and said large ribosomal subunit corresponds to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59972 set forth in Table 10; the set of atom coordinates set forth in Table 10; and the set of atom coordinates set forth in Table 15.

108. The computing platform of claim 92, wherein the antibiotic is roxithromycin and whereas said set of structure coordinates defining at least a portion of a three-dimensional structure of the complex of said roxithromycin and said large ribosomal subunit corresponds to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59979 set forth in Table 11; the set of atom coordinates set forth in Table 11; and the set of atom coordinates set forth in Table 16.

109. The computing platform of claim 92, wherein the antibiotic is chloramphenicol and whereas said set of structure coordinates defining at least a portion of a three-dimensional structure of the complex of said chloramphenicol and said large ribosomal subunit is a set of structure coordinates having a root mean square deviation of not more than 2.0 Å from a set of structure coordinates corresponding to a set of coordinates selected from the group consisting of: nucleotide coordinates 2044, 2430, 2431, 2479 and 2483-2485 set forth in Table 7; nucleotide coordinates 2044-2485 set forth in Table 7; HETATM coordinates 59925-59944 set forth in Table 7; the set of atom coordinates set forth in Table 7; and the set of atom coordinates set forth in Table 12.

110. The computing platform of claim 92, wherein the antibiotic is clindamycin and whereas said set of structure coordinates defining at least a portion of a three-dimensional structure of the complex of said clindamycin and said large ribosomal subunit is a set of structure coordinates having a root mean square deviation of not more than 2.0 Å from a set of structure coordinates corresponding to a set of coordinates selected from the group consisting of: nucleotide coordinates 2040-2042, 2044, 2482, 2484 and 2590 set forth in Table 8; nucleotide coordinates 2040-2590 set forth in Table 8; HETATM coordinates 59922-59948 set forth in Table 8; the set of atom coordinates set forth in Table 8; and the set of atom coordinates set forth in Table 13.

111. The computing platform of claim 92, wherein the antibiotic is clarithromycin and whereas said set of structure coordinates defining at least a portion of a three-dimensional structure of the complex of said clarithromycin and said large ribosomal subunit is a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 9; nucleotide coordinates 2040-2589 set forth in Table 9; HETATM coordinates 59922-59973 set forth in Table 9; the set of atom coordinates set forth in Table 9; and the set of atom coordinates set forth in Table 14.

112. The computing platform of claim 92, wherein the antibiotic is erythromycin and whereas said set of structure coordinates defining at least a portion of a three-dimensional structure of the complex of said erythromycin and said large ribosomal subunit is a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59972 set forth in Table 10; the set of atom coordinates set forth in Table 10; and the set of atom coordinates set forth in Table 15.

113. The computing platform of claim 92, wherein the antibiotic is roxithromycin and whereas said set of structure coordinates defining at least a portion of a three-dimensional structure of the complex of said roxithromycin and said large ribosomal subunit is a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59979 set forth in Table 11; the set of atom coordinates set forth in Table 11; and the set of atom coordinates set forth in Table 16.

114. A computer generated model representing at least a portion of a large ribosomal subunit of a eubacterium, the computer generated model having a three-dimensional atomic structure defined by a set of structure coordinates corresponding to a set of coordinates set forth in Table 3, the set of coordinates set forth in Table 3 being selected from the group consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881-62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

115. The computer generated model of claim 114, wherein the eubacterium is *D. radiodurans.*

116. The computer generated model of claim 114, wherein the eubacterium is a gram-positive bacterium.

117. The computer generated model of claim 114, wherein the eubacterium is a coccus.

118. The computer generated model of claim 114, wherein the eubacterium is a Deinococcus-Thermophilus group bacterium.

119. A computer generated model representing at least a portion of a large ribosomal subunit of a eubacterium, the computer generated model having a three-dimensional atomic structure defined by a set of structure coordinates having a root mean square deviation of not more than 2.0 A from a set of structure coordinates corresponding to a set of coordinates set forth in Table 3, the set of coordinates set forth in Table 3 being selected from the group consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881-62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

120. The computer generated model of claim 119, wherein the eubacterium is *D. radiodurans.*

121. The computer generated model of claim 119, wherein the eubacterium is a gram-positive bacterium.

122. The computer generated model of claim 119, wherein the eubacterium is a coccus.

123. The computer generated model of claim 119, wherein the eubacterium is a Deinococcus-Thermophilus group bacterium.

124. A computer generated model representing at least a portion of a complex of an antibiotic and a large ribosomal subunit of a eubacterium.

125. The computer generated model of claim 124, wherein the eubacterium is *D. radiodurans.*

126. The computer generated model of claim 124, wherein the eubacterium is a gram-positive bacterium.

127. The computer generated model of claim 124, wherein the eubacterium is a coccus.

128. The computer generated model of claim 124, wherein the eubacterium is a Deinococcus-Thermophilus group bacterium.

129. The computer generated model of claim 124, wherein the antibiotic is selected from the group consisting of chloramphenicol, a lincosamide antibiotic, clindamycin, a macrolide antibiotic, clarithromycin, erythromycin and roxithromycin.

130. The computer generated model of claim 124, wherein the antibiotic is clindamycin and whereas the set of structure coordinates define the three-dimensional structure of the computer generated model at a resolution higher than or equal to 3.1 Å.

131. The computer generated model of claim 124, wherein the antibiotic is erythromycin and whereas the set of structure coordinates define the three-dimensional structure of the computer generated model at a resolution higher than or equal to 3.4 Å.

132. The computer generated model of claim 124, wherein the antibiotic is clarithromycin and whereas the set of structure coordinates define the three-dimensional structure of the computer generated model at a resolution higher than or equal to 3.5 Å.

133. The computer generated model of claim 124, wherein the antibiotic is roxithromycin and whereas the set of structure coordinates define the three-dimensional structure of the computer generated model at a resolution higher than or equal to 3.8 Å.

134. The computer generated model of claim 124, wherein the antibiotic is chloramphenicol and whereas the set of structure coordinates define the three-dimensional structure of the computer generated model at a resolution higher than or equal to 3.5 Å.

135. The computer generated model of claim 124, wherein the antibiotic is chloramphenicol and whereas a three-dimensional atomic structure of the portion of a complex of said chloramphenicol and the large ribosomal subunit is defined by a set of structure coordinates corresponding to a set of coordinates selected from the group consisting of: nucleotide coordinates 2044, 2430, 2431, 2479 and 2483-2485 set forth in Table 7; nucleotide coordinates 2044-2485 set forth in Table 7; HETATM coordinates 59925-59944 set forth in Table 7; the set of atom coordinates set forth in Table 7; and the set of atom coordinates set forth in Table 12.

136. The computer generated model of claim 124, wherein the antibiotic is clindamycin and whereas a three-dimensional atomic structure of the portion of a complex of said clindamycin and the large ribosomal subunit is defined by a set of structure coordinates corresponding to a set of coordinates selected from the group consisting of: nucleotide coordinates 2040-2042, 2044, 2482, 2484 and 2590 set forth in Table 8; nucleotide coordinates 2040-2590 set forth in Table 8; HETATM coordinates 59922-59948 set forth in Table 8; the set of atom coordinates set forth in Table 8; and the set of atom coordinates set forth in Table 13.

137. The computer generated model of claim 124, wherein the antibiotic is clarithromycin and whereas a three-dimensional atomic structure of the portion of a complex of said clarithromycin and the large ribosomal subunit is defined by a set of structure coordinates corresponding to a set of coordinates selected from the group consisting of: nucleotide coordinates 2040-2042, 2045, 2484, 2588 and 2589 set forth in Table 9; nucleotide coordinates 2040-2589 set forth in Table 9; HETATM coordinates 59922-59973 set forth in Table 9; the set of atom coordinates set forth in Table 9; and the set of atom coordinates set forth in Table 14.

138. The computer generated model of claim 124, wherein the antibiotic is erythromycin and whereas a three-dimensional atomic structure of the portion of a complex of said erythromycin and the large ribosomal subunit is defined by a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59972 set forth in Table 10; the set of atom coordinates set forth in Table 10; and the set of atom coordinates set forth in Table 15.

139. The computer generated model of claim 124, wherein the antibiotic is roxithromycin and whereas a three-dimensional atomic structure of the portion of a complex of said roxithromycin and the large ribosomal subunit is defined by a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59979 set forth in Table 11; the set of atom coordinates set forth in Table 11; and the set of atom coordinates set forth in Table 16.

140. The computer generated model of claim 124, wherein the antibiotic is chloramphenicol and whereas a three-dimensional atomic structure of the portion of a complex of said chloramphenicol and the large ribosomal subunit is defined by a set of structure coordinates having a root mean square deviation of not more than 2.0 Å from a set of structure coordinates corresponding to a set of coordinates selected from the group consisting of: nucleotide coordinates 2044, 2430, 2431, 2479 and 2483-2485 set forth in Table 7; nucleotide coordinates 2044-2485 set forth in Table 7; HETATM coordinates 59925-59944 set forth in Table 7; the set of atom coordinates set forth in Table 7; and the set of atom coordinates set forth in Table 12.

141. The computer generated model of claim 124, wherein the antibiotic is clindamycin and whereas a three-dimensional atomic structure of the portion of a complex of said clindamycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2044, 2482, 2484 and 2590 set forth in Table 8; nucleotide coordinates 2040-2590 set forth in Table 8; HETATM coordinates 59922-59948 set forth in Table 8; the set of atom coordinates set forth in Table 8; and the set of atom coordinates set forth in Table 13.

142. The computer generated model of claim 124, wherein the antibiotic is clarithromycin and whereas a three-dimensional atomic structure of the portion of a complex of said clarithromycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 9; nucleotide coordinates 2040-2589 set forth in Table 9; HETATM coordinates 59922-59973 set forth in Table 9; the set of atom coordinates set forth in Table 9; and the set of atom coordinates set forth in Table 14.

143. The computer generated model of claim 124, wherein the antibiotic is erythromycin and whereas a three-dimensional atomic structure of the portion of a complex of said erythromycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59972 set forth in Table 10; the set of atom coordinates set forth in Table 10; and the set of atom coordinates set forth in Table 15.

144. The computer generated model of claim 124, wherein the antibiotic is roxithromycin and whereas a three-dimensional atomic structure of the portion of a complex of said roxithromycin and the large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59979 set forth in Table 11; the set of atom coordinates set forth in Table 11; and the set of atom coordinates set forth in Table 16.

145. A computer readable medium comprising, in a retrievable format, data including a set of structure coordinates defining at least a portion of a three-dimensional structure of a crystallized large ribosomal subunit of a eubacterium.

146. The computer readable medium of claim 145, wherein the eubacterium is *D. radiodurans.*

147. The computer readable medium of claim 145, wherein the eubacterium is a gram-positive bacterium.

148. The computer readable medium of claim 145, wherein the eubacterium is a coccus.

149. The computer readable medium of claim 145, wherein the eubacterium is a Deinococcus-Thermophilus group bacterium.

150. The computer readable medium of claim 145, wherein said set of structure coordinates define said portion of a three-dimensional structure of a crystallized large ribosomal subunit at a resolution higher than or equal to a resolution selected from the group consisting of 5.4 Å, 5.3 Å, 5.2 Å, 5.1 Å, 5.0 Å, 4.9 Å, 4.8 Å, 4.7 Å, 4.6 Å, 4.5 Å, 4.4 Å, 4.3 Å, 4.2 Å, 4.1 Å, 4.0 Å, 3.9 Å, 3.8 Å, 3.7 Å, 3.6 Å, 3.5 Å, 3.4 Å, 3.3 Å, 3.2 Å and 3.1 Å.

151. The computer readable medium of claim 145, wherein said set of structure coordinates define said portion of a three-dimensional structure of a crystallized large ribosomal subunit at a resolution higher than or equal to 3.1 Å.

152. The computer readable medium of claim 145, wherein said structure coordinates defining at least a portion of a three-dimensional structure of a crystallized large ribosomal subunit correspond to a set of coordinates set forth in Table 3, said set of coordinates set forth in Table 3 being selected from the group consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881-62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

153. The computer readable medium of claim 145, wherein said structure coordinates defining at least a portion of a three-dimensional structure of a crystallized large ribosomal subunit have a root mean square deviation of not more than 2.0 Å from a set of structure coordinates corresponding to a set of coordinates set forth in Table 3, said set of coordinates set forth in Table 3 being selected from the group consisting of:
nucleotide coordinates 2044, 2430, 2431, 2479 and 2483-2485;
nucleotide coordinates 2044-2485;
nucleotide coordinates 2040-2042, 2044, 2482, 2484 and 2590;
nucleotide coordinates 2040-2590;
nucleotide coordinates 2040-2042, 2045, 2484, 2588 and 2589;
nucleotide coordinates 2040-2589;
atom coordinates 1-59360;
atom coordinates 59361-61880;
atom coordinates 1-61880;
atom coordinates 61881-62151;
atom coordinates 62152-62357;
atom coordinates 62358-62555;
atom coordinates 62556-62734;
atom coordinates 62735-62912;
atom coordinates 62913-62965;
atom coordinates 62966-63109;
atom coordinates 63110-63253;
atom coordinates 63254-63386;
atom coordinates 63387-63528;
atom coordinates 63529-63653;
atom coordinates 63654-63768;
atom coordinates 63769-63880;
atom coordinates 63881-64006;
atom coordinates 64007-64122;
atom coordinates 64123-64223;
atom coordinates 64224-64354;
atom coordinates 64355-64448;
atom coordinates 64449-64561;
atom coordinates 64562-64785;
atom coordinates 64786-64872;
atom coordinates 64873-64889;
atom coordinates 64890-64955;
atom coordinates 64956-65011;
atom coordinates 65012-65085;
atom coordinates 65086-65144;
atom coordinates 65145-65198;
atom coordinates 65199-65245;
atom coordinates 65246-65309;
atom coordinates 65310-65345;
atom coordinates 61881-65345; and
atom coordinates 1-65345.

154. A computer readable medium comprising, in a retrievable format, data including a set of structure coordinates defining at least a portion of a three-dimensional structure of a crystallized complex of an antibiotic and a large ribosomal subunit of a eubacterium.

155. The computer readable medium of claim 154, wherein the eubacterium is *D. radiodurans.*

156. The computer readable medium of claim 154, wherein the eubacterium is a gram-positive bacterium.

157. The computer readable medium of claim 154, wherein the eubacterium is a coccus.

158. The computer readable medium of claim 154, wherein the eubacterium is a Deinococcus-Thermophilus group bacterium.

159. The computer readable medium of claim 154, wherein said antibiotic is selected from the group consisting of chloramphenicol, a lincosamide antibiotic, clindamycin, a macrolide antibiotic, clarithromycin, erythromycin and roxithromycin.

160. The computer readable medium of claim 154, wherein said antibiotic is clindamycin and whereas said set of structure coordinates define said portion of a three-dimensional structure of a crystallized complex of an antibiotic and a large ribosomal subunit at a resolution higher than or equal to 3.1 Å.

161. The computer readable medium of claim 154, wherein said antibiotic is erythromycin and whereas said set of structure coordinates define said portion of a three-dimensional structure of a crystallized complex of an antibiotic and a large ribosomal subunit at a resolution higher than or equal to 3.4 Å.

162. The computer readable medium of claim 154, wherein said antibiotic is clarithromycin and whereas said set of structure coordinates define said portion of a three-dimensional structure of a crystallized complex of an antibiotic and a large ribosomal subunit at a resolution higher than or equal to 3.5 Å.

163. The computer readable medium of claim 154, wherein said antibiotic is roxithromycin and whereas said set of structure coordinates define said portion of a three-dimensional structure of a crystallized complex of an antibiotic and a large ribosomal subunit at a resolution higher than or equal to 3.8 Å.

164. The computer readable medium of claim 154, wherein said antibiotic is chloramphenicol and whereas said set of structure coordinates define said portion of a three-dimensional structure of a crystallized complex of an antibiotic and a large ribosomal subunit at a resolution higher than or equal to 3.5 Å.

165. The computer readable medium of claim 154, wherein said antibiotic is chloramphenicol and whereas said three-dimensional structure of a crystallized complex of an antibiotic and a large ribosomal subunit is defined by a set of structure coordinates corresponding to a set of coordinates selected from the group consisting of: nucleotide coordinates 2044, 2430, 2431, 2479 and 2483-2485 set forth in Table 7; nucleotide coordinates 2044-2485 set forth in Table 7; HETATM coordinates 59925-59944 set forth in Table 7; the set of atom coordinates set forth in Table 7; and the set of atom coordinates set forth in Table 12.

166. The computer readable medium of claim 154, wherein said antibiotic is clindamycin and whereas said three-dimensional structure of a crystallized complex of an antibiotic and a large ribosomal subunit is defined by a set of structure coordinates corresponding to a set of coordinates selected from the group consisting of: nucleotide coordinates 2040-2042, 2044, 2482, 2484 and 2590 set forth in Table 8; nucleotide coordinates 2040-2590 set forth in Table 8; HETATM coordinates 59922-59948 set forth in Table 8; the set of atom coordinates set forth in Table 8; and the set of atom coordinates set forth in Table 13.

167. The computer readable medium of claim 154, wherein said antibiotic is clarithromycin and whereas said three-dimensional structure of a crystallized complex of an antibiotic and a large ribosomal subunit is defined by a set of structure coordinates corresponding to a set of coordinates selected from the group consisting of: nucleotide coordinates 2040-2042, 2045, 2484, 2588 and 2589 set forth in Table 9; nucleotide coordinates 2040-2589 set forth in Table 9; HETATM coordinates 59922-59973 set forth in Table 9; the set of atom coordinates set forth in Table 9; and the set of atom coordinates set forth in Table 14.

168. The computer readable medium of claim 154, wherein said antibiotic is erythromycin and whereas said three-dimensional structure of a crystallized complex of an antibiotic and a large ribosomal subunit is defined by a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59972 set forth in Table 10; the set of atom coordinates set forth in Table 10; and the set of atom coordinates set forth in Table 15.

169. The computer readable medium of claim 154, wherein said antibiotic is roxithromycin and whereas said three-dimensional structure of a crystallized complex of an antibiotic and a large ribosomal subunit is defined by a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59979 set forth in Table 11; the set of atom coordinates set forth in Table 11; and the set of atom coordinates set forth in Table 16.

170. The computer readable medium of claim 154, wherein said antibiotic is chloramphenicol and whereas said three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2044, 2430, 2431, 2479 and 2483-2485 set forth in Table 7; nucleotide coordinates 2044-2485 set forth in Table 7; HETATM coordinates 59925-59944 set forth in Table 7; the set of atom coordinates set forth in Table 7; and the set of atom coordinates set forth in Table 12.

171. The computer readable medium of claim 154, wherein said antibiotic is clindamycin and whereas said three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2044, 2482, 2484 and 2590 set forth in Table 8; nucleotide coordinates 2040-2590 set forth in Table 8; HETATM coordinates 59922-59948 set forth in Table 8; the set of atom coordinates set forth in Table 8; and the set of atom coordinates set forth in Table 13.

172. The computer readable medium of claim 154, wherein said antibiotic is clarithromycin and whereas said three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 9; nucleotide coordinates 2040-2589 set forth in Table 9; HETATM coordinates 59922-59973 set forth in Table 9; the set of atom coordinates set forth in Table 9; and the set of atom coordinates set forth in Table 14.

173. The computer readable medium of claim 154, wherein said antibiotic is erythromycin and whereas said three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 10; nucleotide coordinates 2040-2589 set forth in Table 10; HETATM coordinates 59922-59972 set forth in Table 10; the set of atom coordinates set forth in Table 10; and the set of atom coordinates set forth in Table 15.

174. The computer readable medium of claim 154, wherein said antibiotic is roxithromycin and whereas said three-dimensional structure of a crystallized complex of an antibiotic and a large ribosomal subunit is defined by a set of structure coordinates having a root mean square deviation of not more than 2.0 Å from a set of structure coordinates corresponding to a set of coordinates selected from the group consisting of: nucleotide coordinates 2040-2042, 2045, 2484, 2588 and 2589 set forth in Table 11; nucleotide coordinates 2040-2589 set forth in Table 11; HETATM coordinates 59922-59979 set forth in Table 11; the set of atom coordinates set forth in Table 11; and the set of atom coordinates set forth in Table 16.

175. A method of crystallizing a large ribosomal subunit of a eubacterium comprising:
(a) suspending a purified preparation of the large ribosomal subunit in a crystallization solution, said crystallization solution comprising a buffer component and a volatile component, said volatile component being at a first concentration in the crystallization solution, thereby forming a crystallization mixture; and
(b) equilibrating said crystallization mixture with an equilibration solution, said equilibration solution comprising said buffer component and said volatile component, said volatile component being at a second concentration in the equilibration solution, said second concentration being a fraction of said first concentration,
thereby crystallizing the large ribosomal subunit.

176. The method of claim 175, wherein the eubacterium is *D. radiodurans.*

177. The method of claim 175, wherein the eubacterium is a gram-positive bacterium.

178. The method of claim 175, wherein the eubacterium is a coccus.

179. The method of claim 175, wherein the eubacterium is a Deinococcus-Thermophilus group bacterium.

180. The method of claim 175, wherein said volatile component is an alcohol component.

181. The method of claim 175, wherein said volatile component comprises at least one monovalent alcohol and at least one polyvalent alcohol.

182. The method of claim 181, wherein the volumetric ratio of said at least one multivalent alcohol to said at least one monovalent alcohol is selected from the range consisting of 1:3.0-1:4.1.

183. The method of claim 181, wherein the volumetric ratio of said at least one multivalent alcohol to said at least one monovalent alcohol is 1:3.5.

184. The method of claim 181, wherein said at least one monovalent alcohol is ethanol.

185. The method of claim 181, wherein said at least one polyvalent alcohol is dimethylhexandiol.

186. The method of claim 175, wherein said first concentration is selected from a range consisting of 0.1-10 % (v/v).

187. The method of claim 175, wherein said fraction is selected from a range consisting of 0.33-0.67.

188. The method of claim 175, wherein said fraction is 0.5.

189. The method of claim 175, wherein said buffer component is an optimal buffer for the functional activity of the large ribosomal subunit.

190. The method of claim 175, wherein said buffer component is an aqueous solution comprising:
MgCl₂ in such a quantity as to yield a final concentration of said MgCl₂ in said crystallization solution, said equilibration solution, or both selected from a range consisting of 3-12 mM;
NH₄Cl in such a quantity as to yield a final concentration of said NH₄Cl in said crystallization solution, said equilibration solution, or both selected from a range consisting of 20-70 mM;
KCl in such a quantity as to yield a final concentration of said KCl in said crystallization solution, said equilibration solution, or both selected from a range consisting of 0-15 mM; and
HEPES in such a quantity as to yield a final concentration of said HEPES in said crystallization solution, said equilibration solution, or both selected from a range consisting of 8-20 mM.

191. The method of claim 175, wherein said crystallization solution, said equilibration solution, or both have a pH selected from the range consisting of 6.0-9.0 pH units.

192. The method of claim 175, wherein said equilibrating is effected by vapor diffusion.

193. The method of claim 175, wherein said equilibrating is effected at a temperature selected from a range consisting of 15-25 °C.

194. The method of claim 175, wherein said equilibrating is effected at a temperature selected from a range consisting of 17-20 °C.

195. The method of claim 175, wherein said equilibrating is effected using a hanging drop of the crystallization mixture.

196. The method of claim 175, wherein said equilibrating is effected using Linbro dishes.

197. The method of claim 175, wherein said crystallization solution, said equilibration solution, or both comprise 10 mM MgCl₂, 60 mM NH₄Cl, 5 mM KCl and 10 mM HEPES.

198. The method of claim 175, wherein said crystallization solution, said equilibration solution, or both have a pH of 7.8.

199. The method of claim 175, wherein said crystallization solution comprises an antibiotic.

200. The method of claim 199, wherein said antibiotic is selected from the group consisting of chloramphenicol, a lincosamide antibiotic, clindamycin, a macrolide antibiotic, erythromycin and roxithromycin.

201. The method of claim 199, wherein said crystallization solution comprises said antibiotic at a concentration selected from the range consisting of 0.8-3.5 mM.

202. The method of claim 175, further comprising soaking the crystallized ribosomal subunit in a soaking solution containing an antibiotic.

203. The method of claim 202, wherein said antibiotic is clarithromycin.

204. The method of claim 202, wherein said soaking solution comprises said antibiotic at a concentration selected from the range consisting of 0.004-0.025 mM.

205. The method of claim 202, wherein said soaking solution comprises said antibiotic at a concentration of 0.01 mM.
